# EUROPÄISCHE PATENTSCHRIFT

(11) **EP 1 448 781 B1**
(45) Veröffentlichungstag und Bekanntmachung des Hinweises auf die Patenterteilung: **18.07.2007**
(21) Anmeldenummer: 02796502.9
(22) Anmeldetag: 20.11.2002
(51) Int. Cl.: C12N 15/85, C12N 5/10, G01N 33/50

(54) **VERFAHREN ZUR IDENTIFIZIERUNG VON ZIELEPITOPEN DER T-ZELL-VERMITTELTEN IMMUNANTWORT UND ZUM NACHWEIS EPITOP-SPEZIFISCHER T-ZELLEN**
METHOD FOR IDENTIFYING TARGET EPITOPES OF THE T CELL MEDIATED IMMUNE RESPONSE AND FOR ASSAYING EPITOPE-SPECIFIC T CELLS
PROCEDE D'IDENTIFICATION D'EPITOPES CIBLES DE LA REPONSE IMMUNITAIRE INDUITE PAR LES LYMPHOCYTES T ET DE DETECTION DES LYMPHOCYTES T SPECIFIQUES A L'EPITOPE

(30) Priorität: 20.11.2001 DE 10156863
(43) Veröffentlichungstag der Anmeldung: 25.08.2004
(73) Patentinhaber: Deml, Ludwig, 93128 Regenstauf (DE)
(72) Erfinder: Deml, Ludwig, 93128 Regenstauf (DE)
(74) Vertreter: Dehmel, Albrecht
(86) Internationale Anmeldenummer: PCT/DE2002/004270
(87) Internationale Veröffentlichungsnummer: WO 2003/046212

(56) Entgegenhaltungen:
- WO-A-00/57705
- WO-A-00/67761
- WO-A-01/32204
- TAKAYAMA T ET AL: "TRANSDUCTION OF DENDRITIC CELL PROGENITORS WITH A RETROVIRAL VECTOR ENCODING VIRAL INTERLEUKIN-10 AND ENHANCED GREEN FLUORESCENT PROTEIN ALLOWS PURIFICATION OF POTENTIALLY TOLEROGENIC ANTIGEN-PRESENTING CELLS" TRANSPLANTATION, Bd. 68, Nr. 12, 27. Dezember 1999 (1999-12-27), Seiten 1903-1909, XP000943559 ISSN: 0041-1337

## Beschreibung

Die vorliegende Erfindung betrifft ein Verfahren zum Nachweis Epitop-spezifischer T-Zellen und von Zielepitopen reaktiver T-Zellen. Die vorliegende Erfindung betrifft ferner Vektoren, umfassend einen spezifisch in antigenpräsentierenden Zellen durch den epitop-spezifischen Kontakt mit einer T-Zelle induzierbaren ersten Promotor, eine mit diesem ersten Promotor funktionell verbundene Nukleinsäure kodierend ein Markergen, einen in antigenpräsentierenden Zellen konstitutiven zweiten Promotor, und eine mit diesem zweiten Promotor funktionell verbundene Nukleinsäure. Die vorliegende Erfindung betrifft ferner antigenpräsentierende Zellen, transduziert mit den erfindungsgemäßen Vektoren.

Der erworbene Ast des Immunsystems besteht aus einer humoralen (Immunglobuline) und einer zellulären Immunabwehr. Zelluläre und erregerspezifische Polypeptide werden von Zellen durch spezifische Spaltung aufgearbeitet und Bruchstücke (Epitope) davon zusammen mit MHC-Molekülen der Klassen-I und/oder -II auf der Oberfläche von antigenpräsentierenden Zellen (APC) präsentiert. T-Zellen erkennen mittels ihres T-Zellrezeptors spezifisch Epitope, die im Komplex mit körpereigenen MHC-Proteinen präsentiert werden, und setzen eine Immunreaktion in Gang.

T-Zellen können anhand spezifischer Oberflächenproteine in verschiedene Effektorpopulationen untergliedert werden. CD4⁺ T-Helferzellen haben eine zentrale Bedeutung bei der Steuerung der Immunabwehr. Nach einer spezifischen Erkennung von Epitopen, die ihnen auf der Oberfläche von APC zusammen mit MHC-Proteinen präsentiert werden, steuern sie durch die Sekretion unterschiedlicher Botenstoffe (beispielsweise Zytokine) die Produktion von Antikörpern durch B-Zellen (humoraler Ast der Immunantwort) und die Aktivierung CD8⁺ zytotoxischer T-Zellen (CTL) (zellulärer Ast der Immunantwort). Die Bedeutung von CD8⁺ CTL liegt in der Erkennung und Zerstörung entarteter und von Mikroorganismen oder Parasiten befallener Zellen und Gewebe. T-Zellen stellen somit einen bedeutenden Schutzmechanismus des erworbenen Immunsystems zur Verhinderung und Kontrolle mikrobiell, insbesondere virusbedingter Erkrankungen und zur Erkennung und Zerstörung entarteter körpereigener Zellen dar.

Professionelle APC, wie dendritische Zellen, Monozyten, Makrophagen, aber auch nicht professionelle APC wie B-Zellen spielen sowohl bei der Auslösung einer T-Zell-Antwort gegen körperfremde Immunogene als auch bei der Induktion einer T-Zell-Toleranz gegen körpereigene Gewebe eine zentrale Rolle. Die Aktivierung und Proliferation von T-Zellen erfolgt durch das gleichzeitige Auslösen von zwei Signalen. Das erste Signal wird durch den T-Zellrezeptor, der ein Epitop im Zusammenhang mit MHC auf der Oberfläche von APC erkennt, in die T-Zelle geleitet. Das zweite kostimulatorische Signal wird durch die spezifische Interaktion der kostimulatorischen Moleküle B7.1 (CD80) oder B7.2 (CD86) auf der APC mit dem zugehörigen Rezeptor (CD28) auf der Oberfläche der T-Zelle vermittelt. In Abwesenheit des kostimulatorischen Signals wird die T-Zelle anerg. Anergie beschreibt einen Zustand, in dem die T-Zellen sich nicht vermehren und nicht auf ein Antigen reagieren.

Der Aktivierungsgrad einer APC und die Beschaffenheit einer Fremdsubstanz entscheiden maßgeblich über das Profil der induzierten Immunantwort. So beeinflussen die Konzentration und biochemischen Eigenschaften einer Fremdsubstanz sowie die An- oder Abwesenheit von immunmodulatorischen Substanzen (insbesondere bakterielle Lipopolysaccharide (LPS), bakterielle Nukleinsäuren (mit CpG Motiven) und körperfremde Polypeptide (z.B. bakterielles Flagellin)) maßgeblich, ob der zelluläre (Th-1 zellvermittelte Immunität) oder humorale Ast (Th-2 zellvermittelte Immunantwort) des Immunsystems aktiviert wird oder ob die Immunantwort tolerogen verläuft.

Zur Vermeidung unerwünschter Immunreaktionen gegen körpereigene Proteine und Gewebe werden autoreaktive T-Zellen frühzeitig eliminiert (klonale Deletion) oder inaktiviert (Anergie). Die Folge ist eine antigenspezifische Toleranz gegen körpereigene Strukturen (Polypeptide, Zellen, Gewebe und Organe). Bei Autoimmunerkrankungen sind diese Schutzmechanismen gestört oder nur unzureichend ausgeprägt. Die Ursachen, die der Entstehung von Autoimmunerkrankungen zugrunde liegen, sind bislang nur wenig verstanden. Möglicherweise werden körpereigene Strukturen aufgrund ihrer Ähnlichkeit mit Erreger- und Fremdgewebespezifischen Polypeptiden fälschlicherweise als fremd erkannt und von fehlgeleiteten Effektoren des erworbenen Immunsystems (T-Zellen und/oder Antikörpern) geschädigt oder sogar zerstört.

Daneben kommt es im Zusammenhang mit chronisch persistierenden Virusinfektionen nicht selten zu entzündlichen Prozessen, die zum Teil lebenswichtige Organe wie die Leber (virale Hepatiden) betreffen, obwohl die Immunantwort primär gegen erregerspezifische Strukturen gerichtet ist. In solchen Fällen spricht man von Immunpathogenese, da der Schaden und die Krankheitssymptome primär durch das eigene Immunsystem und nicht durch den Erreger verursacht werden.

Ähnliche Immunreaktionen liegen auch der Abstoßung von transplantierten Geweben und Organen zugrunde. Hier wird eine Kombination aus fremden MHC-Proteinen des Spenders und körpereigenen Epitopen von T-Zellen des Empfängers als fremd erkannt und angegriffen.

Im Unterschied zu den oben genannten Erkrankungen ist bei Tumorerkrankungen oftmals eine unzureichende Immunerkennung zu beobachten. Häufig werden tumorinfiltrierende Lymphozyten (TIL) nachgewiesen, die den Tumor in begrenztem Umfang angreifen, deren Spezifität auf molekularer Ebene aber meist nicht bekannt ist. Warum diese TILs *in vivo* nicht in der Lage sind, den Tumor zu eliminieren, ist unklar. Die Konzentration dieser TILs im Blut, die in der Regel sehr gering ist und oftmals sogar unter der Nachweisgrenze liegt, ist vermutlich ein entscheidender Faktor. Zur Stimulation und Steigerung einer solchen tumorspezifischen Immunantwort wird heute versucht, *ex vivo* (außerhalb des Körpers) TILs mit Tumormaterial zu stimulieren und in den Körper zurückzuführen. In anderen Ansätzen wird Tumorbiopsiematerial *ex vivo* mittels unterschiedlicher Antigene markiert und nach Bestrahlung wieder in den Körper zurückgegeben. Die Überlegung hierbei ist, den Tumor aufgrund der neuen Antigene für das Immunsystem auffällig zu machen und dabei durch die Zerstörung der Tumorzellen *in vivo* auch TILs gegen natürlich bedeutsame tumorassoziierte Antigene zu stimulieren. Diese Ansätze sind jedoch nur sehr beschränkt wirksam, da die Tumorzellen kaum eine Immunreaktion stimulieren können. Die Ursachen liegen einerseits in der geringen Konzentration spezifischer Antigene. Andererseits führt das Fehlen kostimulatorischer Moleküle oder MHC Moleküle zu einer ineffizienten Präsentation.

Weiterhin spielt die zelluläre Immunantwort bei der Kontrolle einer Vielzahl von viralen Infekten, beispielsweise bei HIV Infektionen oder herpesviralen Infekten eine zentrale Rolle. Unter Berücksichtigung der Bedeutung der zellulären Immunantwort zur Kontrolle mikrobieller Infekte und Tumore werden derzeit vielfältige neue Impfstrategien zur Induktion antigenspezifischer T-Zellen getestet. Diese beinhalten den Einsatz von lebend attenuierten Viren und Bakterien, rekombinanten Lebendimpfstoffen (auf der Basis verschiedener rekombinanter Bakterien und Viren), partikulären Immunogenen, (Lipo-) Proteinen, (Lipo-) Peptiden und DNS-Impfstoffen. Zudem werden verschiedene Verabreichungsformen dieser Impfstoffgruppen, beispielsweise die Kombination unterschiedlicher Impfstoffe in "*Prime-Boost*" Verfahren und die kombinierte Gabe mit Adjuvanzien und Trägersubstanzen bezüglich ihrer Eignung zur Induktion einer T-Zellantwort hin getestet. Die meisten der genannten Impfvektoren sind im Prinzip geeignet, neben einer CD4⁺ T-Helfer, Zellantwort auch eine CD8⁺ T-zellvermittelte, zytotoxische Immunantwort hervorzurufen. Die Effizienz dieser Verfahren zur gezielten Induktion einer effizienten CD8⁺ T-Zellantwort ist jedoch bislang dadurch limitiert, dass bei sehr vielen, durch Mikroorganismen induzierten Erkrankungen und Tumoren die Zielepitope protektiver T-Zellen nicht, oder nur in geringer Zahl bekannt sind. Das gezielte Einbringen rational ausgewählter T-Zellepitope würde die Wirksamkeit und Effizienz der zuvor beschriebenen Impfvektoren erheblich steigern.

Insbesondere bei Impfstoffen und Impfvektoren mit einer begrenzten Kapazität zur Aufnahme von Fremdgenen (insbesondere DNS-Impfstoffe, Replikons, rekombinante bakterielle und virale Vektoren) oder Fremdepitopen (insbesondere Impfstoffen auf der Basis von Peptiden, Polypeptiden, Lipoproteinen und chimären partikulären Immunogenen) ist die Kenntnis relevanter Zielstrukturen der T-Zellantwort von entscheidender Bedeutung. Bei der ungezielten Expression einer großen Zahl von Epitopen kann es zudem durch (zum größten Teil unbekannte) Epitope zu einer Ablenkung der Immunantwort auf nicht relevante Ziele kommen.

Mit den derzeit verfügbaren immunologischen Techniken zur Identifizierung von Zielepitopen reaktiver T-Zellen werden APC üblicherweise in der Art verändert, dass sie zuvor ausgewählte Epitope von Polypeptiden auf ihrer Oberfläche präsentieren. Die veränderten APC werden hierbei zusammen mit autologen T-Zellen, oder solchen, die in der Erkennung von MHC-Molekülen mit den autologen T-Zellen übereinstimmen inkubiert und spezifische Reaktionen der T-Zellen (beispielsweise Proliferation, Zytokinausschüttung oder zytotoxische Aktivität) nach einer spezifischen Erkennung von Epitop/MHC Komplexen auf der Oberfläche von APC gemessen.

APC werden hierzu beispielsweise mit Peptiden oder Gemischen unterschiedlicher Peptide, die potenzielle oder bereits bekannte Zielepitope reaktiver T-Zellen beinhalten, inkubiert. Bei diesem experimentellen Ansatz werden Peptide direkt auf membranständige MHC-Proteine geladen. Alternativ können für Polypeptide, die potenzielle Zielepitope reaktiver T-Zellen beinhalten, kodierende Polynukleotide mittels Transduktion verschiedener rekombinanter Transfersysteme (Plasmide, nicht virale oder virale Vektoren) in APC eingebracht und die Polypeptide in den APC exprimiert werden. Diese werden dann im Zytoplasma der APC aufbereitet, auf MHC Proteine der Klassen I und II geladen und mit diesen zusammen auf der Oberfläche der APC präsentiert.

Alternativ kann eine Epitop-Beladung von MHC-Proteinen durch eine Inkubation von APC mit Polypeptiden, (Lipo-) Proteinen, oder (Lipo-) Proteinaggregaten erzielt werden. Zur Steigerung der Aufnahme dieser Polypeptide oder Polypeptidkomplexe können diese einer Behandlung mit physikalischen (Hitzedenaturierung) oder chemischen Verfahren (Natrium-dodecylsulfat (SDS)-, Harnstoff- oder Säurebehandlung) unterzogen werden. Weiterhin steigert die Fusion der Polypeptide mit CpG-haltigen Nukleinsäuren oder Polypeptiden des SV40 T-Antigens deren Aufnahme, Prozessierung und Epitoppräsentation. Polypeptide, die möglicherweise antigene Epitope tragen, werden zuvor entweder auf biochemischem Wege aus Organen, Geweben, Zellen, Mikroorganismen oder sonstigem biologischem Material aufgereinigt. Zudem können Polypeptide auf rekombinantem Wege beispielsweise mittels gentechnisch veränderter Zellen, Hefen, Bakterien oder Viren produziert und aus diesen aufgereinigt werden. Weiterhin eignet sich auch die Inkubation von APC mit vitalen oder abgetöteten polypeptidproduzierenden Bakterien, Hefen, Säuger- und Insektenzellen oder deren Lysaten zum Einbringen dieser Polypeptide in den MHC-Klasse-I und -II Antigenprozessierungs- und Präsentationsweg. Anschließend werden die in dieser Art vorbehandelten APC mit T-Zellen oder T-zellhaltigen Zellgemischen kokultiviert. Die Identifizierung von Polypeptiden, welche Zielepitope reaktiver T-Zellen enthalten, erfolgt durch die Bestimmung charakteristischer Reaktionen reaktiver T-Zellen (beispielsweise Proliferation, Zytokinausschüttung oder zytotoxische Aktivität) infolge einer spezifischen Erkennung von Epitop/MHC Komplexen auf der Oberfläche von APC. Ähnliches trifft auch zu für die Bestimmung von tumorassoziierten Antigenen, die durch tumorinfiltrierende Lymphozyten erkannt werden.

WO 00/57705 betrifft die Herstellung und Verwendung Antigen-spezifischer T-Zellen. Die Herstellung der Antigen-spezifischen T-Zellen erfolgt dabei mittels Co-Kultivierung der T-Zellen mit Tumorzellen, virusinfizierten Zellen oder Hybridomen aus dendritischen Zellen mit Tumorzellen. Die solchermaßen erzeugten Antigen-spezifischen T-Zellen werden prophylaktisch sowie therapeutisch gegen Tumor- oder Vieruserkrankungen verwendet. Ferner betrifft WO 00/57705 die Identifizierung von Antigenen mittels Beladung Antigen-präsentierender Zellen mit extrahierten Peptiden.

WO 00/67761 betrifft Verfahren und Zusammensetzungen, die eine Immunreaktion hervorrufen, sowie die prophylaktische Verwendung der Zusammensetzungen als Impfstoff zur Immunisierung und für die Therapie von Krebs, Infektionskrankheiten oder Autoimmunerkrankungen. Die Immunreaktion beruht dabei auf einem Expressionsvektor, der ein Polynukleotid umfasst, das spezifische Epitope kodiert, die Immunzell-spezifische, immunologische, Reaktionen hervorgerufen. Die Expression des Vektors erfolgt dabei u. a. in antigenpräsentierenden Zellen. Ferner betrifft WO 00/67761 Verfahren zur Identifizierung von Polynukleotiden, die für Epitope/Antigene kodieren, die eine Immunreaktion hervorrufen können, wobei die solchermaßen neu gefundenen Epitope/Antigene wiederum als Impfstoffe dienen können. Die Identifizierung erfolgt dabei durch Co-Inkubation mit autologen T-Zellen und anschließender Bestimmung spezifischer Reaktionen der T-Zellen.

Alternativ zu einzelnen zuvor ausgewählten Polypeptiden kann theoretisch auch eine große Zahl von Polypeptiden simultan bezüglich ihrer Erkennung durch T-Zellen getestet werden. Zu diesem Zweck werden mittels unterschiedlicher nichtviraler- und viraler Transfersysteme Polynukleotide einer Genbank, die beispielsweise für alle exprimierten Polypeptide einer Zelle kodiert, in APC eingebracht. Die modifizierten APC werden wiederum mit T-Zellen inkubiert und in die Vertiefungen von Mikrotiterplatten verteilt. Die Identifizierung von Zielepitopen oder Polypeptiden, welche Zielepitope reaktiver T-Zellen enthalten, erfolgt wiederum durch Bestimmung einer spezifischen Reaktion der T-Zellen. Da es sich um Gemische unterschiedlicher APC, die verschiedene Epitope präsentieren, handelt, werden die APC aus einer Vertiefung mit T-Zellerkennung erneut auf verschiedene Vertiefungen einer weiteren Mikrotiterplatte verteilt (*Limiting Dilution*) und erneut auf eine Erkennung durch spezifische T-Zellen getestet. Dieses Verdünnungs-Verfahren wird so lange wiederholt, bis statistisch in jeder Vertiefung mit meßbarer T-Zellreaktion nur noch Abkömmlinge einer Epitop-präsentierenden APC vorhanden sind.

Keines der bislang verfügbaren Verfahren zur Epitopsuche ist jedoch geeignet, um mit einem tragbaren Kosten- und Arbeitsaufwand die Zielepitope protektiver oder autoaggressiver T-Zellen in einer für die vielfältigen, dringend benötigten prophylaktischen, therapeutischen und diagnostischen Anwendungen erforderlichen Geschwindigkeit und Vollständigkeit zu bestimmen. Die Verwendung der oben genannten Verfahren zur schnellen und umfassenden Bestimmung von T-Zellepitopen scheitert in erster Linie an den nachstehend aufgeführten Problemen.

Die Peptidbeladung von membranständigen MHC-Proteinen auf APC stellt zwar eine sehr effiziente Methode zur Stimulation von T-Zellen dar; jedoch besteht die erhebliche Einschränkungen, dass mit dieser Methode nur bekannte Polypeptidsequenzen ausgetestet werden können. Die selbe Limitation besteht auch für die Verfahren, welche auf der Inkubation der APC mit gereinigten Polypeptiden, (Lipo-) Proteinen, (Lipo-) Proteinaggregaten, Zelllysaten oder apoptotischen, Polypeptid produzierenden Zellen basieren. Verfahren der Transformation von APC mit Polynukleotiden, die für diese Polypeptide kodieren, besitzen die selben Einschränkungen. Diese Beschränkung auf wenige bekannte Epitope oder Polypeptide kann aufgrund der Komplexität der biologischen Nachweissysteme nicht durch eine Erhöhung der Zahl an getesteten Polynukleotiden, Polypeptiden oder Zelllysaten umgangen werden. So erfordert beispielsweise die exakte Untersuchung der T-Zell-Erkennung eines einzigen Proteins mittlerer Größe von 20-40 kDa (etwa 200 bis 400 Aminosäuren) die Synthese und Austestung von mehr als 50 überlappenden Peptiden. Nach neuesten Schätzungen auf der Grundlage von Ergebnissen des Humanen Genom-Projektes zur Aufklärung des menschlichen Genoms beträgt die Gesamtzahl der menschlichen Gene etwa 30000. Die Gesamtzahl an spezifisch in einer differenzierten Zelle exprimierten Proteinen, welche alle putative Zielepitope reaktiver T-Zellen enthalten können, ist somit deutlich zu hoch, um mit den bislang verfügbaren molekularbiologischen bzw. biochemischen Methoden umfassend auf die Anwesenheit von T-Zellepitopen hin untersucht werden zu können.

Derzeit verfügbare *Screening-*Verfahren von Genbanken zur Suche nach T-Zellepitopen haben ebenfalls zwei entscheidende Einschränkungen. Aufgrund des sehr hohen zeitlichen Aufwands dieser Untersuchungen von mehreren Wochen und der begrenzten Lebensdauer der modifizierten APC und der T-Zellen in Kultur, können diese Untersuchungen in der Regel aufgrund der begrenzten Fähigkeit zur Zellteilung nicht mit primären APC und T-Zellen durchgeführt werden. Statt dessen erfordern diese Untersuchungen die Herstellung immortalisierter T-Zell- und APC-Populationen. In jedem Fall ist dieser Schritt sehr zeitaufwendig und aufgrund der Notwendigkeit übereinstimmender MHC-Muster in seiner Anwendung limitiert.

Durch die Verwendung immortalisierter T-Zelllinien findet zudem eine Reduktion der ursprünglich polyklonalen T-Zell-Population auf oligoklonale T-Zelllinien statt. Selbst bei der Untersuchung tumorinfiltrierender Lymphozyten, in denen einzelne T-Zellklone angereichert sind, müssen diese zum Zwecke der Untersuchung erst subkloniert werden.

Die Verwendung willkürlich ausgewählter Peptide und Polypeptide zur T-Zellsuche ist somit aufgrund der hohen Zahl potentieller Ziele einer T-Zellantwort und der MHC-Restriktion der spezifischen Epitop-Erkennung durch T-Zellen sowie dem extrem hohen Kosten- und Zeitaufwand zur Herstellung und Reinigung von Polypeptiden und zur Durchführung der bislang verfügbaren Verfahren zur Epitopsuche nicht praktikabel.

Zum Nachweis von T-Zellen mit einer bestimmten Antigenspezifität in einer Population von T-Zellen werden die T-Zellen mit APC, die spezifische Epitope präsentieren, inkubiert. Der Nachweis Antigen-spezifischer CD4⁺ T-Helferzellen erfolgt anschließend entweder durch die Bestimmung der Zytokinsekretion (mittels der FACS, ELISPOT oder ELISA Technik), durch Messung der T-Zellproliferation (mittels der Bestimmung des Einbaus von ³H Tritium in die DNS proliferierender Zellen) nach spezifischer Restimulation der T-Zellen mit Polypeptiden oder Epitopen geeigneter Länge oder dem Nachweis von Epitop-spezifischen T-Zellen mittels der Dimer- oder Tetramer Technologie.

Der Nachweis von spezifischen CD8⁺ T-Zellen (CTL) erfolgt durch eine Kokultivierung dieser Zellen mit Peptid beladenen APC und die Bestimmung der zytolytischen Aktivität der CD8⁺ CTL (beispielsweise mittels eines ⁵¹Chrom-Freisetzungstest) oder durch die Messung der IFN-γ Sekretion aus CTL (mittels der FACS, ELISPOT oder ELISA Technik). Alternativ können Epitop-spezifische T-Zellen wiederum mittels der Dimer- oder Tetramertechnologie spezifisch nachgewiesen werden.

Einer der Nachteile der heute verfügbaren Verfahren zum Nachweis von T-Zellen mit einer bestimmten Antigenspezifität liegt in ihrer geringen Sensitivität. Bisher ist es nur möglich, Reaktion der T-Zellen infolge einer Epitop-spezifischen Erkennung der APC zu messen. Dadurch ist die Sensitivität des Nachweises direkt von der Konzentration der Epitop-spezifischen T-Zellen abhängig. Im Falle des *Screenings* von Genbanken ist dies ein besonderer Nachteil. Aufgrund der relativ hohen Zahl an Polynukleotidfragmenten in einer Genbank ist die Kopienzahl einzelner Polynukleotidfragmente der Genbank nach Einbringen in die APC sehr gering.

Eine Vorstimulation der T-Zellpopulation mit epitoppräsentierenden APC erhöht die Konzentration der Zellen und damit die Empfindlichkeit des Nachweises. Für bestimmte Anwendungen, bei denen zwischen aktivierten und nicht aktivierten T-Zellen unterschieden werden soll, wie beispielsweise dem Nachweis autoagressiver T-Zellen bei Verdacht auf Multiple Sklerose, ist eine *in vitro* Restimulation jedoch nicht anwendbar.

Die Di- und Tetramertechnologien sind neue und elegante Verfahren zum Nachweis Epitop-spezifischer CD8⁺ und CD4⁺ T-Zellen. Beschränkungen dieser Verfahren für einen breiten Einsatz in der T-Zelldiagnostik sind jedoch in den sehr hohen Kosten für die Herstellung der Di- und Tetramere begründet. Zudem ist die Di- und Tetramertechnologie bislang nur für eine begrenzte Anzahl von MHC Typen, insbesondere für häufig in der Bevölkerung vertretene MHC-Klasse I Proteine verfügbar. Zudem erlaubt diese Technik nur den Nachweis definierter Epitop-spezifischer T-Zellen mit bekannter MHC-Restriktion. Die Austestung von T-Zellreaktivitäten gegen multiple Epitope ist mit diesem Verfahren nur mit einen großen Zeit- und Kostenaufwand möglich.

Daher liegt der vorliegenden Erfindung die Aufgabe zu Grunde, ein Verfahren zur Identifizierung von Epitopen, die von reaktiven T-Zellen spezifisch erkannt werden, bereitzustellen. Ferner liegt der vorliegenden Erfindung die Aufgabe zugrunde, Verfahren zum Nachweis Epitop-spezifischer T-Zellen bereitzustellen.

Die Aufgabe wird durch den in den Patentansprüchen definierten Gegenstand gelöst.

Die folgenden Figuren dienen der Erläuterung der Erfindung.

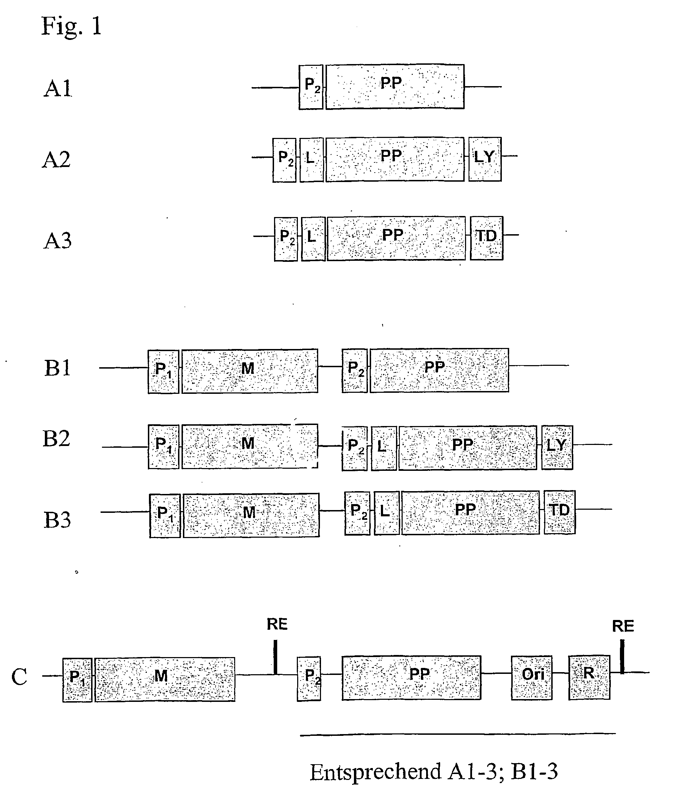

Figur 1 A und B zeigen in einer schematischen Darstellung die Anordnung der Promotoren und funktionell verbundenen Nukleinsäuren in den erfindungsgemäßen Vektoren. (P₂) kennzeichnet einen in APC konstitutiv aktiven Promotor, (PP) kennzeichnet beliebige Polypeptide, (P₁) kennzeichnet einen in APC durch den epitop-spezifischen Kontakt mit einer T-Zelle induzierbaren Promotor, (M) kennzeichnet einen Marker, (L) kennzeichnet ein Signalpeptid, (LY) kennzeichnet ein Endo/Lysosomales Targeting Signal, (TD) eine heterologe Transmembrandomäne, RE kennzeichnet Restriktionsschnittstellen, Ori bedeutet Replikationsursprung und R bedeutet Resistenzgen.

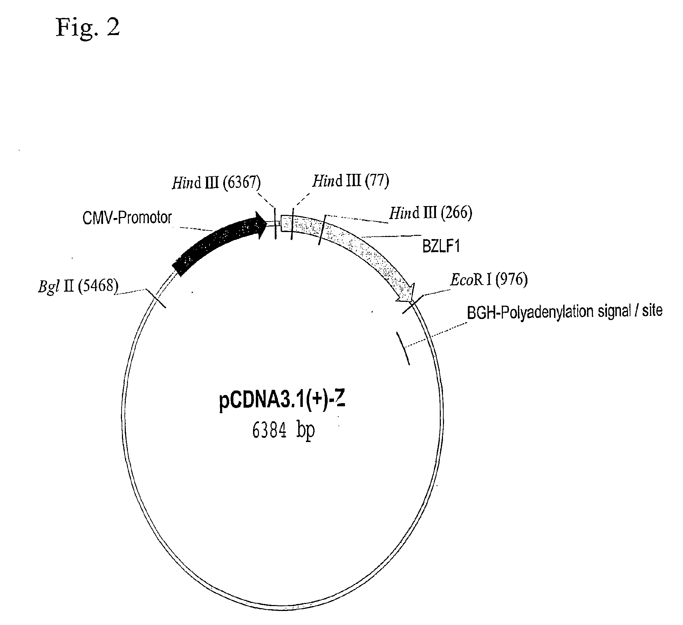

Figur 2 zeigt das erfindungsgemäße Vektorbackbone pcDNA3.1(+)-Z exemplarisch für Vektorbackbones zur Expression von Polypeptiden unter der Kontrolle eines in APC konstitutiv aktiven Promotors (P2). Dieses Plasmidbackbone enthält den kodierenden Bereich des Epstein-Barr Virus BZLF1 Proteins unter der transkriptionellen Kontrolle des in APC konstitutiv aktiven Cytomegalievirus (CMV)-Promotors.

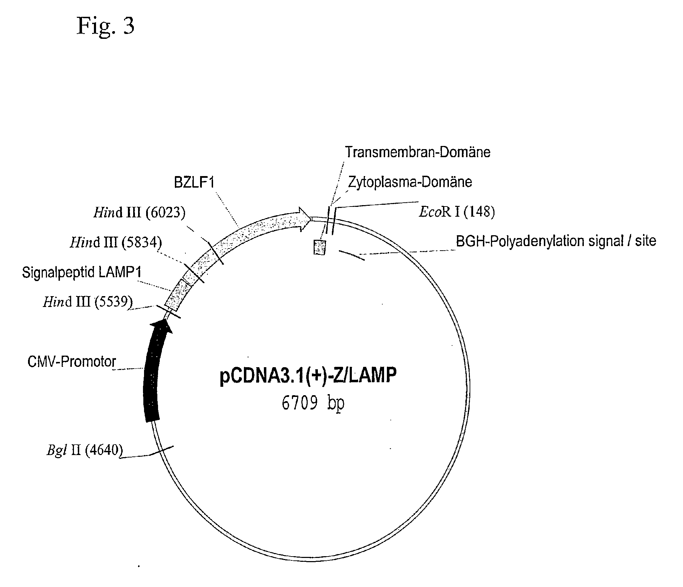

Figur 3 zeigt das erfindungsgemäße Vektorbackbone pcDNA3.1(+)-Z/LAMP exemplarisch für Vektorbackbones welche einen gezielten Transport der mittels des konstitutiven Promotors (P2) exprimierten Polypeptide in das Endolysosom vermitteln. Dieses Plasmidbackbone enthält den kodierenden Bereich des Epstein-Barr Virus BZLF1 Proteins, welcher im 5' Bereich mit dem kodierenden Bereich des LAMP-1 Signalpeptids und im 3' Bereich mit dem kodierenden Bereich der Transmembran- und cytoplasmatischen Domäne des humanen LAMP-1 Proteins gekoppelt ist. Der kodierende Bereich für das chimäre Protein steht unter der transkriptionellen Kontrolle des in APC konstitutiv aktiven Cytomegalievirus (CMV)-Promotors.

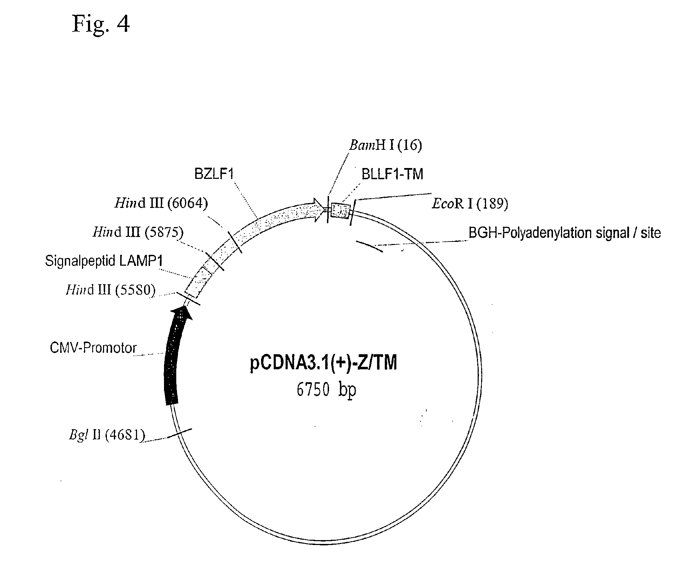

Figur 4 zeigt das erfindungsgemäße Vektorbackbone pcDNA3.1(+)-Z/TM exemplarisch für Vektorbackbones welche eine Membranverankerung der mittels des konstitutiven Promotors (P2) befindlichen Polypeptide gewährleisten. Dieses Plasmidbackbone enthält den kodierenden Bereich des Epstein-Barr Virus BZLF1 Proteins, welcher im 5' Bereich mit dem kodierenden Bereich des LAMP-1 Signalpeptids und im 3' Bereich mit dem kodierenden Bereich der Transmembrandomäne des Epstein-Barr Virus gp220/350 Hüllproteins gekoppelt ist. Der kodierende Bereich für das chimäre Protein steht unter der transkriptionellen Kontrolle des in APC konstitutiv aktiven Cytomegalievirus (CMV)-Promotors.

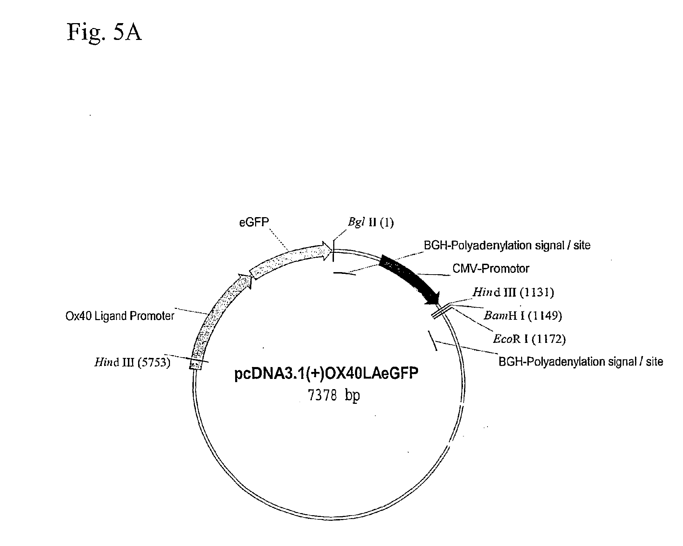

Die Figuren 5A-D zeigen exemplarisch vier erfindungsgemäße Vektorbackbones, umfassend einen spezifisch in antigenpräsentierenden Zellen durch den Epitop-spezifischen Kontakt mit einer T-Zelle induzierbaren ersten Promotor (P1), eine mit diesem ersten Promotor funktionell verbundene Nukleinsäure kodierend ein Markergen, einen in antigenpräsentierenden Zellen konstitutiven zweiten Promotor (T2), und eine mit diesem zweiten Promotor funktionell verbundene Nukleinsäure.
Figur 5A zeigt das erfindungsgemäße Vektorbackbone pcDNA3(+)OX40LAeGFP. Dieses Plasmidbackbone enthält das Gen für das *enhanced green fluorescent Protein* (eGFP) als Marker unter der transkriptionellen Kontrolle des in APC nach einer epitopspezifischen Erkennung durch eine T-Zelle induzierbaren OX40-Ligand Promotors.

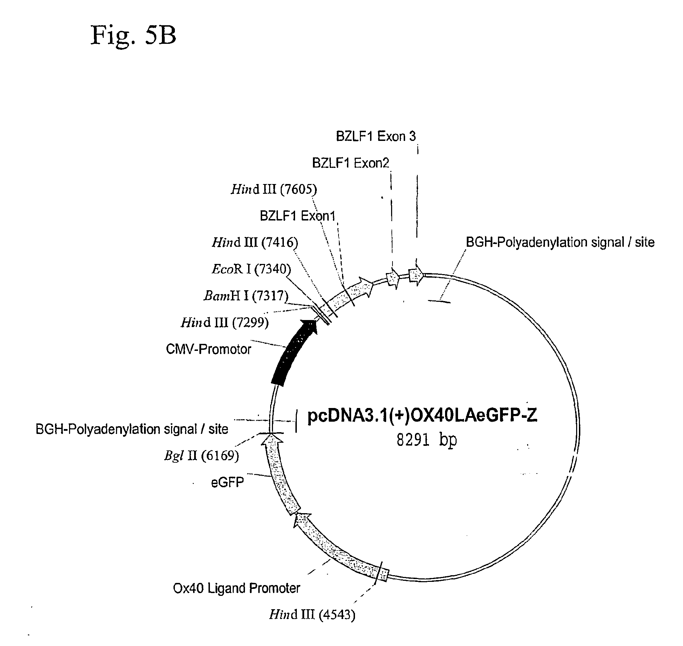

Figur 5B zeigt den erfindungsgemäßen Vektor pcDNA3(+)OX40LAeGFP-Z. Dieses Plasmid beinhaltet das Gen für das eGFP als Marker unter der Kontrolle des induzierbaren OX40-Ligand Promotors. Zudem beinhaltet dieses Plasmid den kodierenden Bereich des Epstein-Barr Virus BZLF1 Proteins unter der transkriptionellen Kontrolle des in APC konstitutiv aktiven Cytomegalievirus (CMV)-Promotors.

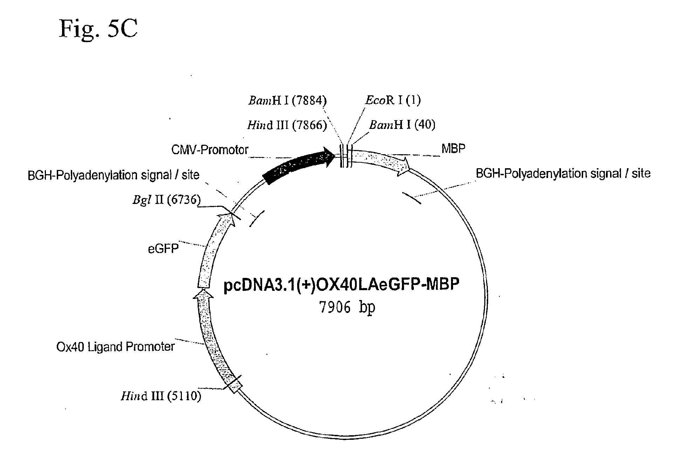

Figur 5C zeigt den erfindungsgemäßen Vektor pcDNA3(+)OX40LAeGFP-MBP. Dieses Plasmid beinhaltet das Gen für eGFP als Marker unter der Kontrolle des induzierbaren OX40-Ligand Promotors. Zudem beinhaltet dieses Plasmid den kodierenden Bereich des humanen Myelinbasischen Proteins (MBP) unter der transkriptionellen Kontrolle des in APC konstitutiv aktiven Cytomegalievirus (CMV)-Promotors.

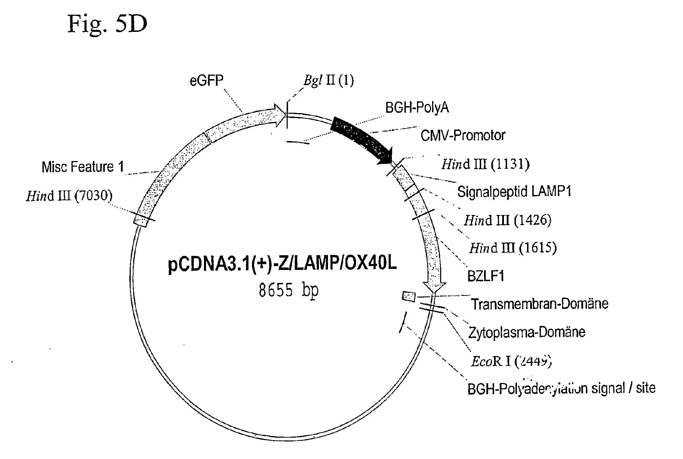

Figur 5D zeigt den erfindungsgemäßen Vektor pcDNA3.1(+)Z/LAMP/OX40L. Dieses Plasmidbackbone enthält den kodierenden Bereich des Epstein-Barr Virus BZLF1 Proteins, welcher im 5' Bereich mit dem kodierenden Bereich des LAMP-1 Signalpeptids und im 3' Bereich mit dem kodierenden Bereich der Transmembrandomäne des Epstein-Barr Virus gp220/350 Hüllproteins gekoppelt ist. Der kodierende Bereich für das chimäre Protein steht unter der transkriptionellen Kontrolle des in APC konstitutiv aktiven Cytomegalievirus (CMV)-Promotors. Zudem enthält dieses Plasmid das Gen für eGFP als Marker unter der Kontrolle des induzierbaren OX40-Ligand Promotors.

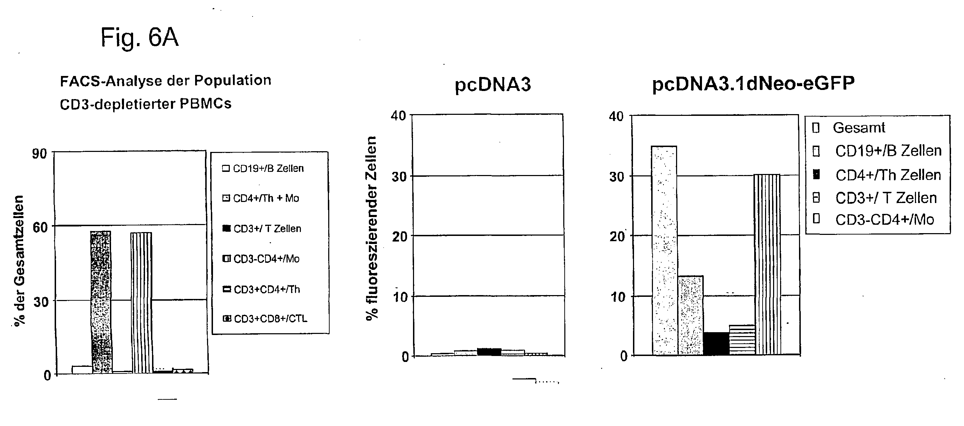

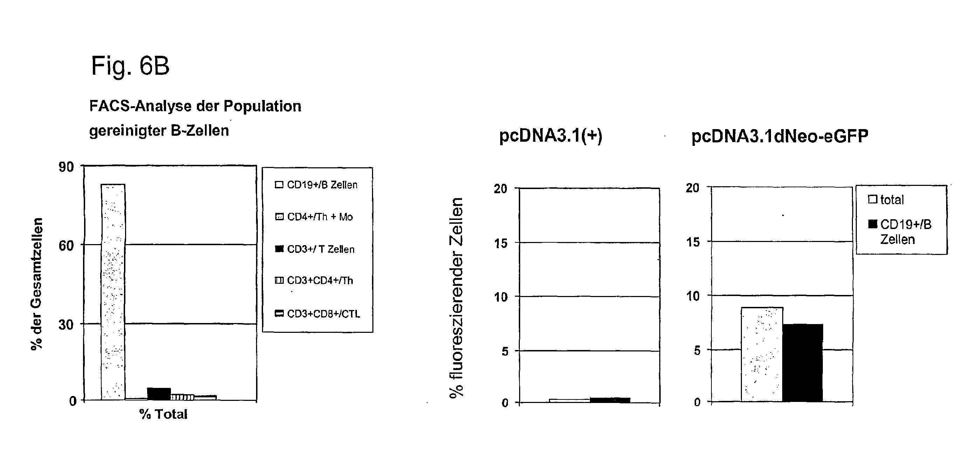

Die Figuren 6 A-B zeigen die Effizienz des Plasmidtransfers in APC mittels der Nukleovektor^{™} Technologie. Hierbei wurden entweder Populationen CD3⁺ depletierter PBMCs (Figur 6A) oder gereinigter B-Zellen (Figur 6B) mittels der Miltenyi Zellseparations-Technologie gewonnen und die Zusammensetzung der erhaltenen Zellpopulationen mittels der FACS Technologie unter Verwendung geeigneter Antikörper bestimmt (Figur 6A,B; jeweils linke Abbildung). Je 3 x 10⁵ gereinigte CD3⁺ Zell-depletierte PBMC oder isolierte B-Zellen wurden dann mit 5 µg des erfindungsgemäßen Vektors pcDNA3.1(+)dNeo-eGFP (Figur 6A,B; jeweils rechte Abbildung) und zur Kontrolle mit dem pcDNA3.1(+) Vektor (Figur 6A,B; jeweils mittlere Abbildung). mittels der Nukleovektor^{™} Technologie transfiziert. Die Effizienz des Nukleinsäuretransfers in die jeweils aufgeführten Zellpopulaionen wurde 17 Stunden nach Transfektion mittels eines FACS Gerätes durch die Messung der Anzahl fluoreszierender Zellen (Nachweis eines GFP-Reporters) bestimmt.

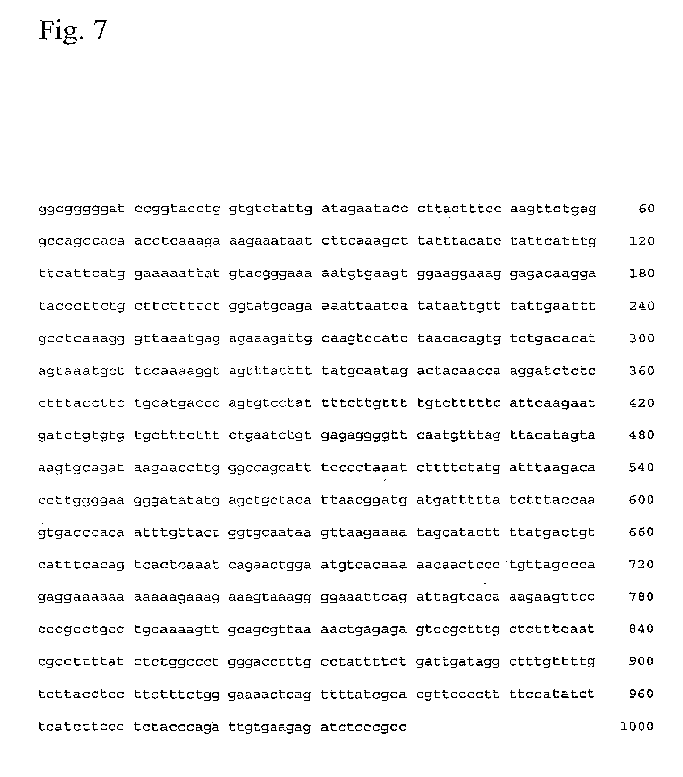

Figur 7 zeigt die Nukleinsäuresequenz des mittels PCR amplifizierten humanen OX40 Ligand Promotors (SEQ ID NO:1).

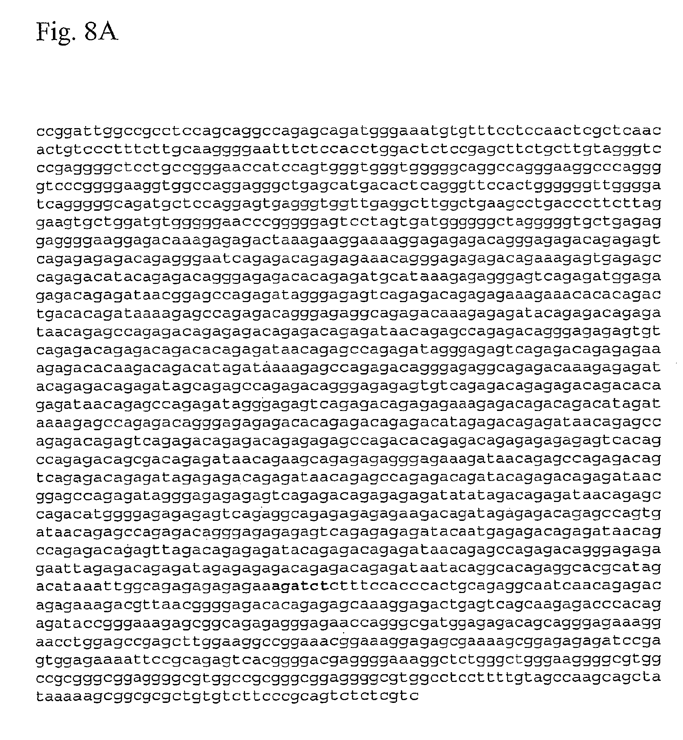

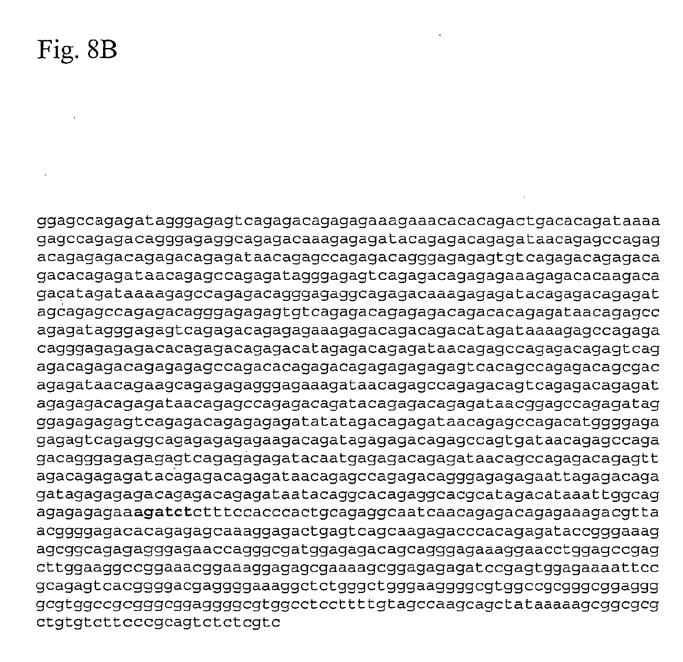

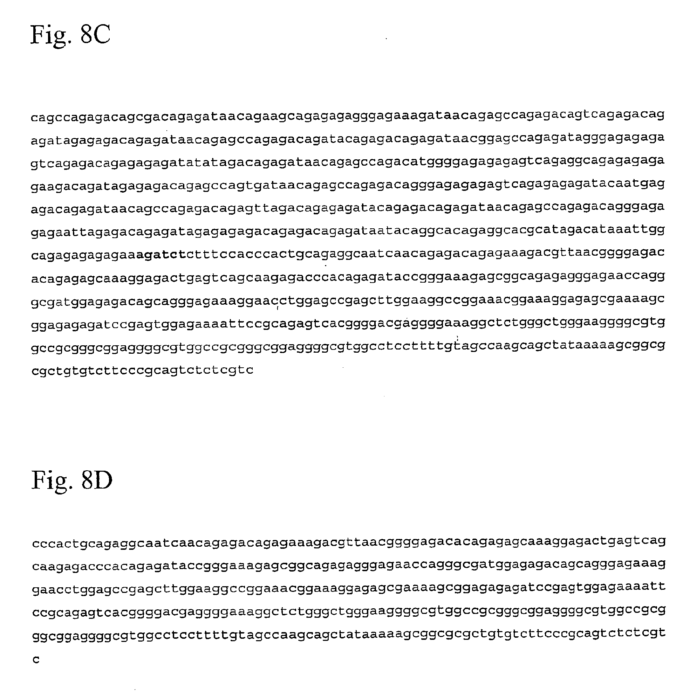

Die Figuren 8 A-D zeigen die Nukleinsäuresequenzen des mittels PCR amplifizierten humanen 4-1BB Ligand Promotors (4-1 BBL) und dreier fortschreitend im 5' Bereich verkürzter Varianten des humanen 4-1BB Ligand Promotors. Fig. 8A: lange Variante (V1) (SEQ ID NO:2); Fig. 8B: C-terminal verkürzte Variante (V2) (SEQ ID NO:3); FIG. 8C: C-terminal weiter verkürzte Variante (V3).(SEQ ID NO:4); Fig. 8D: C-terminal noch weiter verkürzte Variante (V4) (SEQ ID NO:5). Die in den Sequenzen enthaltene *Bgl*II Schnittstelle ist gekennzeichnet. Diese Schnittstelle wurde durch einen G zu A Austausch mutagenisiert.

Der hier verwendete Begriff "genomisch" bezeichnet die Gesamtheit oder Fragmente des genetischen Materials eines Organismus.

Der hier verwendete Begriff "Polynukleotid" bezeichnet die polymere Form von Nukleotiden beliebiger Länge, präferentiell Desoxyribonukleotiden (DNS) oder Ribonukleotiden (RNS). Dieser Begriff bezeichnet nur die Primärstruktur des Moleküls. Der Begriff beinhaltet doppel- und einzelsträngige DNS und RNS sowie Antisense-Polynukleotide.

Der hier verwendete Begriff "Kontrollsequenzen" bezeichnet Polynukleotidsequenzen, die erforderlich sind für die Expression von kodierenden Polynukleotidsequenzen, mit denen sie verknüpft sind. Kontrollsequenzen befinden sich auf dem Genom von Organismen und regulieren die Transkription von Genen, das heißt die Synthese vom mRNA. Kontrollsequenzen befinden sich auch auf mRNA-Polynukleotiden und regulieren die Translation, das heißt die Synthese von Polypeptiden. Die Eigenschaften derartiger Kontrollsequenzen unterscheiden sich in Abhängigkeit vom Wirtsorganismus; in Prokaryoten beinhalten solche Kontrollsequenzen üblicherweise einen Promotor, eine ribosomale Eintrittsstelle, und eine Transkriptionsterminations-Sequenz; in Eukaryoten beinhalten solche Kontrollsequenzen üblicherweise Promotoren und Transkriptionsterminations-Sequenzen. Der Begriff "Kontrollsequenz" beinhaltet zudem alle Polynukleotide, deren Anwesenheit für die konstitutive oder induzierbare Expression von kodierenden Sequenzen erforderlich sind und schließt zudem zusätzliche Komponenten mit ein, deren Anwesenheit für die Expression eines Polypeptids förderlich ist, wie z.B. Leadersequenzen und die Sequenzen eines Fusionspartners.

Der hier verwendete Begriff "Polypeptid" bezeichnet ein Polymer von Aminosäuren beliebiger Länge. Der Begriff Polypeptid umfasst auch die Begriffe (Ziel-) Epitop, Peptid, Oligopeptid, Protein, Polyprotein, und Aggregate von Polypeptiden. Ebenso sind in diesem Begriff Polypeptide eingeschlossen, die posttranslationelle Modifikationen wie z.B. Glykosylierungen, Acetylierungen, Phosphorylierungen und ähnliche Modifikationen aufweisen. Weiterhin umfasst dieser Begriff zum Beispiel Polypeptide, die ein oder mehrere Analoge von Aminosäuren (z.B. unnatürliche Aminosäuren), Polypeptide mit substituierten Verknüpfungen, sowie anderen Modifikationen, die Stand der Technik sind, aufweisen, unabhängig davon ob sie natürlicherweise vorkommen oder nicht natürlichen Ursprungs sind.

Der hier verwendete Begriff "Epitop" bezeichnet den Bereich eines Polypeptides, das antigene Eigenschaften besitzt und beispielsweise als Erkennungsstelle von T-Zellen oder Immunglobulinen dient. Im Sinne dieser Erfindung sind Epitope beispielsweise solche Bereiche von Polypeptiden, die von Immunzellen wie z.B. CD4⁺ T-Helferzellen, CD8⁺ zytotoxischen T-Zellen, CD161⁺ NKT Zellen oder CD4⁺CD25⁺ regulatorische T-Zellen erkannt werden. Ein Epitop kann 3 oder mehr Aminosäuren umfassen. Üblicherweise besteht ein Epitop aus mindestens 5 bis 7 Aminosäuren oder, was häufiger ist, aus mindestens 8-11 Aminosäuren, oder aber aus mehr als 11 Aminosäuren, oder aber aus mehr als 20 Aminosäuren, seltener sogar aus mehr als 30 Aminosäuren. Der Begriff "Epitop" umfasst sowohl lineare als auch eine für das Epitop einzigartige räumliche Konformation. Die räumliche Konformation ergibt sich aus der Abfolge der Aminosäuren im Bereich des Epitops.

Der hier verwendete Begriff "Mikroorganismus" bezeichnet Viren sowie prokaryotische und eukaryotische Mikroben wie z.B. Archebakteriterien," Bakterien, Einzeller und Pilze; die letztere Gruppe umfasst beispielsweise Hefen und filamentöse Pilze.

Der hier verwendete Begriff "Vektor" oder "Gentransfervektor" bezeichnet natürlich vorkommende oder künstlich erschaffene Organismen und Konstrukte zur Aufnahme, Vermehrung, Expression oder Übertragung von Nukleinsäuren in Zellen. Vektoren sind beispielsweise Viren, wie Lentiviren, Retroviren, Adenoviren, Adeno-assoziierte Viren, Pockenviren, Alphaviren, Bakuloviren, Tollwutviren oder Herpesviren. Vektoren sind beispielsweise auch Bakterien, wie Listerien, Shigellen oder Salmonellen. Vektoren sind aber beispielsweise auch nackte DNS, wie bakterielle Plasmide und MIDGES, von Viren abgeleitete Plasmide, Phagemide, Cosmide, Bakteriophagen oder künstlich hergestellte Nukleinsäuren wie artifizielle Chromosomen. Vektoren besitzen die Fähigkeit, sich in einer Zelle autonom zu vermehren. Der Vektor kann zudem ein oder mehrere zusätzliche Polynukleotide in der Art beinhalten, dass dieses repliziert und/oder exprimiert wird. Vektoren können zudem einen oder mehrere Selektionsmarker enthalten.

T-Zellen im Sinne der Erfindung sind Lymphozyten mit regulatorischen oder zytolytischen Eigenschaften, wie beispielsweise CD4⁺ T-Helferzellen, CD161⁺ NKT Zellen, CD8⁺ zytotoxische T-Zellen und CD4⁺CD25⁺ regulatorische T-Zellen.

Der verwendete Begriff "antigenpräsentierende Zelle" (APC) umfasst Zellen, die in der Lage sind Polypeptide aufzunehmen, zu prozessieren und Bruchstücke dieser Polypeptide (Epitope) dem Immunsystem in Verbindung mit MHC I und MHC II Proteinen zu präsentieren. Insbesondere umfasst der Begriff "antigenpräsentierende Zelle" dendritische Zellen (z.B. Langerhans Zellen), Monozyten, Makrophagen, B-Zellen aber auch vaskuläre endotheliale Zellen und verschiedene epitheliale, mesenchymale Zellen, sowie Microgliazellen des Gehirns.

Der Begriff "gekoppelt" bezeichnet eine Anheftung mittels kovalenter Bindung oder starker, nichtkovalenter Wechselwirkungen (z.B. hydrophobe Wechselwirkungen, Wasserstoff Brückenbindungen, etc.). Kovalente Bindungen können beispielsweise, Ester, Äther, Phosphoester, Amide, Peptide, Imide, Kohlenstoff-Schwefel-Bindungen, Kohlenstoff-Phophat-Bindungen oder ähnliche Bindungen sein.

Ein Aspekt der vorliegenden Erfindung betrifft Vektoren, umfassend einen spezifisch in APC durch den epitop-spezifischen Kontakt mit einer T-Zelle induzierbaren ersten Promotor (P₁), eine mit diesem ersten Promotor funktionell verbundene Nukleinsäure kodierend ein Markergen, einen in APC konstitutiven zweiten Promotor (P₂), und eine mit diesem zweiten Promotor funktionell verbundene Nukleinsäure. Vorzugsweise enthält der Vektor ferner einen bakteriellen Replikationsursprung (ori) und ein Resistenzgen. Ferner kann der erfindungsgemäße Vektor geeignete Erkennungssequenzen für Restriktionsendonukleasen enthalten, welche den konstitutiven zweiten Promotor (P₂), und die mit diesem zweiten Promotor funktionell verbundene Nukleinsäuresequenz, sowie den bakteriellen Replikationsursprung und die kodierende Sequenz für die bakterielle Resistenz flankieren. Ferner kann der erfindungsgemäße Vektor einen dritten Promotor enthalten, der funktionell mit einem weiteren Markergen verbunden ist.

Die für einen Marker kodierenden Polynukleotide stehen unter der Kontrolle eines in APC infolge einer Epitop-spezifischen Erkennung mittels einer T-Zelle induzierbaren Promotors (P₁). Im Sinne der Erfindung geeignete Marker sind beispielsweise, aber nicht ausschließlich, leicht detektierbare Polypeptide oder Polypeptidfragmente sowie beliebig modifizierte Abkömmlinge davon, die durch einfache enzymatische Reaktionen oder immunologische Techniken oder aufgrund ihrer Fluoreszenz nachgewiesen werden können. Beispiele für autofluoreszente Marker sind das Vitality^{™} humane rekombinante (hr)GFP (Stratagene, Amsterdam, Holland), das "green-fluoreszent" Protein (GFP) (BD Clontech, Heidelberg), FACS-optimierte Varianten des GFP, das "blue-fluoreszent" Protein (BFP), das "DsRed fluorescent Protein" (BD Clontech), das "red-fluoreszent" Protein (RFP), das "yellow-fluoreszent" Protein (YFP), das "Cyan fluoreszent" Protein (CFP) (BD Clontech) oder Derivate dieser Proteine, die eine erhöhte Fluoreszenz aufweisen, wie das "enhanced green-fluoreszent" Protein eGFP (BD Clontech), das "enhanced blue-fluoreszent" Protein eBFP, das "enhanced red-fluoreszent" Protein eRFP, das "enhanced Cyan-fluorescent" Protein eCFP (BD Clontech) oder das "enhanced yellow-fluoreszent" Protein eYFP (BD Clontech). Beispiele für Marker mit enzymatischer Aktivität sind beispielsweise die Luziferase (LUC) (BD Clontech), die alkalische Phosphatase (AP) (BD Clontech), die sekretorische alkalische Phosphatase (SEAP) (BD Clontech), die Chloramphenicol Acetyltransferase (CAT) (Promega, Mannheim), die Photinus-Luciferase (BD PharMingen), die β-Glucuronidase (GUS) (Research Diagnostics, New York, USA), die Renilla Luciferase (Promega) und die β-Galaktosidase (β-Gal) (BD Clontech). Beispiele für mittels immunologischer Verfahren leicht nachweisbare Marker sind zudem beliebige intrazelluläre und membranständige Polypeptide, die in der durch die erfindungsgemäßen Vektoren modifizierten APC natürlicherweise nicht vorkommen und mittels gängiger immunologischer oder biochemischer Verfahren, beispielsweise mittels polypeptidspezifischer Antikörper detektierbar sind. So eignen sich beispielsweise, aber nicht ausschließlich, die membranständigen murinen Proteine CD4, CD5, CD8a, CD11b, CD11c, CD19, CD43, CD45, CD62L, CD90, aber auch die Proteine CD4, CD8a, CD45, CD45RA, und CD134 (OX40) der Ratte als Marker, sofern sie keine störenden Kreuzreaktivität mit den entsprechenden humanen Oberflächenproteinen aufweisen. In Analogie eignen sich humane Oberfächenproteine, wie beispielsweise CD2, CD3, CD4, CD8, CD11b, CD14, CD15, CD16, CD19, CD22, CD27, CD30, CD45RO, CD45RA, CD56, CD69, CD138 als Marker für den Nachweis von Zielepitopen reaktiver T-Zellen mittels des erfindungsgemäßen Verfahrens bei nicht humanen Vertebraten. Zum Nachweis all dieser Proteine mittels der FACS-Technologie sind Fluoreszenz- (beispielsweise R-Phycoerythrin (R-PE), Peridin-Chlorophyll c (PerCP), Fluoescein (FITC), Texas Red (TX), Allophycocyanin (APC), Tandem PE-TX, Tandem PE-Cy5, PE-Cy7, Tandem APC-Cy7) gekoppelte primäre und sekundäre Antikörper geeignet. Diese sind entweder kommerziell erhältlich (beispielsweise von dem Firmen Becton Dickinson, Dako, Coulter) oder können mittels kommerziell erhältlicher Kits nach Anleitung des Herstellers hergestellt werden. Weiterhin eignen sich beispielsweise mikrobielle, insbesondere virale Hüllproteine, wie beispielsweise aber nicht ausschließlich die Hüllproteine des humanen Immundefizienzvirus (HIV), des Affenimmundefizienzvirus (SIV), der humanen T-Zell Leukämieviren (HTLV), und des Vesikulären Stomatitis Virus (VSV) als Marker. Als Marker sind aber auch Hüllproteine und Strukturproteine beliebiger Viren und Bakterien geeignet. Die zu untersuchenden Probanden/Patienten dürfen jedoch natürlicherweise keine Antikörper gegen diese Markerproteine im Serum aufweisen.

Als Marker sind zudem Polypeptide geeignet, die zelltransformierenden Eigenschaften aufweisen. Beispiele für derartige Polypeptide sind virale Proteine wie die adenoviralen Proteine E1A und E1B, die E6 und E7 Proteine des humanen Papillomvirus, das große T-Antigen des SV 40 Virus, das LMP1 Protein des Epstein Barr Virus (EBV) sowie das X Antigen des Hepatitis B Virus. Zudem können auch andere nichtvirale Polypeptide mit transformierenden Eigenschaften verwendet werden.

Als Marker eignen sich insbesondere auch Polypeptide, die natürlicherweise nach einer Epitopspezifischen Erkennung einer APC durch eine T-Zelle in der APC in ihrer Expression meßbar gesteigert werden. Beispiele für diese Polypeptide sind der OX40 Ligand (OX40L) und der 4-1BB Ligand (4-1BBL) (Ohshima et al. 1997, J. lmmunol. 159, 3838-3848; den Haan and Bevan; 2000, PNAS 97, 12950-12952). Geeignet sind zudem die kostimulatorischen Proteine B7.1 (CD80), B7.2 (CD86) und der Fas Ligand (FasL). Geeignet im Sinne der Erfindung ist zudem jedes beliebige Polypeptid, dessen Expression infolge der spezifischen Erkennung eines zusammen mit MHC Proteinen auf der Oberfläche von APC präsentierten Epitops in derselben APC messbar gesteigert oder reduziert wird.

Weiterhin sind auch Polypeptide als Marker geeignet, die durch eine beliebige Kombination verschiedener natürlich vorkommender Polypeptide erzeugt wurden, sowie nicht in der Natur vorkommende Polypeptidsequenzen. Diese Polypeptide sind dann als Marker geeignet, wenn sie durch immunologische, (bio)chemische oder physikalische Verfahren im Zytoplasma oder auf der Oberfläche der mit den erfindungsgemäßen Transfersystemen modifizierten APC meßbar sind.

Die Expression des Markers steht unter der Kontrolle eines in APC durch den epitopspezifischen Kontakt mit einer T-Zelle induzierbaren Promotors (erster Promotor, P₁). Geeignet im Sinne der Erfindung ist hierbei jeder Promotor, der infolge einer spezifischen Interaktion des T-Zellrezeptors (TCR) einer spezifischen T-Zelle mit einem in Verbindung mit MHC-Proteinen auf der Oberfläche der durch den erfindungsgemäßen Vektor modifizierten APC präsentierten Peptid in der APC angeschaltet oder in seiner Aktivität signifikant gesteigert wird. Beispiele für solche Promotoren sind die Promotoren für den OX40 Liganden (OX40L) und den 4-1BB Liganden (4-1BBL) (den Haan and Bevan; 2000). Geeignet sind zudem die Promotoren für die kostimulatorischen Proteine B7.1 (CD80), B7.2 (CD86) und der Promotor für den Fas Liganden (FasL). Geeignet im Sinne der Erfindung ist zudem jeder beliebige Promotor, der infolge der spezifischen Erkennung eines zusammen mit MHC Proteinen auf der Oberfläche von APC präsentierten Epitops durch eine spezifische T-Zelle in derselben APC angeschaltet oder in seiner Aktivität messbar gesteigert wird.

Geeignete Promotoren im Sinne der Erfindung sind ferner solche, welche infolge einer spezifischen Interaktion des T-Zellrezeptors (TCR) einer spezifischen T-Zelle mit einem in Verbindung mit MHC-Proteinen auf der Oberfläche der durch den erfindungsgemäßen Vektoren modifizierten APC präsentierten Peptid in der APC abgeschaltet oder deren Aktivität meßbar reduziert werden. Hierbei kann die Abnahme der Expression eines natürlicherweise von APC produzierten Polypeptids aber auch die Abnahme der Expression eines Markers in der APC infolge eines Epitop-spezifischen Kontakts mit einer T-Zelle als Selektionskriterium dienen.

Die erfindungsgemäßen Vektoren kodieren für einen Marker oder eine Kombination von zwei oder mehreren unterschiedlichen Markern, die unter der Kontrolle desselben oder verschiedener, in der oben beschriebenen Weise, induzierbaren ersten Promotoren stehen. Wenn zwei oder mehr Marker auf den erfindungsgemäßen Vektoren vorhanden sind, muß nur das erste Markergen unter der transkriptionellen Kontrolle eines in APC induzierbaren Promotors stehen. Die Expression der weiteren Marker unter der Kontrolle eines konstitutiven Promotors eignet sich beispielsweise zur Überprüfung der Transfektions/Transformationseffizienz der mit den erfindungsgemäßen Vektoren bzw. Transfersystemen behandelten APC.

Der erfindungsgemäße Vektor enthält ferner einen zweiten Promotor (P₂), der in APC eine konstitutive Expression der mit ihm funktionell verbundenen Nukleinsäure bewirkt. Gene von Viren, die Säuger infizieren, werden oftmals sehr effizient in Säugerzellen exprimiert und weisen ein breites Wirtsspektrum auf. Deshalb sind die zugehörigen viralen Kontrollsequenzen für die Expression von Gensequenzen in Säugerzellen als Promotor P₂ besonders geeignet. Einige Vertreter geeigneter viraler Kontrollsequenzen sind beispielsweise der frühe SV40 Promotor der Cytomegalovirus (CMV) Promotor der *Respiratory Syncytial Virus* (RSV) Promotor, der Maus Mamma Tumorvirus (MMTV) LTR Promotor, der humane Immundefizienz Virus Typ 1 (HIV-1) LTR Promotor, der Adenovirus *major late*-Promotor (Ad MLP) und der Herpes Simplex Virus (HSV) Promotor und davon abgeleitete Promotorsequenzen. Ferner sind auch Promotoren nicht viraler Gene, wie z.B. der murine 3-Phosphoglycerat-Kinase (PGK) Promotor, der humane PGK-1 Promotor, der humane Ubiquitin C-Promotor, der humane EF-1α Promotor, der humane β-Caseinpromotor, der murine Metallothionein Promotor, der humane Actin 5c Promotor oder der humane ICI Promotor zur effizienten Expression von Polynukleotiden in Säugern geeignet. Wenn sich die Kontrollsequenzen (1) für die konstitutive Expression des Polypeptids und die (2) induzierbare Expression des Marker auf einem Vektor befinden, sind nur solche Kontrollsequenzen für die konstitutive Expression des Polypeptids geeignet, welche die Kontrollsequenzen für die induzierbare Expression des Markers in ihrer erfindungsgemäßen Funktionalität nicht wesentlich beeinträchtigen.

Die beiden funktionellen Bereiche (1) für die konstitutive Expression des Polypeptids und die (2) induzierbare Expression des Marker befinden sich üblicherweise auf einem Vektor. Jedoch können sich diese beiden funktionellen Bereiche auch auf getrennten Vektoren befinden, die dann gemeinsam in eine APC eingebracht werden.

Die mit dem zweiten, konstitutiven Promotor funktionell verbundene Nukleinsäure kodiert für Polypeptide, die bekannte oder putative Ziele reaktiver T-Zellen darstellen oder beinhalten. Die Nukleinsäure kann dabei eine natürlicherweise vorkommende Sequenz oder willkürliche Sequenz aufweisen. Die Nukleinsäuresequenz kann aber beispielsweise auch aus einer beliebigen genomischen oder cDNS- Bank stammen.

Sollen die erfindungsgemäßen Vektoren zur Suche nach Epitopen verwendet werden, können beliebige Nukleinsäuren, welche für Polypeptide mit unbekannten, potentiellen Zielepitopen reaktiver T-Zellen kodieren, beispielsweise Polynukleotide einer cDNA-Bank oder einer genomischen Bank, unter die Kontrolle des konstitutiven Promotors (P₂) kloniert werden. Die cDNA-Bank kann z.B. eine speciesspezifische, erregerspezifische, gewebespezifische, entwiclclungsspezifische, oder eine subtraktive sein. Eine Größenbeschränkung hinsichtlich der klonierten Polynukleotide besteht nicht. Die klonierten Polynukleotide können eine beliebige Länge aufweisen, z.B. können sie weniger als 20 Nukleotide umfassen, oder, was häufiger ist, 20 bis 100 Nukleotide, aber auch 100 bis 500, 500 bis 1500 Nukleotide, aber auch bis zu 5000 Nukleotiden, seltener bis zu 10000 Nukleotiden, aber auch mehr als 10000 Nukleotide.

Sollen die erfindungsgemäßen Vektoren zum Nachweis Epitop-spezifischer T-Zellen verwendet werden, können Nukleinsäuren, welche für Polypeptide mit bekannten Zielepitopen reaktiver T-Zellen kodieren, unter die Kontrolle des konstitutiven Promotors (P₂) kloniert werden. Die klonierten Polynukleotide können eine beliebige Länge aufweisen, z.B. können sie weniger als 20 Nukleotide umfassen, oder, was häufiger ist, 20 bis 30 Nukleotide, aber auch 30 bis 100 Nukleotide, 100 bis 500 Nukleotide, aber auch bis zu 1000 Nukleotiden, seltener bis zu 10000 Nukleotiden.

Die verwendeten beliebigen oder bekannten Nukleinsäuren können von Menschen oder Säugetieren stammen, aber auch von beliebigen Tieren, Parasiten oder Mikroorganismen, z.B. Bakterien oder Viren. Sie können aber auch Pflanzen oder Algen entstammen. Sie können zudem Prionproteinen entstammen.

Beispiele für Viren, deren Polypeptide oder Fragmente die im erfindungsgemäßen Vektor enthaltenen Nukleinsäuresequenzen kodieren sind nachfolgend aufgelistet. Besonders bedeutende virale Erreger mit zum Teil (human)pathogenen Eigenschaften sind beispielsweise, aber nicht ausschließlich, Polioviren, Coxsachie-Viren, Echo-Viren, Enteroviren, Rhino-Viren, Orthomyxo-Viren (insbesondere Typ A, B, C Influenza-Viren), Paramyxo-Viren (insbesondere Parainfluenza-Viren, Mumpsviren, Masern-Viren, Respiratorische Syncytial-Viren (RS-Virus), Corona-Viren, Flavi- (insbesondere Gelbfieber-, Dengue-, Japan B-Enzephalitis-, Zeckenenzephalitis (FSME)-Viren), das Hepatitis C Virus (HCV)), Toga (insbesondere Alpha- und Rubiviren)- und Bunya (insbesondere die Bunya-, Hanta-, Nairo-, Phlebo- und Tospo-Virus Genera)-Viren, Röteln-Viren, Tollwut-Viren, Arena-Viren (insbesondere das Lymphozytäre Chorio-Meningitis Virus (LCMV) und das Lassa-Fieber Virus), Gastroenteritis-Viren (insbesondere Rota-Viren, Adenoviren, Calici-Viren, Astro-Viren, Corona-Viren), Retroviren (insbesondere Typ A, B, C und D Retroviren, Lentiviren (insbesondere die humanen Immundefizienz-Viren Typ-1 (HIV-1) und -2 (HIV-2)), das Affenimmundefizienzvirus (SIV), Katzenimmundefizienzvirus (FIV), und Rinderimmundefizienzvirus (BIV)), Spumaviren, die humanen T-Zell-Leukämie-Viren Typ-1 (HTLV-1) und -2 (HTLV-2)), Parvo-Viren (insbesondere Parvo-Virus B 19 und adeno-assoziierte Viren (AAV)), Papova-Viren (insbesondere Papillom-Viren, das Virus der progressiven multifokalen Leukoenzephalopathie (PML), BK-Virus), Adenoviren, Herpes-Viren (insbesondere das Herpes simplex Virus Typ-1 (HSV-1) und- 2 (HSV-2), das Varizellen Zoster Virus (VZV), Zytomegalie Virus (CMV) und Epstein-Barr-Virus (EBV), die Humanen Herpesviren 6, 7 und 8 (HHV 6, 7 und 8), Hepatitis-Viren (insbesondere das Hepatitis A Virus (HAV), Hepatitis B Virus (HBV), Hepatitis D Virus (HDV), Hepatitis C Virus (HCV), Hepatitis E Virus (HEV) und Hepatitis G Virus (HGV) sowie das *Transfusion-transmitted* Virus (TTV)) und Pocken Viren (insbesondere Orthopocken-Viren (wie das Menschenpocken-Virus, Vaccinia-Viren, Kuhpocken-Viren und Parapox-Viren)). Desweiteren können die Polypeptide von viralen Erregern seltener, subakuter oder chronischer Krankheiten (insbesondere Marburg- und Ebola-Viren, sowie Borna-Viren entstammen.

Beispielsweise können die Polypeptide wichtigen tierpathogenen Viren entstammen. Bedeutende Vertreter tierpathogener viraler Erreger sind beispielsweise, aber nicht ausschließlich, das equine Morbillivirus (EMP), Picornaviren (insbesondere Enteroviren, Aphthoviren (mit dem Erreger der Maul- und Klauenseuche (MKS)), das vesikuläre Stomatitis-Virus, Paramyxoviren (insbesondere Morbilliviren, aviäre Paramyxoviren), Pockenviren (insbesondere Capripoxviren), Bunyaviren, Reoviren (insbesondere Orbiviren), Flaviviren (insbesondere Pestiviren), Orthomyxoviren (insbesondere das Influenza A Virus), Herpesviren (insbesondere Alphaherpesviren), Tollwutviren, Retroviren (insbesondere Lentiviren und C-Typ Retroviren), Togaviren, Rhabdoviren, Birnaviren, Coronaviren und Caliciviren.

Eine umfassende Auflistung der derzeit beschriebenen Viren wurde beispielsweise von dem Internationalen Committee für Virustaxonomie (*The International Committee on Taxonomy of Viruses* (ICTV)) zusammengestellt und ist' über das Internet abrufbar (http://www.ncbi.nlm.nih.gov/ICTV/).

Beispiele für Bakterien, deren Polypeptide oder Fragmente die im erfindungsgemäßen Vektor enthaltenen Nukleinsäuresequenzen kodieren sind nachfolgend aufgelistet. Prinzipiell können die Polypeptide von jedem beliebigen Bakterium entstammen. Vorzugsweise stammen sie jedoch von intrazellulären Bakterien. Bedeutende intrazelluläre Bakterien sind beispielsweise, aber nicht ausschließlich, Listerien (insbesondere *L. monocytogenes*), Salmonellen (insbesondere *S. typhimurium*) und Mykobacterien (insbesondere *M. tuberculosis*).

Ferner können die Polypeptide humanpathogenen Bakterien entstammen. Bedeutende Vertreter humanpathogener bakterielle Erreger sind beispielsweise, aber nicht ausschließlich Staphylokokken, Streptokokken, Enterokokken, Neisserien, Enterobakterien (insbesondere *Escherichia coli (E. coli),* inklusive Säuglingspathogenen *E*. *coli* Stämmen (EPEC), Enteroaggregativen *E*. *coli* Stämmen (EAggEC), Klebsiellen, Enterobacter, Serratia, Proteus, Citrobacter und Typhösen Salmonellen), Enteritis Salmonellen, Shigellen, Yersinien), Vibrionen (insbesondere *Vibrio cholerae* und *Vibrio El Tor*), Pseudomonaden, Burkholderia, Stenotrophomas, Acinetobacter, Campylobacter, Helicobacter (insbesondere *Helicobacter pylori*), Hämophilus, Bordetellen, Legionellen, Listerien, Brucellen, Francisellen, Erysipelothrix, Korynebakterien, Bacillus, Clostridien, Bacteroide, Prevotella, Porphyromonas, Fusobacterium, Anaerobiospirillum, Anaerorhabdus, Anaerovibrio, Butyrivibrio, Centripedia, Desulfomonas, Dichelobacter, Fibrobacter, Leprotricha, Megamonas, Mitsuokella, Rikenella, Sebaldella, Selenomonas, Succinovibrio, Succinimonas, Tisserella, Mycobacterien (insbesondere *M tuberculosis,* atypische Mycobakterien (MOTT) und *M. leprae*), Nocardien, Treponemen (insbesondere *T. pallidum* und *T. carateum*), Borrelien (insbesondere *B. burgdorferi* und *B*. *recurrentis*), Leptospiren, Ricksettsien, Coxiellen, Ehrlichien, Bartonellen, Mycoplasmen (insbesondere *M. pneumoniae* und *M*. *hominis*), Ureaplasmen, Actinomyceten, Chlamydien. Zudem können die Polypeptide weiteren medizinisch bedeutsamen Bakterien entstammen, wie beispielsweise Tropheryma, Pasteurella, Branhamella, Streptobacillus, Spirillum und Gardnerella.

Ferner können Polypeptide tierpathogenen Bakterien entstammen. Bedeutende Vertreter tierpathogener bakterielle Erreger sind beispielsweise, aber nicht ausschließlich, Mycoplasmen, Bacillus (insbesondere *bacillus anthracis*), Brucellen, Mycobakterien (insbesondere *M*. *tuberculosis und M. bovis*), Campylobacter, Tritrichomonas, Leptospiren, Rickettsien, Salmonellen, Clostridien, Actinobazillen, Clamydien, Echinokokken, Listerien, Yersinien, Corynebakterien und Francisella.

Beispiele für Pilze, deren Polypeptide oder Fragmente die im erfindungsgemäßen Vektor enthaltenen Nukleinsäuresequenzen kodieren sind nachfolgend aufgelistet. Besonders bedeutende (human)pathogene Pilze sind beispielsweise, aber nicht ausschließlich, Sproßpilze (insbesondere Candida, Cryptococcus, Malassetia), Fadenpilze (insbesondere Aspergillus, Trichphyton, Microsporum, und Epidermophyton), Dimorphe Pilze (insbesondere Histoplasma, Blastomyces, Coccidioides, Paracoccidioides, Sporothrix) und Pneumocystis.

Beispiele für Parasiten, deren Polypeptide oder Fragmente die im erfindungsgemäßen Vektor enthaltenen Nukleinsäuresequenzen kodieren sind nachfolgend aufgelistet. Bedeutende Vertreter humanpathogener Parasiten sind insbesondere auch Protozoen wie Tryphanosomen, Leishmanien, Trichomonen, Giardia, Amöben, Plasmodien, Toxoplasma, Kryptosporidien, Mikrosporidien. Bedeutende Vertreter humanpathogener Parasiten sind insbesondere auch Trematoden wie beispielsweise Schistosomen, sowie Cestoden wie beispielsweise Bandwürmer und Echinococcen, sowie Nematoden, wie beispielsweise Trichuris, Trichinella, Strongyloides, Ancyclostoma, Necator, Enterobius, Ascaris und Filarien. Bedeutende Vertreter tierpathogener Parasiten sind beispielsweise, aber nicht ausschließlich Protozoen (insbesondere Protomonaden, Diplomonaden, Polymastigiden, Amoben, Toxoplasmen und Coccidien), Microsporen, Helminthen, Trematoden, Cestoden und Nematoden.

Die für Polypeptide des Menschen, von Primaten, anderen Säugetieren, beliebigen anderen Tieren, Parasiten, Mikroorganismen, Pflanzen, Algen oder Prionproteinenkodierenden Polynukleotide können zudem in beliebiger Weise miteinander gekoppelt sein.

Sie können aber auch mit beliebigen Nukleinsäuren gekoppelt sein, welche für funktionelle Polypeptide kodieren. Polypeptide mit funktionellen Eigenschaften sind beispielsweise Signalpeptide für das Polypeptidtargeting in das Endoplasmatische Retikulum (ER), beispielsweise die Signalpeptide des Mellitins, des Erytropoeitins, des humanen Interleukin 3, des humanen Interleukin-8, des humanen LAMP-1 und -2 Proteins oder die tPA Signalsequenz. Derartige Polypeptide sind aber auch Endo- und Lysosomale Signalsequenzen oder Polypeptide Endo- und Lysosomalen Signalsequenzen wie beispielsweise die zytoplasmatische Region der Ii Kette, oder Bereichen der "invariant chain", oder von LAMP-1, LAMP-2, LIMP-1 (CD63), LAP oder MHC-Klasse II Proteinen, welche Endo/Lysosomale Signalsequenzen enthalten.

Derartige Polypeptide sind sind zudem solche, die Transmembrandomänen darstellen, beispielsweise, aber nicht ausschließlich die Transmembrandomäne Epstein Barr Virus gp220/350 Hüllproteins, des HIV gp41 Transmembranproteins, aber auch beliebige Transmembrandomänen anderer natürlich vorkommender Transmembranproteine, aber auch synthetisch hergestellte Transmembrandomänen.Die erfindungsgemäßen Vektoren enthalten üblicherweise einen bakteriellen Replikationsursprung (ori) und ein Gen, das eine Resistenz beispielsweise gegen Ampicillin, Kanamycin, Tetracyclin oder Zeozin vermittelt. Die kodierenden Bereiche für den bakteriellen Replikationsursprung und die Antibiotikaresistenz befinden sich vorzugsweise benachbart zu der Expressionseinheit zur konstitutiven Expression von Polypeptiden (P₂) mit bekannten oder vermuteten T-Zellepitopen. Die für die konstitutive Expression eines Polypeptids, den Replikationsursprung und das Resistenzgen erforderlichen Nukleinsäuren können auf dem erfindungsgemäßen Vektor, beiderseitig durch eine oder mehrere Erkennungssequenz(en) für Restriktionsschnittstellen begrenzt werden, die bevorzugt innerhalb der begrenzten Nukleinsäuresequenz inklusive der Sequenz für den Replikationsursprung, das Resistenzgen und der Expressionseinheit zur konstitutiven Expression des Polypeptids mit dem bekannten oder vermuteten T-Zellepitop nicht vorkommt. Dieser Sequenzabschnitt auf dem Vektor kann auch durch zwei unterschiedliche, jedoch kompatible Schnittstellen für Restriktionsendonukleasen begrenzt werden, wenn beide Schnittstellen nicht innerhalb der den Replikationsursprung, das Resistenzgen und der Expressionseinheit zur konstitutiven Expression des Polypeptids umfassenden Nukleinsäuresequenz vorkommen. Die Anwesenheit dieser Schnittstellen für Restriktionsenzyme ist nicht erforderlich, wenn episomal vorliegende Plasmide als Transfersystem für die erfindungsgemäßen Vektoren verwendet werden.

Der erfindungsgemäße Vektor kann ferner die dem Fachmann bekannten und zum Stand der Technik gehörenden folgenden Kontrollsequenzen enthalten: Transkriptionsterminations- und Polyadenylierungssequenzen, Enhancer, Introns mit funktionellen Splice-Donor und Akzeptorstellen sowie Leadersequenzen, eine TATA-Box, eine GC-Box, eine CAAT-Box und andere Promotorelemente, die üblicherweise stromaufwärts der TATA-Box lokalisiert sind, sowie eine optimale aber auch suboptimale Kozak-Sequenz.

Expressionskassetten sind oft in einem Replikon enthalten, wie z.B. in extrachromosomalen Elementen (z.B. Plasmiden), die in der Lage sind, stabil in einem Wirt, wie z.B. einer Säugerzelle zu überdauern. Säugerreplikationssysteme beinhalten solche, die von animalen Viren, beispielsweise Papovaviren, Polyomaviren, Rinderpapillomviren oder dem Epstein-Barr-Virus abgeleitet sind und trans-aktive Faktoren zur Replikation benötigen.

Weiterhin kann die Expressioneffizienz des gewünschten Fremdgens durch die Auswahl und Verwendung geeigneter, wirtsspezifischer Codons gesteigert werden. Diese Beobachtung beruht auf dem Befund, dass sowohl prokaryontische als auch eukaryontische Gene keinen statistischen Gebrauch synonymer Codons aufweisen.

Als Vektorbackbone für die Herstellung erfindungsgemäßer Plasmide eignen sich beispielsweise, aber nicht ausschließlich auf dem Prototyp Plasmid pBR322 (Bolivar et al. In: DNA Insertion Elements, Plasmids and S. Episomes. Bukhari, A., Shapiro J.A., and Adhya S.L. (eds.) Cold Spring Harbor Laboratory, USA pp. 686-687, (1977).) basierende pcDNA Expressionsvektoren, die eine effiziente Genexpression in Säugerzellen erlauben.

Ferner ist eine Vielzahl unterschiedlicher nicht-viraler und viraler Gentransfervektoren geeignet, um Nukleinsäuren in Säugerzellen einzubringen. Einige der bedeutendsten Vektorsysteme zur Genexpression in Säugerzellen sind in dem Review von Makrides (Makrides, Protein Expr. Purif. (1999), 17(7), 183-202) aufgeführt. Viele der beschriebenen Vektoren sind zur Transduktion von Nukleinsäuren in APC besonders geeignet und kommerziell verfügbar.

Beispielsweise kann Plasmid-DNS, insbesondere auch die erfindungsgemäßen Vektoren direkt zur Transduktion von Zellen eingesetzt werden. Insbesondere eignen sich eine zweite Generation von linearen DNS-Plasmiden, die sogenannten MIDGE-Transfektionsvektoren (Mologen AG, Berlin) zum effizienten Transfer der in dieser Patentschrift beschriebenen Nukleinsäuresequenzen. Verschiedene Verabreichungsformen zur Transduktion von Säugerzellen (beispielsweise aber nicht ausschließlich die Lipofektion, Elektroporation oder Calziumphosphat Präzipitation) sowie zur Steigerung der Effizienz der Aufnahme von Vektoren sind publiziert und Stand der Technik. Besonders geeignet zum Transfer von Nukleinsäuren in APC sind beispielsweise die Femtosekunden Lasertechnologie Protokoll (Tirlapur and König, (2001) Nature, 418, 290), die Nucleovector^{™} Technologie (Amaxa, Deutschland) oder das Fugene 6 Transfektionsreagens (Roche, Deutschland).

Die Effizienz des Nukleinsäuretransfers kann zudem durch die Verwendung miniaturisierter Liposomen mit einem Durchmesser von etwa 25 nm (Copernicus Therapeutics, USA) drastisch gesteigert werden.

Die Effizienz des Gentransfers mittels retroviraler Transfersysteme kann z.B. durch Adjustierung der Zielzellen in der S Phase gesteigert werden. Methoden zur Optimierung der Effizienz der Transduktion von Säugerzellen mittels viraler Transfersysteme umfassen zudem die Variation der "*multiplicity of infection*" (M.O.I.), die Depletion von Ionen wie z.B. von Phospationen, Zugabe von polykationischen Substanzen, beispielsweise von Protaminsulfat, die Variation der Kontaktzeit, Temperatur, des pH-Werts, eine gemeinsame Zentrifugation der Zellen und Virus- bzw. Vektorstocks oder die Inkubation der Zellen mit den Transfersystemen in einem kleinen Mediumvolumen.

Die in der Art hergestellten erfindungsgemäßen Transfersysteme eignen sich sowohl für die Epitopsuche als auch zum Nachweis Epitop-spezifischer T-Zellen.

Die Erfindung betrifft somit die Bereitstellung von Gentransfervektoren zur Veränderung von APC mittels dieser Vektoren. Die erfindungsgemäßen Vektoren übertragen auf APC eine Kombination aus zwei Eigenschaften:
(1) Die APC erlangen die Fähigkeit, einzelne bekannte Zielstrukturen, z.B. bei der Anwendung in der Diagnostik, oder noch unbekannte potentielle Zielstrukturen z.B. bei der Epitopsuche, konstitutiv intrazellulär zu produzieren und Bruchstücke davon in Verbindung mit MHC-Proteinen der Klassen I und/oder II auf ihrer Oberfläche zu präsentieren.
(2) Bei Erkennung der auf der Oberfläche der APC präsentierten Zielstruktur (Epitop) durch eine spezifische T-Zelle wird die Expression des Markers über das von der T-Zelle ausgelöste Signal in der APC induziert oder inhibiert. Die veränderte Expression des Markers und damit die aktivierte APC kann in einfacher und quantitativer Weise beispielsweise mittels kommerziell erhältlicher Geräte und Verfahren zuverlässig und schnell nachgewiesen (beispielsweise FACS-, Immunfluoreszenz-, ELISA-, ELIspot-Technologie) und isoliert (beispielsweise FACS*sorting,* magnetisches Zell*-Sorting*) werden.

Die mittels der erfindungsgemäßen Vektoren veränderten APC eignen sich zu einer schnellen Detektion und Charakterisierung von bislang unbekannten Zielstrukturen aktivierter T-Zellen und stellen somit ein geeignetes Werkzeug zur Erstellung Krankheits- oder Patientenspezifischer Datenbanken dar. Zudem können die mittels der erfindungsgemäßen Vektoren modifizierten APC zum Nachweis beliebiger T-Zellpopulationen verwendet werden, deren Zielstrukturen bekannt sind. Die erfindungsgemäßen Verfahren finden Anwendung bei allen Ventebraten, die T-Zellen besitzen, insbesondere am Menschen, Primaten und Nagetieren. Die Kenntnis von bislang unbekannten Zielregionen aktivierter T-Zellen eröffnet zudem neue Perspektiven für die Entwicklung neuartiger Diagnostik-, Therapie- und Impfkonzepte zur Vorbeugung und Behandlung von Erreger-induzierten Erkrankungen, Autoimmunerkrankungen, Transplantatabstoßungen und chronischen entzündliche Erkrankungen. Weiterhin eröffnet die Kenntnis von T-Zellepitopen in nichthumanen Vertebraten die Etablierung neuer Tiermodellsysteme für Forschungszwecke.

Ein weiteren Aspekt der vorliegenden Erfindung betrifft mit den erfindungsgemäßen Vektoren transduzierte antigenpräsentierende Zellen (APC). Die Herstellung von erfindungsgemäßen APC erfolgt durch die Behandlung von APC mit den erfindungsgemäßen Gentransfervektoren. Die Behandlung der APC mit den erfindungsgemäßen Gentransfervektoren kann durch eine Inkubation von heparinisiertem Vollblut oder definierten aus dem Vollblut aufgereinigten peripheren blutmononukleären Zellen (PBMC), sowie gereinigten Populationen definierter APC (beispielsweise B-Zellen, dendritische Zellen, Monozyten, Makrophagen) mit erfindungsgemäßen viralen und bakteriellen Gentransfervektoren erfolgen, welche einen spezifischen Tropismus für alle APC oder definierte Subpopulationen von APC aufweisen. Alternativ eignen sich insbesondere gereinigte Populationen von PBMC und gereinigte Populationen definierter APC (beispielsweise B-Zellen, dendritische Zellen, Monozyten, Makrophagen) zur Behandlung mit den erfindungsgemäßen nichtviralen Gentransfervektoren, beispielsweise Plasmiden, MIDGE Vektoren und Replikons. Diese nichtviralen Vektoren werden vorzugsweise mittels physikalisch/chemischer Verfahren (beispielsweise Femtosekunden Lasertechnologie, Nucleovector^{™} Technologie, Transfektion mittels des Fugene 6 Reagens (Roche, Deutschland), aber auch der Lipofektion, Elektroporation oder Calziumphosphat Präzipitation) in die Zielzellen eingebracht. Verfahren zur Reinigung von PBMC und definierten Populationen von APC aus heparinisiertem Vollblut, sowie der Nukleinsäuretransfer in APC mittels viraler, bakterieller sowie nichtviraler/nicht bakterieller Vektoren sind Stand der Technik.

Der Erfolg des Transfers der erfindungsgemäßen Nukleinsäuren kann anhand eines Nachweises der Expression des unter der Kontrolle des konstitutiven Promotors (Promotor P₂) stehenden Polypeptids oder des unter der Kontrolle des dritten Promotors stehenden Markers mittels gängiger molekularbiologischer und immunologischer Methoden, beispielsweise der Immunoblot-, der Immunfluoreszenz- oder FACS-Technologie unter Verwendung geeigneter Antikörper überprüft werden. Derartige molekularbiologischen und immunologischen Methoden zum spezifischen Nachweis von Polypeptiden sind vielfach publiziert und Stand der Technik.

Zur Herstellung der erfindungsgemäßen APC zur Epitopsuche werden erfindungsgemäße Vektoren verwendet, die unter anderem die unter der Kontrolle des konstitutiv aktiven Promotors (P₂) beliebige Polynukleotide, welche für Polypeptide mit potentiellen Zielepitopen reaktiver T-Zellen kodieren, beispielsweise Polynukleotide einer (subtraktiven) Genbank aufweisen.

Zur Herstellung erfindungsgemäßer APC zum Nachweis Epitop-spezifischer T-Zellen werden erfindungsgemäße Transfersysteme verwendet, die unter anderem die unter der Kontrolle des konstitutiv aktiven Promotors (P₂) definierte Polynukleotide, welche für ein oder mehrere Polypeptide mit bekannten Zielepitopen reaktiver T-Zellen kodieren, aufweisen.

Ein Aspekt der Erfindung betrifft ferner APC zur Epitopsuche oder zum Nachweis Epitop-spezifischer T-Zellen, die mit einem Vektor transduziert wurden, der den erfindungsgemäßen Gentransfervektoren entsprecht, mit der Einschränkung, dass er die Expressionseinheit für den Marker, umfassend den in APC induzierbaren Promotor (P₁) und die funktionell verbundene Nukleinsäure kodierend ein Markergen nicht aufweist.

Ein weiteren Aspekt der vorliegenden Erfindung betrifft ein Verfahren zum Nachweis Epitop-spezifischer T-Zellen und zum Nachweis von Zielepitopen reaktiver T-Zellen, umfassend die folgenden Schritte:
a) Isolierung von APC-haltiger und/oder T-zellhaltiger Körperflüssigkeit, vorzugsweise von Blut oder Liquor,
b) in Kontakt bringen und Transduktion von APC-haltiger Körperflüssigkeit mit denerfindungsgemäßen Gentransfervektoren,
c) Inkubieren der Körperflüssigkeit enthaltend die transduzierten APC oder von isolierten transduzierten APC mit der Körperflüssigkeit enthaltend die T-Zellen oder die isolierten T-Zellen, bevorzugt für 0,5 bis 36 Stunden oder länger, besonders bevorzugt für 0,5 bis 2, 2 bis 6, 6 bis 12, 12 bis 36 Stunden oder 36 bis 168 Stunden,
d) Nachweis von Marker exprimierenden APC, und
e) gegebenenfalls Isolierung und Charakterisierung der mit dem zweiten Promotor funktionell verbundenen für die Zielepitope reaktiver T-Zellen kodierenden Nukleinsäure.

Ferner betrifft die Erfindung ein weiteres Verfahren zum Nachweis Epitop-spezifischer T-Zellen und zum Nachweis von Zielepitopen reaktiver T-Zellen, umfassend die folgenden Schritte:
a) Isolierung von APC-haltiger und/oder T-zellhaltiger Körperflüssigkeit, vorzugsweise von Blut,
b) in Kontakt bringen und Transduktion von APC-haltiger Körperflüssigkeit mit einer Nukleinsäure unter anderem umfassend einen in APC konstitutiven Promotor (P₂), und einer mit diesem Promotor funktionell verbundenen Nukleinsäure,
c) Inkubieren der Körperflüssigkeit enthaltend die transduzierten APC oder von isolierten transduzierten APC mit der Körperflüssigkeit enthaltend die T-Zellen oder die isolierten T-Zellen, bevorzugt für 0,5 bis 36 Stunden oder länger, besonders bevorzugt für 0,5 bis 2, 2 bis 6, 6 bis 12, 12 bis 36 Stunden oder 36 bis 168 Stunden,
d) Nachweis von Marker exprimierenden APC, und
e) gegebenenfalls Isolierung und Charakterisierung der mit dem zweiten Promotor funktionell verbundenen für die Zielepitope reaktiver T-Zellen kodierenden Nukleinsäure.

Die in Schritt b) des weiteren Verfahrens verwendeten Vektoren entsprechen den erfindungsgemäßen Gentransfervektoren, mit der Einschränkung, dass die Expressionseinheit für den Marker, umfassend den in APC induzierbaren Promotor (P₁) und die funktionell verbundene Nukleinsäure kodierend ein Markergen nicht beinhalten. Ansonsten entsprechen die verwendeten Gentransfervektoren in allen funktionellen Bereichen den erfindungsgemäßen Gentransfervektoren. Vielmehr werden in diesem Verfahren anstelle der durch den Gentransfervektor kodierten Expressionseinheiten für den Marker (P₁) genomisch vorliegende Promotoren, welche in ihrer Aktivität infolge einer Epitop-spezifischen Erkennung der erfindungsgemäßen APC durch eine T-Zelle meßbar verändert werden, sowie die in natürlicher Weise funktionell verbundenen Nukleinsäuren als funktionelle Einheit zur Markerexpression, d.h. Induktion oder Inhibition, verwendet. Das weitere Verfahren umfasst auch regulierbare genomische Promotoren, welche funktionell mit Polynukleotiden, kodierend für beliebige Marker, verknüpft sind.

Vorzugsweise ist die APC-haltiger Körperflüssigkeit in Schritt b) der Verfahren Blut, Liquor, eine gereinigte PBMC-Population oder eine separierte APC-Population. Vorzugsweise sind die isolierten T-Zellen CD4⁺ T-Zellen, CD8⁺ T-Zellen, CD4⁺CD25⁺ regulatorische T-Zellen, CD161⁺ NKT Zellen oder ein beliebiges Gemisch aus NKT, CD4⁺, CD8⁺ und CD4⁺CD25⁺ T-Zellen.

Der Nachweis und die Quantifizierung Epitop-spezifischer T-Zellen und die Suche von Zielepitopen reaktiver T-Zellen kann beispielsweise aus Patienten, die an einer Autoimmunerkrankung, einer chronisch entzündlichen Erkrankung, einer mikrobiellen Infektion, einer Tumorerkrankung oder einer Transplantatabstoßung leiden, erfolgen, oder aber aus gesunden Probanden oder Teilnehmern therapeutischer oder präventiver Studien. Zudem kann der Nachweis von epitopspezifischen T-Zellen und die Suche von Zielepitopen reaktiver T-Zellen mittels der erfindungsgemäßen APC auch in Primaten oder anderen Tieren erfolgen, die T-Zellen besitzen.

Zur Durchführung des Verfahrens wird einem Probanden oder Patienten beispielsweise Blut oder eine andere APC-haltige und/oder T-zellhaltige Körperflüssigkeit entnommen und die APC mit den erfindungsgemäßen Gentransfervektoren transduziert. Die Transduktion der APC mit den erfindungsgemäßen Vektoren kann in Blut, gereinigten PBMC Populationen, oder separierten APC Populationen erfolgen. Die Isolierung der gereinigten oder der separierten Populationen kann auch nach der Transduktion erfolgen. Die in der Art hergestellten erfindungsgemäßen APC werden dann mit isolierten T-Zellpopulationen, insbesondere CD4⁺ T-Zellen, aber auch CD8⁺ T-Zellen, CD161⁺ NKT Zellen, CD4⁺CD25⁺ regulatorische T-Zellen, einem beliebigem Gemisch aus, CD4⁺-, CD8⁺-, CD4⁺CD25⁺- und CD161⁺ NKT Zellen, oder auch T-zellhaltigen Zellgemischen oder T-zellhaltiger Körperflüssigkeit desselben Patienten für 0,5 bis 2 Stunden, 2 bis 6 Stunden oder aber für 6-12 Stunden oder aber für 12 bis 36 Stunden, oder aber für 36 bis 168 Stunden, oder aber für mehr als 168 Stunden unter geeigneten Kultivierungsbedingungen, beispielsweise bei 37° C in einer befeuchteten Atmosphäre mit 5 bis 8 % CO₂ in T-Zellmedium (RPMI 1640 mit 2 bis 10% hitzeinaktiviertem (30 min, 56°C) humanem AB Serum, 2 mM Glutamin und 100 mg/ml Kanamycin oder Gentamycin (alle Komponenten von PanSystems, Aidenbach) inkubiert. Die Inkuation kann in Ab- oder Anwesenheit geeigneter (ko)-stimulatorischer Substanzen, beispielsweise Zytokine Mitogene oder Antikörper durchgeführt werden. Alternativ können die T-zellhaltigen Zellpopulationen auch aus Patienten/Probanden mit kompatiblem MHC-Muster stammen. Befinden sich in der gemischten APC/T-Zellkultur reaktive T-Zellen, welche mit den Gentransfervektoren transduzierte APC erkennen, die *spezifische* T-Zellepitope präsentieren, so induzieren oder inhibieren diese in den Subpopulationen der erfindungsgemäßen APC die Markerexpression. Befinden sich in der gemischten APC/T-Zellkultur erfindungsgemäße APC, die T-Zellepitope präsentieren, welche von reaktiven T-Zellen des Probanden/Patienten erkannt werden, so wird infolge der Epitopspezifischen Erkennung in diesen APC die Markerexpression induziert oder inhibiert.

In dem weiteren Verfahren dient in Schritt (d) die infolge einer Epitop-spezifischen Erkennung der erfindungsgemäßen APC durch eine T-Zelle in natürlicher Weise meßbar veränderte (gesteigerte oder reduzierte) Expression der unter der Kontrolle des genomischen regulierbaren Promotors exprimierten Polypeptide in der APC als Marker für die T-Zellerkennung.

Beispielsweise kann die infolge der Epitop-spezifischen Erkennung in der APC gesteigerte Expression des OX40 Liganden (OX40L) und des 4-1BB Liganden (4-1BBL) (den Haan and Bevan; 2000), sowie der kostimulatorischen Proteine B7.1 (CD80), B7.2 (CD86) und des Fas Liganden (FasL) als Marker dienen. Geeignet als Marker ist zudem die in der erfindungsgemäßen APC infolge der Epitop-spezifischen Erkennung der erfindungsgemäßen APC durch eine reaktive T-Zelle meßbar gesteigerte oder verringerte Expression jedes beliebigen Polypeptids.

Das Vorhandensein und die Anzahl Marker-positiver APC kann beispielsweise mittels der FACS, ELISA oder der Immunfluoreszenztechnologie unter Verwendung spezifischer, chemisch modifizierter Antikörper, bzw. Antikörperpärchen nachgewiesen werden. Alternativ werden Marker mit Enzymfunktion durch Zugabe und Umsetzung geeigneter Substrate nachgewiesen. Erfindungsgemäße epitoppräsentierende APC, welche die Expression eines der beschriebenen Marker aufweisen, können mittels verschiedener immunologischer und biochemischer Verfahren unter Verwendung kommerziell erhältlicher Geräte und Testsysteme nachgewiesen, isoliert und vereinzelt werden. So kann die Expression ausgewählter Marker beispielsweise mittels der FACS-, ELISA- oder Immunfluoreszenztechnologie beispielsweise unter Verwendung spezifischer, chemisch modifizierter Antikörper, bzw. Antikörperpärchen nachgewiesen werden. Alternativ werden Marker mit Enzymfunktion durch Zugabe und Umsetzung geeigneter Substrate nachgewiesen. Die Isolierung und Vereinzelung markerpositiver APC kann unter Verwendung der FACSSort Technologie (beispielsweise der Firmen Coulter, Becton Dickinson) erfolgen. Die molekularbiologischen und immunologischen Methoden Verfahren, welche bei der Epitopsuche verwendet werden, z.B. die Durchführung von FACSscan Analysen, FACSsorting, ELISA Assays, Immunfluoreszenzanalysen, Nachweis von Enzymreaktionen, Gesamt DNS Präparation aus Zellen, PCR sowie Sequenzierung von Nukleinsäuren sind Stand der Technik.

Die Isolierung und Charakterisierung der für die Zielepitope reaktiver T-Zellen kodierenden Polynukleotide aus selektionierten, markerpositiven erfindungsgemäßen APC kann mittels unterschiedlicher Verfahren erfolgen, die nachfolgend beispielhaft beschrieben sind. Beispielsweise ist das PCR-Verfahren zur Identifizierung der die Zielepitope reaktiver T-Zellen kodierenden Polynukleotide aus den selektionierten, markerpositiven erfindungsgemäßen APC geeignet. Aus den vereinzelten, erfindungsgemäßen Marker-positiven APC wird mittels eines kommerziellen Kits (beispielsweise der Firmen Stratagene oder Qiagen) Gesamt-DNS präpariert und die unbekannte Nukleinsäuresequenz des gesuchten T-Zellepitops unter Verwendung geeigneter Oligonukleotide (Primer) mittels des PCR-Verfahrens amplifiziert. Die Primer weisen bevorzugt konstante Bereiche innerhalb der erfindungsgemäßen Nukleinsäuren in unmittelbarer Nachbarschaft zu den zu identifizierenden Polynukleotiden auf.

Alternativ kann auch das Lysat der selektionierten und vereinzelten markerpositiven APC als Template für die PCR-Reaktion verwendet werden. Durch die Verwendung spezifischer Primer außerhalb der unter der Kontrolle des konstitutiven Promotors (P₂) klonierten Polynukleotide in den flankierenden Bereichen auf den erfindungsgemäßen nicht viralen bzw. viralen Vektoren können die unbekannten, unter der Kontrolle des konstitutiven Promotors befindlichen cDNA amplifiziert, durch Sequenzierung beschrieben und eventuell durch Vergleich mit Sequenzen einer Datenbank identifiziert werden.

Alternativ kann aus den vereinzelten markerpositiven APC oder einem Gemisch an markerpositiven APC Gesamt-DNS z.B. mittels eines kommerziellen Kits präpariert werden. Die erhaltene DNS wird anschließend mit einer Restriktionsendonuklease, deren Erkennungssequenz den konstitutiven Promotor inklusive des unter seiner Kontrolle stehenden Polynukleotides, sowie eine bakterielle Resistenz und den bakteriellen Replikationsursprung enthält, (siehe Figur 1B) im 5' und 3' Bereich flankiert, geschnitten. Die erhaltenen DNS Fragmente werden anschließend religiert und in geeignete Bakterien, beispielsweise DH10B Bakterien transformiert. Falls episomale Plasmide als erfindungsgemäße Transfersysteme zur Transduktion der APC verwendet wurden, kann die aus Marker-positiven APC gewonnene Gesamt-DNS direkt zur Transformation von geeigneten Bakterien verwendet werden.

Bakterien, die nach diesem Verfahren mit Plasmiden transformiert wurden, welche den erfindungsgemäßen Promotor P₂ und die unter dessen Kontrolle befindlichen Polynukleotide in Kombination mit einer bakteriellen Antibiotikaresistenz und den bakteriellen Replikationsursprung enthalten, können durch das Ausstreichen auf Agarplatten, die das entsprechende Antibiotikum enthalten, selektiv kultiviert und ausvereinzelt werden. Nach einer Anzucht der klonalen Bakterienkolonien in einem entsprechenden antibiotikahaltigem Selektivmedium wird die Plasmid DNS aus den Bakterien mittels gängiger Verfahren, beispielsweise der alkalischen Schnelllyse, gewonnen.

Die aus den Bakterien gereinigten Plasmide werden direkt als *Template* für die Sequenzierungsreaktion eingesetzt. Durch die Verwendung spezifischer Primer, welche in konservierten Bereichen des erfindungsgemäßen Vektors nahe dem zu identifizierenden Poynukleotid unter der Kontrolle des erfindungsgemäßen Promotors P₂ binden, können die unter der Kontrolle des Promotors P₂ befindlichen Polynukleotide durch Sequenzierung bestimmt, und deren Identität möglicherweise durch Vergleich mit Sequenzen biologischer und medizinischer Datenbanken bestimmt werden. Zur Bestätigung der mittels dieser Verfahren identifizierten cDNS-Sequenzen und zum Ausschluß falsch positiver Ergebnisse werden die erhaltenen Nukleinsäuresequenzen nochmals unter die Kontrolle des konstitutiven Promotors in die erfindungsgemäßen Vektoren kloniert und mittels eines erfindungsgemäßen Transfersystems in APC transfiziert. Die in der Art hergestellten APC werden dann nochmals auf Erkennung durch reaktive T-Zellen desselben Patienten getestet. Durch eine kontinuierliche 5'- und 3' seitige Verkürzung der identifizierten Polynukleotide und das Einbringen dieser mittels erfindungsgemäßer Transfersysteme in APC kann die minimale Länge der erkannten Zielregion in Kokultivierungsexperimenten mit reaktiven T-Zellen des Patienten/Probanden bestimmt werden.

Mit den erfindungsgemäßen Verfahren können beispielsweise Polypeptide und Epitope identifiziert werden, die bei der Erkennung und Kontrolle von durch Mikroorganismen und Parasiten induzierten Erkrankungen durch T-Zellen, bei der Erkennung und Kontrolle von Tumorerkrankungen durch T-Zellen bedeutsam sind, Zielstrukturen fehlgeleiteter, körpereigener T-Zellen bei Autoimmunerkrankungen und chronisch entzündlichen Prozessen oder Zielstrukturen körpereigener T-Zellen bei der Abstoßung transplantierter Gewebe oder Organe darstellen.

Die erfindungsgemäßen Verfahren sind geeignet für eine Vielzahl unterschiedicher medizinischer Anwendungen. Mit dem erfindungsgemäßen Verfahren können Epitop- und Polypeptid-spezifische T-Zellen identifiziert werden zur frühzeitigen Diagnostik von Autoimmunerkrankungen; zum Monitoring reaktiver T-Zellen in Patienten mit Autoimmunerkrankungen, die einen chronisch progressiven oder *relapsing*/*remitting* Krankheitsverlauf aufweisen; zur Bestimmung der Effizient therapeutischer Behandlungen von Erkrankungen mit T-Zellbeteiligung; zum Screening der Sicherheit und Effizienz von Medikamenten, die eine Deletion oder Anergie von T-Zellen hervorrufen oder eine generelle Immunsuppression bewirken; zum Monitoring der Effizienz therapeutischer und prophylaktischer Impfungen zur Induktion von T-Zellen; zum Monitoring und der Diagnostik von durch Mikroorganismen und Parasiten induzierten Erkrankungen mit T-Zellbeteiligung; zum Monitoring und zur Diagnostik chronischer Entzündungen mit T-Zellbeteiligung; zum Monitoring und zur Diagnostik von Tumorantigen-spezifischen T-Zellen; zum Monitoring und zur Diagnostik von T-Zellen, die bei der Transplantatabstoßung eine Rolle spielen oder zur gezielten Auswahl von Probanden mit für Impfstudien und die Testung therapeutischer Behandlungen

Desweiteren umfasst die Erfindung einen Kit zum diagnostischen Nachweis autoreaktiver T-Zellen, umfassend geeignete erfindungsgemäße Vektoren oder Transfersysteme zur Herstellung erfindungsgemäßer APC, verpackt in geeigneten Behältern; eine detaillierte Anleitung zur Durchführung des diagnostischen Nachweises und einen geeigneten Behälter zum Transport und der Lagerung des erfindungsgemäßen Kits.

Für die Epitopsuche ergeben sich aufgrund der vorliegenden Erfindung die folgenden Vorteile. Die Verwendung der erfindungsgemäßen Vektoren in Kombination mit Genbanken ermöglicht die schnelle Identifizierung von T-Zellepitopen aus derzeit bekannten und charakterisierten Polypeptiden aber auch aus bislang noch nicht beschriebenen bzw. charakterisierten Polypeptiden. Durch die Verwendung von Genbanken kann zudem in einem oder einer sehr begrenzten Anzahl von Versuchsansätzen gleichzeitig sehr viele Zielepitope reaktiver T-Zellen eines Probanden/Patienten isolieren und beschreiben werden. Dieses Verfahren ist somit im Vergleich zu den bisher verwendeten Verfahren zur Epitopsuche mit einem deutlich geringerem Zeit- und Kostenaufwand verbunden.

Zur Epitopsuche können polyspezifische T-Zellen eines Patienten verwendet werden. Dadurch kann der sehr zeit- und arbeitsaufwendige Schritt der Erzeugung von T-Zellinien umgangen werden. APC, die Zielepitope reaktiver T Zellen exprimieren, können aufgrund der Markerexpression sehr schnell mittels kommerziell erhältlicher Geräte (beispielsweise FACSsort) selektioniert und die Gene der gesuchten T-Zellepitope mittels molekularbiologischer Routineverfahren identifiziert werden.

Das erfindungsgemäße Verfahren eignet sich universell für die Epitopsuche bei unterschiedlichen Probanden/Patienten. Genfähren, welche die erfindungsgemäßen Vektoren tragen, können unabhängig von dem Haplotyp bei allen Probanden/Patienten universell zur gentechnischen Veränderung der APC für die Epitopsuche eingesetzt werden. Das erfindungsgemäße Verfahren eignet sich zur Suche von Zielepitopen unterschiedlicher T-Zellpopulationen (insbesondere von CD4⁺ T-Helferzellen, aber auch von CD8⁺ zytotoxischen T-Zellen, CD161⁺ NKT-Zellen und CD4⁺CD25⁺ regulatorische T-Zellen).

Für den Nachweis und die Diagnostik Epitop-spezifischer T-Zellen ergeben sich aufgrund der vorliegenden Erfindung die folgenden Vorteile. Ein wesentlicher Vorteil des erfindungsgemäßen Verfahrens liegt darin begründet, dass es auf der Messung einer durch eine epitopspezifische Erkennung durch eine T-Zelle in APC induzierten Markergenexpression und nicht, wie bei allen bisherigen Verfahren, auf der Messung von Reaktionen der T-Zelle infolge einer Epitoperkennung auf APC beruht. Dadurch wird die Sensitivität des Assaysystems erheblich gesteigert. Das Nachweisverfahren für die reaktiven T-Zellen ist im Vergleich zu den herkömmlichen Diagnostikverfahren (CTL-Assay, ELISPOT, Zytokin-ELISA, Proliferationsassay) universell einsetzbar, leichter handhabbar, deutlich kostengünstiger, weniger zeitaufwendig und sensitiv. Die Anwesenheit und Anzahl reaktiver T-Zellen kann einfach mittels, kommerziell erhältlicher und in vielen Diagnostiklabors routinemäßig verwendeter Gerätschaften (FACS) leicht nachgewiesen und quantifiziert werden. Das erfindungsgemäße Diagnostikverfahren kann unabhängig von dem Haplotyp des Probanden/Patienten universell für den Nachweis reaktiver T-Zellen eingesetzt werden. Das erfindungsgemäße Verfahren eignet sich zum Nachweis verschiedenster Epitop-spezifischer T-Zellpopulationen (insbesondere CD4⁺ T-Helferzellen, aber auch CD8⁺ zytotoxische T-Zellen, CD161⁺ NKT-Zellen und CD4⁺CD25⁺ regulatorische T-Zellen).

**Die folgenden Beispiele erläutern die vorliegende Erfindung:**

### Beispiel 1:

### Klonierung der erfindungsgemäßen Vektorbackbones zur Expression von Polypeptiden unter der Kontrolle eines in APC konstitutiv aktiven Promotors (P2)

Als Ausgangsvektor zur Klonierung der erfindungsgemäßen Vektorbackbones dienten das 5446 Basenpaare (bp) umfassende Plasmid pcDNA3.1(+) (Invitrogen), sowie der Vektor pcDNA3.1(+)dNeo, der durch die Deletion eines 1905 bp umfassenden DNS Fragments, inclusive des kodierenden Bereichs für Neomycin (bp 1289-3194) aus dem Plasmid pcDNA3.1(+) erzeugt wurde. Hierzu wurde die Nukleinsäuresequenz des pcDNA3.1(+)dNeo Plasmids mittels der Polymerasekettenreaktion (PCR) unter Verwendung nachfolgender Amplifikationsbedingungen gewonnen: Einleitender Denaturierungsschritt: 95° C, 1 Min., danach folgte eine 3-Stufen PCR (35 Zyklen) mit folgenden Bedingungen: Denaturierung: 94° C, 1 Min., Annealing 55° C, 1 Min., Elongation: 72° C, 3,5 Min. mit einem abschließenden Polymerisationsschritt bei 72° C für 10 Min. mit anschließender Dauerkühlung bei 4° C. Zur Amplifikation wurden die Primer P1 (5'-GCTGGTTCTTTCCGCCTCAGAAGC-3'; SEQ ID NO:6) und P2 (5'-CACTGCATTCTAGTTGTGGTTTG-3'; SEQ ID NO:7) verwendet. Das erhaltene PCR-Produkt wurde mittels eines QIAquick *PCR Purification Kits* (Qiagen) nach Angaben des Herstellers aufgereinigt, phosphoryliert und schließlich religiert. Das erhaltene 3541 bp umfassende Plasmid wurde pcDNA3.1(+)dNeo genannt.

Zur Insertion von verschiedenen in APC konstitutiv aktiven Kontrollsequenzen wurden diese mittels PCR aus geeignetem Material (chromosomale DNA humanen und murinen Ursprungs) amplifiziert und mittels geeigneter Restriktionsschnittstellen in die Vektorbackbones pcDNA3.1(+) und pcDNA3.1(+)dNeo eingebracht.

### Amplifikation des murinen PGK Promotors

Die 621 bp umfassende Sequenz des murinen PGK Promotors wurde mittels PCR aus isolierter chromosomaler DNA der murinen Zellinie C2C12 (ATCC Nummer CRL-1772) unter Verwendung nachfolgender Bedingungen gewonnen: Einleitender Denaturierungsschritt: 95° C, 2 Min., danach folgte eine 3-Stufen PCR (35 Zyklen) mit folgenden Bedingungen: Denaturierung: 95° C, 45 Sek., Annealing 66° C, 1 Min., Elongation: 72° C, 1,5 Min., mit einem abschließenden Polymerisationsschritt bei 72° C für 10 Min. mit anschleißender Dauerk-ühlung bei 4° C. Zur Amplifikation wurden die Primer P3 (5' Primer: 5'-GCAGGC*TCGCGA*CTACCGGGTAGGGGAGGCGC-3'; SEQ ID NO:8) und P4 (3' Primer: 5'-GCAGGC*GGATCC*ACGCGCTTCTACAAGGCGCTTGC-3'; SEQ ID NO:9) verwendet.

### Amplifikation des humanen PKG Promotors

Die 531 bp umfassende Sequenz des humanen PGK Promotors wurde mittels PCR aus isolierter chromosomaler DNA aus humanen PBMCs unter Verwendung nachfolgender Amplifikationsbedingungen isoliert: Einleitender Denaturierungsschritt: 98° C, 2 Min., danach folgten zwei aufeinanderfolgende 3-Stufen PCR mit folgenden Bedingungen: Erste PCR Amplifikationsrunde (15 Zyklen): Denaturierung: 95° C, 1 Min., Annealing 58° C, 1 Min., Elongation: 72° C, 1,5 Min., direkt anschließend folgte eine zweite PCR Amplifikationsrunde (25 Zyklen) mit folgenden Bedingungen: Denaturierung: 95° C, 1 Min., Annealing 69° C, 1 Min., Elongation: 72° C, 1,5 Min. mit einem abschließenden Polymerisationsschritt bei 72° C für 10 Min. mit anschleißender Dauerkühlung bei 4° C. Zur Amplifikation wurden die Primer P5 (5' Primer: 5'- GCAGGC*TCGCGA*CGGGGTTGGGGTTGCGCC-3'; SEQ ID NO:10) und P6 (3' Primer: 5'-GCAGGC*GGATCC*TTTGGAAATACAGCTGGGGAG-3'; SEQ ID NO:11) verwendet.

### Amplifikation des humanen DHFR Promotors

Die 479 bp umfassende Sequenz des humanen Dihydrofolat-Reduktase (DHFR) Promotors wurde mittels PCR aus isolierter chromosomaler DNA entstammend humanen PBMCs unter Verwendung nachfolgender Amplifikationsbedingungen gewonnen: Einleitender Denaturierungsschritt: 95° C, 2 Min., danach folgte eine 3-Stufen PCR (30 Zyklen) mit folgenden Bedingungen: Denaturierung: 95° C, 45 Sek., Annealing 71° C, 1 Min., Elongation: 72° C, 1,5 Min., mit einem abschließenden Polymerisationsschritt bei 72° C für 10 Min. mit anschleißender Dauerkühlung bei 4° C. Zur Amplifikation wurden die Primer P7 (5' Primer: 5'-GCAGGC*TCGCGA*CGATGGCCCTGCCCAGTCCC-3'; SEQ ID NO:12) und P8 (3' Primer: 5'-GCAGGC*GGATCC*GACAGCAGCGGGAGGACCTC-3'; SEQ ID NO:13) verwendet.

### Amplifikation des humanen EF-1α Promotors

Hierbei wurde die 1244 bp umfassende Sequenz des humanen EF-1α Promotors mittels PCR aus isolierter chromosomaler DNA entstammend humanen PBMCs unter Verwendung nachfolgender Amplifikationsbedingungen gewonnen: Einleitender Denaturierungsschritt: 98° C, 2 Min., danach folgten zwei aufeinanderfolgende 3-Stufen PCR mit folgenden Bedingungen: Erste PCR Amplifikationsrunde: (15 Zyklen) Denaturierung: 95° C, 1 Min., Annealing 58° C, 1 Min., Elongation: 72° C, 10 Min., direkt anschließend folgte eine zweite PCR Amplifikationsrunde (30 Zyklen) mit folgenden Bedingungen: Denaturierung: 98° C, 1 Min., Annealing 67° C, 1 Min., Elongation: 72° C, 2 Min. mit einem abschließenden Polymerisationsschritt bei 72° C für 10 Min. mit anschleißender Dauerkühlung bei 4° C. Zur Amplifikation wurden die Primer P9 (5' Primer: 5'-GCAGGC*TCGCGA*GGCTCCGGTGCCCGTCAGTG-3'; SEQ ID NO:14) und P10 (3' Primer: 5'-GCAGGC*GGATCC*ACCTAGCCAGCTTGGGTCTCC-3'; SEQ ID NO:15) verwendet.

### Amplifikation des humanen Ubiquitin C (UbC) Promotors

Die 1210 bp umfassende Sequenz des humanen UbC Promotors wurde mittels PCR aus isolierter chromosomaler DNA entstammend humanen PBMCs unter Verwendung nachfolgender Bedingungen amplifiziert: Einleitender Denaturierungsschritt: 98° C, 2 Min., danach folgten zwei aufeinanderfolgende 3-Stufen PCR mit folgenden Bedingungen: Erste PCR Amplifikationsrunde: (15 Zyklen) Denaturierung: 95° C, 50 Sek., Annealing 58° C, 45 Sec., Elongation: 72° C, 3 Min., direkt anschließend folgte eine zweite PCR Amplifikationsrunde (25 Zyklen) mit folgenden Bedingungen: Denaturierung: 95° C, 50 Sek., Annealing 71° C, 45 Sec., Elongation: 72° C, 3 Min. mit einem abschließenden Polymerisationsschritt bei 72° C für 10 Min. mit anschleißender Dauerkühlung bei 4° C. Zur Amplifikation wurden die Primer P11 (5' Primer: 5'-GCAGGC*TCGCGA*GGCCTCCGCGCCGGGTTTTGG-3'; SEQ ID NO:16) und P12 (3' Primer: 5'-GCAGGC*GGATCC*GTCTAACAAAAAAGCCAAAAACG-3'; SEQ ID NO:17) verwendet.

### Amplifikation des humanen ICI Promotors

Die 569 bp umfassende Sequenz des humanen ICI Promotors wurde mittels PCR aus isolierter chromosomaler DNA entstammend humanen PBMCs unter Verwendung nachfolgender Bedingungen amplifiziert: Einleitender Denaturierungsschritt: 95° C, 2 Min., danach folgten zwei aufeinanderfolgende 3-Stufen PCR mit folgenden Bedingungen: Erste PCR Amplifikationsrunde: (15 Zyklen) Denaturierung: 95° C, 45 Sek., Annealing 58° C, 1 Min., Elongation: 72° C, 1,5 Min., direkt anschließend folgte eine zweite PCR Amplifikationsrunde (25 Zyklen) mit folgenden Bedingungen: Denaturierung: 95° C, 45 Sek., Annealing 66° C, 1 Min., Elongation: 72° C, 1,5 Min. mit einem abschließenden Polymerisationsschritt bei 72° C für 10 Min. mit anschleißender Dauerkühlung bei 4° C. Zur Amplifikation wurden die Primer P13 (5' Primer: 5'-GCAGGC*TCGCGA*GCTGTAATTTCTAATCTAAACC-3'; SEQ ID NO:18) und P14 (3' Primer: 5'-GCAGGC*GGATCC*AGCAGCAGAGTGCGGCAACAC-3'; SEQ ID NO:19) verwendet.

Die in den, für die Amplifikation der oben genannten Promotoren verwendeten Primern enthaltenen Schittstellen (*Nru*I im 5' Primer und *Bam*HI im 3' Primer) sind jeweils kursiv dargestellt.

Die Gewinnung humaner PBMC erfolgte wie nachfolgend beschrieben: Frisch abgenommenes Vollblut eines gesunden Probanden wurde mit 20 IU/ml Heparin versetzt. 50 ml-Leukosep-Gefäße mit Trennmembran wurden mit 15 ml Ficoll-Histopaque (Sigma, Deisenhofen) befüllt und 2 Min. bei 500 x g zentrifugiert. Die Röhrchen wurden mit dem heparinisierten Vollblut befüllt und 1:1 mit sterilem PBS/0,5% BSA verdünnt. Die Ansätze wurden 30-40 Min. bei 400 x g zentrifugiert, ohne den Rotor am Ende des Laufes abzubremsen. Die lymphozytenhaltige, trübe Interphase wurde vorsichtig abgezogen und in 3 Volumina T-Zell-Medium (RPMI 1640 Medium mit 10% hitzeinaktiviertem (30 Min. bei 56° C) humanem AB Serum, 2 mM Glutamin und 100 mg/ml Kanamycin bzw. Gentamycin (alle Komponenten von PanSystems, Aidenbach)) dreimal gewaschen.

Die Isolierung von genomischer DNS aus humanen PBMC oder murinen C2C12 Muskelzellen wurde mit dem "*DNA Extraction Kit*" (Stratagene) nach der Anleitung des Herstellers durchgeführt. Hierzu wurden die Zellen 2 x mit kaltem PBS (*phosphate buffered saline*) gewaschen und die Zellzahl bestimmt. Je 1 x 10⁸ Zellen wurden anschließend in 15 ml Plastikröhrchen überführt und sofort auf Eis gestellt. Auf Eis erfolgten auch alle nachfolgenden Arbeitsschritte. Nach einer 15-minütigen Zentrifugation (350 x g, 4 °C) wurden die Zellen in 11 ml Lösung 2 (50 mM Tris/HCl, pH 8,0, 20 mM EDTA, 2 % SDS) resuspendiert und mit einem Dounce Homogenisator homogenisiert. Danach wurde Pronase (Stratagene) bis zu einer Endkonzentration von 100 µg/ml hinzugefügt und der Ansatz über Nacht unter leichtem Schütteln bei 37 °C inkubiert. Anschließend wurde der Ansatz 10 Min. auf Eis abgekühlt und 4 ml einer eiskalten Lösung 3 (gesättigte NaCl-Lösung) hinzupipettiert. Nach kurzem Vermischen der Lösungen wurde für weitere 5 Min. auf Eis inkubiert und das Pellet bei 4 °C abzentrifugiert (15 Min., 2.000 x g). Danach wurde der Überstand mit einer abgeschnittenen Plastikpipette in ein steriles 50 ml Zentrifugenröhrchen überführt, RNAse in einer Endkonzentration von 20 µg/ml zugegeben und 15 Min. bei 37 °C inkubiert. Nach Zugabe von 2 Volumenteilen 100 %-igem Ethanol und leichtem Schütteln konnte das Ausfallen der genomischen DNS in weißen Schlieren beobachtet werden. Die DNS konnte nun entweder auf einem Glasstab aufgewickelt oder durch 15-minütige Zentrifugation bei 4 °C und 2.000 x g pelletiert werden. Nach einem Waschschritt mit 2 ml kaltem 70 %-igem Ethanol wurde die DNS in ein Eppendorf-Reaktionsgefäß überführt, abzentrifugiert und 15 Min. bei Raumtemperatur luftgetrocknet. Das DNS-Pellet wurde in H₂O_{bid}. aufgenommen und über Nacht bei Raumtemperatur bzw. für 1-2 Std. bei 55 °C gelöst. Die Bestimmung der DNS-Konzentration erfolgte photometrisch bei 258 nm.

Die erhaltenen PCR-Produkte wurden mittels eines QIAquick *PCR Purification Kits* (Qiagen) nach Angaben des Herstellers aufgereinigt und anschließend wurden 20 µg der jeweiligen gereinigten PCR Banden im 100 µl Maßstab mit den Restriktionsenzymen *Nru*I und *Bam*H1 verdaut. Gleichzeitig wurden jeweils 20 µg der Vektorbackbones pcDNA3.1(+) und pcDNA3.1(+)dNeo mit denselben Restriktionsenzymen (*Nru*I und *Bam*HI) verdaut. Die erhaltenen Spaltprodukte wurden über ein 1%-iges Agarosegel aufgetrennt, die Polynukleotide für die linearisierten Vektorbackbones und die verschiedenen Promotoren aus dem Gel ausgeschnitten und die Nukleinsäuren mittels eines QIAquick Gel Extraktion Kits (Qiagen) aufgereinigt. Anschließend wurden die erhaltenen Polynukleotide für die Vektorbackbones mit den unterschiedlichen verdauten PCR Amplifikaten mit den jeweiligen Promotorsequnzen über Nacht bei 16°C ligiert und der Ligationsansatz dann in Bakterien (DH5α) transformiert. Nach einer 1 stündigen Inkubation bei 37°C in LB-Medium ohne Selektionsantibiotikum wurden die transformierten Bakterien auf LB_{Amp} Platten ausgestrichen und über Nacht bei 37°C inkubiert. Die erhaltenen Bakterienkolonien wurden dann in LB_{Amp} Medium kultiviert, die bakterielle Plasmid DNS mittels der Technik der alkalischen Schnelllyse isoliert und mittels eines Restriktionsverdaus unter Verwendung der Restriktionsendonukleasen *Bgl*II/*Eco*RI charakterisiert. Die in der Art hergestellten Vektoren wurden pcDNA3.1(+)mPGK, pcDNA3.1(+)hPGK, pcDNA3.1(+)hDHFR, pcDNA3.1(+)hEF-1α, pcDNA3.1(+)hUbC, pcDNA3.1(+)hIGI, beziehungsweise pcDNA3.1(+)dNeomPGK, pcDNA3.1(+)dNeohPGK, pcDNA3.1(+)dNeohDHFR, pcDNA3.1(+)dNeohEF-1α, pcDNA3.1(+)dNeohUbC und pcDNA3.1(+)dNeohICI genannt.

Die 224 bp umfassende *bovine growth hormone* (BGH) Polyadenylierungssequenz wurde aus dem pcDNA3.1(+) Vektor mittels PCR unter Verwendung geeigneter PCR Bedingungen (Einleitender Denaturierungsschritt: 95° C, 2 Min., anschließende 3-Stufen PCR (35 Zyklen) mit folgenden Bedingungen: Denaturierung: 95° C, 30 Sek.; Annealing 55° C, 1 Min.; Elongation: 72° C, 1 Min., und einem abschließenden Polymerisationsschritt bei 72° C für 10 Min. mit anschließender Dauerkühlung bei 4° C) amplifiziert. Zur Amplifikation wurden die Primer P15 (5' Primer: 5'-GGCGGG*GGATCC*CTGTGCCTTCTAGTTGCC-3'; SEQ ID NO:20) und P 16 (3' Primer: 5'-GGCGGG*AGATCT*CCATAGAGCCCACCGC-3'; SEQ ID NO:21) verwendet. Die in den Primern enthaltenen Schittstellen (*Bam*HI im 5' Primer; *Bgl*II im 3' Primer sind kursiv dargestellt). Die mittels der PCR amplifizierte Bande wurde dann wie vorstehend beschrieben aufgereinigt, mit den Restriktionsenzymen *Bgl*II und B*am*HI gespalten und die in der Art erhaltene Bande in die mit *Bam*HI linearisierten Plasmide pcDNA3.1(+)mPGK, pcDNA3.1(+)hPGK, pcDNA3.1(+)hDHFR, pcDNA3.1(+)hEF-1α, pcDNA3.1(+)hUbC, pcDNA3.1(+)hICI, beziehungsweise pcDNA3.1(+)dNeomPGK, pcDNA3.1(+)dNeohPGK, pcDNA3.1(+)dNeohDHFR, pcDNA3.1(+)dNeohEF-1α, pcDNA3.1(+)dNeohUbC und pcDNA3.1(+)dNeohICI ligiert. Die so erzeugten Vektor erhielten die Bezeichnungen pcDNA3.1(+)mPGKPA, pcDNA3.1(+)hPGKPA, pcDNA3.1(+)hDHFRPA, pcDNA3.1(+)hEF-1αPA, pcDNA3.1(+)hUbCPA, pcDNA3.1(+)hICIPA, beziehungsweise pcDNA3.1(+)dNeomPGKPA, pcDNA3.1(+)dNeohPGKPA, pcDNA3.1(+)dNeohDHFRPA, pcDNA3.1(+)dNeohEF-1αPA, pcDNA3.1(+)dNeohUbCPA und pcDNA3.1(+)dNeohICIPA.

In Analogie sind jedoch auch die Vektorbackbones pcDNA3.1(+) (Invitrogen), sowie der Vektor pcDNA3.1(+)dNeo als Vektoren zur Expression von beliebigen Polypeptiden in APC geeignet. Sie enthalten den konstitutiv in APC aKtiven CMV Promotor in Verbindung mit der *bovine growth hormone* (BGH) Polyadenylierungssequenz.

Anschließend wurden für Polypeptide, beispielsweise das Epstein-Barr Virus BZLF1 Protein, das humane Myelin-basische Protein (MBP), und das HIV-1 p24 Kapsidprotein kodierende Polynukleotide unter die Kontrolle des oben genannten in APC konstitutiv aktiven Promotoren kloniert. Dazu wurden die gewünschten Polynuleotide mittels PCR amplifiziert, die gereinigten PCR Amplifikate gereinigt, mit geeigneten Restrinktionsendonukleasen geschnitten und in mit den entsprechenden Restrinktionsendonukleasen verdauten Vektoren inseriert.

### Erzeugung von Plasmiden zur konstitutiven Expression des Epstein-Barr Virus BZLF1 Proteins

Die cDNS für das EBV BZLF1 Protein wurde mittels PCR unter Verwendung geeigneter PCR-Bedingungen (Einleitender Denaturierungsschritt: 95° C, 2 Min., darauffolgend eine 3-Stufen PCR (15 Zyklen) mit folgenden Bedingungen: Denaturierung: 95° C, 30 Sek.; Annealing 52° C, 1 Min.; Elongation: 72° C, 2 Min., danach nochmals (25 Zyklen) mit folgenden Bedingungen: Denaturierung: 95° C, 30 Sek.; Annealing 63° C, 1 Min.; Elongation: 72° C, 2 Min., und einem abschließenden Polymerisationsschritt bei 72° C für 10 Min. mit anschließender Dauerkühlung bei 4°C) aus viraler DNS des EBV Stammes B95-8 in der Gesamt-DNS infizierter Marmoset-Zellen amplifiziert. Bei der PCR Reaktion zur Amplifikation des BZLF1 cDNS wurde dem PCR Ansatz 2,5% DMSO beigefügt. Zur Amplifikation wurden die Primer P17 (5' Primer: 5'-GGCGG*AGATCT*TTAGAAATTTAAGAGATCC-3'; SEQ ID NO:22) und P18 (3' Primer: 5'-GGCGGG*AGATCT*ATGATGGACCCAAACTCG-3'; SEQ ID NO:23) verwendet. Die mittels der PCR amplifizierte 970 Nukleotide umfassende Bande wurde dann wie vorstehend beschrieben aufgereinigt, mit dem Restriktionsenzym *Bgl*II gespalten und die erhaltene Bande in die mit *Bam*HI linearisierten Expressionsvektoren (pcDNA3.1 (+)mPGKPA, pcDNA3.1(+)hPGKPA, pcDNA3.1(+)hDHFRPA, pcDNA3.1(+)hEF-1αPA, pcDNA3.1(+)hUbCPA, pcDNA3.1(+)hICIPA, beziehungsweise pcDNA3.1(+)dNeomPGKPA, pcDNA3.1(+)dNeohPGKPA, pcDNA3.1(+)dNeohDHFRPA, pcDNA3.1(+)dNeohEF-1αPA, pcDNA3.1(+)dNeohUbCPA, pcDNA3.1(+)dNeohICIPA, pcDNA3.1(+) und pcDNA3.1(+)dNeo ligiert. Die so erzeugten Expressionsvektoren erhielten die Bezeichnungen (pcDNA3.1(+)mPGKPA-Z, pcDNA3.1(+)hPGKPA-Z, pcDNA3.1(+)hDHFRPA-Z, pcDNA3.1(+)hEF-1αPA-Z, pcDNA3.1(+)hUbCPA-Z, pcDNA3.1(+)hICIPA-Z, beziehungsweise pcDNA3.1(+)dNeomPGKPA-Z, pcDNA3.1(+)dNeohPGKPA-Z, pcDNA3.1(+)dNeohDHFRPA-Z, pcDNA3.1(+)dNeohEF-1αPA-Z, pcDNA3.1(+)dNeohUbCPA-Z, pcDNA3.1(+)dNeohICIPA-Z, pcDNA3.1(+)-Z (Figur 2) und pcDNA3.1(+)dNeo-Z.

### Erzeugung von Plasmiden zur konstitutiven Expression des humanen MBP-Proteins

Die cDNS für das humane MBP-Protein wurde mittels PCR unter Verwendung geeigneter Bedingungen (Einleitender Denaturierungsschritt: 95° C, 2 Min., darauf folgend eine 3-Stufen PCR (35 Zyklen) mit folgenden Bedingungen: Denaturierung: 95° C, 30 Sek.; Annealing 55° C, 1 Min.; Elongation: 72° C, 2 Min., und einem abschließenden Polymerisationsschritt bei 72° C für 10 Min. mit anschließender Dauerkühlung bei 4° C) aus Gesamt DNS von Hirnzellen amplifiziert. Zur Amplifikation wurden die Primer P19 (5' Primer: 5'-GGCGG*AGATCT*ATGGCGTCACAGAAGAGACC-3'; SEQ ID NO:24) und P20 (3' Primer: 5'-GGCGGG*AGATCT*TCAGCGTCTAGCCATGGG-3'; SEQ ID NO:25) verwendet. Die mittels der PCR amplifizierte 585 Nukleotide umfassende Bande wurde dann wie vorstehend beschrieben aufgereinigt, mit dem Restriktionsenzym *Bgl*II gespalten und die erhaltene Bande in die mit *Bam*H1 linearisierten Plasmide (pcDNA3.1(+)mPGKPA, pcDNA3.1(+)hPGKPA, pcDNA3.1(+)hDHFRPA, pcDNA3.1(+)hEF-1αPA, pcDNA3.1 (+)hUbCPA, pcDNA3.1(+)hICIPA, beziehungsweise pcDNA3.1(+)dNeomPGKPA, pcDNA3.1(+)dNeohPGKPA, pcDNA3.1(+)dNeohDHFRPA, pcDNA3.1(+)dNeohEF-1 aPA, pcDNA3.1(+)dNeohUbCPA, pcDNA3.1(+)dNeohICIPA, pcDNA3.1(+) und pcDNA3.1(+)dNeo ligiert. Die so erzeugten Expressionsvektoren erhielten die Bezeichnungen (pcDNA3.1(+)mPGKPA-MBP, pcDNA3.1(+)hPGKPA-MBP, pcDNA3.1(+)hDHFRPA-MBP, pcDNA3.1(+)hEF-1αPA-MBP, pcDNA3.1(+)hUbCPA-MBP, pcDNA3.1(+)hICIPA-MBP, beziehungsweise pcDNA3.1(+)dNeomPGKPA-MBP, pcDNA3.1(+)dNeohPGKPA-MBP, pcDNA3.1(+)dNeohDHFRPA-MBP, pcDNA3.1(+)dNeohEF-1αPA-MBP, pcDNA3.1(+)dNeohUbCPA-MBP, pcDNA3.1(+)dNeohICIPA-MBP, pcDNA3.1(+)-MBP und pcDNA3.1(+)dNeo-MBP.

Eine kodonoptimierte DNS für das HIV-1 p24 Kapsidprotein wurde mittels PCR unter Verwendung geeigneter Bedingungen (Einleitender Denaturierungsschritt: 95° C, 2 Min., darauf folgend eine 3-Stufen PCR (30 Zyklen) mit folgenden Bedingungen: Denaturierung: 95° C, 45 Sek.; Annealing 66° C, 1 Min.; Elongation 72° C, 2 Min., und einem abschließenden Polymerisationsschritt bei 72° C für 10 Min. mit anschließender Dauerkühlung bei 4° C) aus einer synthetisch hergestellten Nukleinsäure entsprechend einer publizierten HIV-1 gag Sequenz (Graf et al. (2000), J. Virol. 74:10822) amplifiziert. Kodonoptimierte Gene können über verschiedene Firmen (beispielsweise GeneArt, Regensburg; Entelechion, Regensburg) bezogen werden. Zur Amplifikation wurden die Primer P21 (5' Primer: 5'-GGCGG*TCTAGA*GCCGCCACCATGCCCATCGTG-3'; SEQ ID NO:26) und P22 (3' Primer: 5'-GGCGGG*GAATTC*TCACAGCAGCCTGGCCTTGTG-3'; SEQ ID NO:27) verwendet. Das erhaltene PCR Produkt wurde dann wie vorstehend beschrieben aufgereinigt, mit dem Restriktionsenzym *Bgl*II gespalten und die erhaltene Bande in die mit *Bam*H1 linearisierten Plasmide (pcDNA3.1(+)mpGKpA, pcDNA3.1(+)hPGKPA, pcDNA3.1(+)hDHFRPA, pcDNA3.1(+)hEF-1αPA, pcDNA3.1(+)hUbCPA, pcDNA3.1(+)hICIPA, beziehungsweise pcDNA3.1(+)dNeomPGKPA, pcDNA3.1(+)dNeohPGKPA, pcDNA3.1(+)dNeohDHFRPA, pcDNA3.1(+)dNeohEF-1αPA, pcDNA3.1(+)dNeohUbCPA, pcDNA3.1(+)dNeohICIPA, pcDNA3.1(+) und pcDNA3.1(+)dNeo ligiert. Die so erzeugten Expressionsvektoren erhielten die Bezeichnungen (pcDNA3.1(+)mPGKPA-p24, pcDNA3.1(+)hPGKPA-p24, pcDNA3.1(+)hDHFRPA-p24, pcDNA3.1(+)hEF-1αPA-p24, pcDNA3.1(+)hUbCPA-p24, pcDNA3.1(+)hICIPA-p24, beziehungsweise pcDNA3.1(+)dNeomPGKPA-p24, pcDNA3.1(+)dNeohPGKPA-p24, pcDNA3.1(+)dNeohDHFRPA-p24, pcDNA3.1(+)dNeohEF-1αPA-p24, pcDNA3.1(+)dNeohUbCPA-p24, pcDNA3.1(+)dNeohICIPA-p24, pcDNA3.1(+)-p24 und pcDNA3.1(+)dNeo-p24.

### Erzeugung von Plasmiden zur konstitutiven Expression des "enhanced green fluorescent protein" (eGFP)

Das Gen für das "*enhanced green fluorescent protein*" (eGFP) wurde mittels PCR unter Verwendung geeigneter PCR Bedingungen (Einleitender Denaturierungsschritt: 95° C, 2 Min., anschließende 3-Stufen PCR (35 Zyklen) mit folgenden Bedingungen: Denaturierung: 95° C, 30 Sek.; Annealing 55° C 1 Min.; Elongation: 72° C, 2 Min., und einem abschließenden Polymerisationsschritt bei 72° C für 10 Min. mit anschließender Dauerkühlung bei 4° C) aus dem Plasmid pRC/CMV-EGFP (Vogel et al., 1998, BioTechniques 143, 1967-1983) amplifiziert. Zur Amplifikation wurden die Primer P23 (5' Primer: 5'-GGCGGG*AGATCT*CGCCACCATGGTGAGCAAGG-3'; SEQ ID NO:28) und P24 (3' Primer: 5'-GGCGGG*AGATCT*TTACTTGTACAGCTCGTCC-3',SEQ ID NO:29) verwendet. Die mittels der PCR amplifizierte spezifische Bande mit einer Größe von 752 bp wurde dann wie vorstehend beschrieben aufgereinigt, mit dem Restriktionsenzym *Bgl*II gespalten und die erhaltene Bande in die mit *Bam*H1 linearisierten Expressionsvektoren (pcDNA3.1(+)mPGKPA, pcDNA3.1(+)hPGKPA, pcDNA3.1(+)hDHFRPA, pcDNA3.1(+)hEF-1αPA, pcDNA3.1(+)hUbCPA, pcDNA3.1(+)hICIPA, beziehungsweise pcDNA3.1(+)dNeomPGKPA, pcDNA3.1(+)dNeohPGKPA, pcDNA3.1(+)dNeohDHFRPA, pcDNA3.1(+)dNeohEF-laPA, pcDNA3.1(+)dNeohUbCPA, pcDNA3.1(+)dNeohICIPA, pcDNA3.1(+) und pcDNA3.1(+)dNeo ligiert. Die so erzeugten Expressionsvektoren erhielten die Bezeichnungen (pcDNA3.1(+)mPGKPA-eGFP, pcDNA3.1(+)hPGKPA-eGFP, pcDNA3.1(+)hDHFRPA-eGFP, pcDNA3.1(+)hEF-1αPA-eGFP, pcDNA3.1(+)hUbCPA-eGFP, pcDNA3.1(+)hICIPA-eGFP, beziehungsweise pcDNA3.1(+)dNeomPGKPA-eGFP, pcDNA3.1(+)dNeOhPGKPA-eGFP, pcDNA3.1(+)dNeohDHFRPA-eGFP, pcDNA3.1(+)dNeohEF-1αPA-eGFP, pcDNA3.1(+)dNeohUbCPA-eGFP, pcDNA3.1(+)dNeohICIPA-eGFP, pcDNA3.1(+)-eGFP und pcDNA3.1(+)dNeo-eGFP.

### Beispiel 2:

### Herstellung von Vektoren, welche einen gezielten Transport der mittels des konstitutiven Promotors (P2) exprimierten Polypeptide in das Endolysosom gewährleisten

Zur Steigerung des Endo/Lysosomalen Abbaus der mittels des konstitutiven Promotors (P2) exprimierten Polypeptide wurden zudem verschiedene lysosomale Targetingsignale, beispielsweise die zytoplasmatische Region der Ii Kette, oder Bereichen der "invariant chain", oder von LAMP-1, LAMP-2, LIMP-1 (CD63), LAP oder MHC-Klasse II Proteinen in die Plasmide pcDNA3.1(+)mPGKPA, pcDNA3.1(+)hPGKPA, pcDNA3.1(+)hDHFRPA, pcDNA3.1(+)hEF-1αPA, pcDNA3.1(+)hUbCPA, pcDNA3.1(+)hICIPA, beziehungsweise pcDNA3.1(+)dNeomPGKPA, pcDNA3.1(+)dNeohPGKPA, pcDNA3.1(+)dNeohDHFRPA, pcDNA3.1(+)dNeohEF-1αPA, pcDNA3.1(+)dNeohUbCPA, pcDNA3.1(+)dNeohICIPA, pcDNA3.1(+) und pcDNA3.1(+)dNeo eingefügt.

### Konstruktion von Expressionsvektoren, welche Signalpeptid/Polypeptidpeptid/LAMP-1 chimäre Proteine exprimieren

Die Klonierung von chimären Polypeptiden, welche aus dem.N-terminalen Signalpeptid des humanen LAMP-1 Proteins, einem beliebigen Polypeptid, beispielsweise dem EBV BZLF1 Protein oder dem humanen MBP Protein, sowie der Transmembrandomäne und dem zytoplasmatischen Anteil von LAMP-1 zusammengesetzt sind, erfolgte sequentiell unter Verwendung der kompatiblen Schnittstellen *Bam*H1 und *Bgl*II*.*

Die für das Signalpeptid von LAMP-1 kodierende Nukleinsäuresequenz wurde mittels PCR unter Verwendung einer *High fidelity Pfu Polymerase* (Stratagene) aus isolierter chromosomaler DNA entstammend humanen PBMCs unter Verwendung nachfolgender Bedingungen amplifiziert: Einleitender Denaturierungsschritt: 95° C, 2 Min., danach folgte eine 3-Stufen PCR (35 Zyklen) mit folgenden Bedingungen: Denaturierung: 95° C, 1 Min., Annealing 60° C, 1 Min., Elongation: 72° C, 1 Min., abschließender Polymerisationsschritt bei 72° C für 10 Min. mit anschleißender Dauerkühlung bei 4° C. Zur Amplifikation wurden die Primer P25 (5' Primer: 5'- GCAGG*AGATCT*TATGGCGCCCCGC-3 ; SEQ ID NO:30) und P26 (3' Primer: 5'-GCAGGC*GGATCC*TCAAAGAGTGCTGA-3'; SEQ ID NO:31) verwendet.

### Die Amplifikation der für die BZLF-1 und MBP Proteine kodierenden Polynukleotide unter Verwendung der bereits beschriebenen Amplifikationsbedingungen

Zur Amplifikation des BZLF-1 Gens wurden hierbei die Primer P27 (5' Primer: 5'-GGCGG*GGATCC*TTAGAAATTTAAGAGATCC-3; SEQ ID NO:32) und Primer P28 (3' Primer: 5'-GGCGGG*AGATCT*ATGATGGACCCAAACTCG-3'; SEQ ID NO:33) verwendet. Zur Amplifikation des humanen MBP-Proteins wurden die Primer P29 (5' Primer: 5'-GGCGG*AGATCT*ATGGCGTCACAGAAGAGACC-3 ; SEQ ID NO:34) und P30 (3' Primer: 5'-GGCGGG*GGATCC*TCAGCGTCTAGCCATGGG-3 ; SEQ ID NO:35) verwendet.

Die Amplifikation der Transmembranregion und zytoplasmatischen Domäne von LAMP-1 erfolgte mittels PCR unter Verwendung einer *High fidelity Pfu Polymerase* (Stratagene) aus isolierter chromosomaler DNA entstammend humanen PBMCs unter Verwendung nachfolgender Bedingungen: Einleitender Denaturierungsschritt: 95° C, 2 Min., danach folgte eine 3-Stufen PCR (35 Zyklen) mit folgenden Bedingungen: Denaturierung: 95° C, 1 Min., Annealing 60° C, 1 Min., Elongation: 72° C, 1,5 Min., abschließender Polymerisationsschritt bei 72° C für 10 Min. mit anschleißender Dauerkühlung bei 4° C. Zur Amplifikation wurden die Primer P31 (5' Primer: 5'- GCAGG*AGATC*TAACAGCACGCTGATC-3'; SEQ ID NO:36) und P32 (3' Primer: 5'- GCAGGC*AGATC*TCTAGATAGTCTGGTA-3 ; SEQ ID NO:37) verwendet.

Diese drei Komponenten der für das chimäre Signalpeptid/Polypeptidpeptid/LAMP-1 Polypeptidfragment kodierenden Nukleinsäure wurden jeweils nach der PCR Reaktion mit den entsprechenden Restrinktionsendonukleasen geschnitten, gereinigt und nacheinander in drei Schritten jeweils in die nachfolgend genannten, mit *Bam*H1 geschnittenen, Expressionsvektoren eingebracht. Bei diesem Verfahren wurden nacheinander die kodierenden Regionen des LAMP-1 Leaders, eines beliebiges Polynukleotids, hier beispielsweise des EBV-BZLF-1 Proteins oder des humanen MBP Proteins, sowie des C-terminalen, die Transmembrandomäne und den cytoplasmatischen Anteil des LAMP-1 Proteins codierenden Nukleinsäuren in die Plasmide pcDNA3.1(+)mPGKPA, pcDNA3.1(+)hPGKPA, pcDNA3.1(+)hDHFRPA, pcDNA3.1(+)hEF-1αPA, pcDNA3.1(+)hUbCPA, pcDNA3.1(+)hICIPA, beziehungsweise pcDNA3.1(+)dNeomPGKPA, pcDNA3.1(+)dNeohPGKPA, pcDNA3.1(+)dNeohDHFRPA, pcDNA3.1(+)dNeohEF-1αPA, pcDNA3.1(+)dNeohUbCPA, pcDNA3.1(+)dNeohICIPA, pcDNA3.1(+) und pcDNA3.1(+)dNeo ligiert. Die so erzeugten Expressionsvektoren erhielten die Bezeichnungen (pcDNA3.1(+)mPGKPA-Z/LAMP, pcDNA3.1(+)hPGKPA-Z/LAMP, pcDNA3.1(+)hDHFRPA-Z/LAMP, pcDNA3.1(+)hEF-1αPA-Z/LAMP, pcDNA3.1(+)hUbCPA-Z/LAMP, pcDNA3.1(+)hICIPA-Z/LAMP, sowie pcDNA3.1(+)dNeomPGKPA-Z/LAMP, pcDNA3.1(+)dNeohPGKPA-Z/LAMP, pcDNA3.1(+)dNeohDHFRPA-Z/LAMP, pcDNA3.1(+)dNeohEF-1αPA-Z/LAMP, pcDNA3.1(+)dNeohUbCPA-Z/LAMP, pcDNA3.1(+)dNeohICIPA-Z/LAMP, pcDNA3.1(+)-Z/LAMP (Figur 3) und pcDNA3.1(+)dNeo-Z/LAMP beziehungsweise (pcDNA3.1(+)mPGKPA-MBP/LAMP, pcDNA3.1(+)hPGKPA-MBP/LAMP, pcDNA3.1(+)hDHFRPA-MBP/LAMP, pcDNA3.1(+)hEF-1αPA-MBP/LAMP, pcDNA3.1(+)hUbCPA-MBP/LAMP, pcDNA3.1(+)hICIPA-MBP/LAMP, sowie pcDNA3.1(+)dNeomPGKPA-MBP/LAMP, pcDNA3.1(+)dNeohPGKPA-MBP/LAMP, pcDNA3.1(+)dNeohDHFRPA-MBP/LAMP, pcDNA3.1(+)dNeohEF-1αPA-MBP/LAMP, pcDNA3.1(+)dNeohUbCPA-MBP/LAMP, pcDNA3.1(+)dNeohICIPA-MBP/LAMP, pcDNA3.1(+)-MBP/LAMP und pcDNA3.1(+)dNeo-MBP/LAMP.

### Beispiel 3:

### Herstellung von Vektoren, welche eine Memranverankerung der mittels des konstitutiven Promotors (P2) befindlichen Polypeptide induzieren

Die Klonierung von chimären Polypeptiden, welche aus dem N-terminalen Signalpeptid des humanen LAMP-1 Proteins, einem beliebigen Polypeptid, beispielsweise dem EBV BZLF1 Protein oder dem humanen MBP Protein, sowie einer heterologen Transmembrandomäne, beispielsweise der des Epstein-Barr Virus gp220/350 Hüllproteins erfolgte sequentiell unter Verwendung der kompatiblen Schnittstellen *Bam*H 1 und *Bgl*II*.*

Die Amplifikation der für das Signalpeptid von LAMP-1 kodierenden Nukleinsäuresequenz, sowie der kodierenden Sequenzen des BZLF-1 und MBP Protein erfolgten, wie in Beispiel 2 detailliert beschrieben.

Die Amplifikation der EBV gp220/350 Transmembrandomäne (EBV-TM) erfolgte mittels PCR unter Verwendung einer *High fidelity Pfu Polymerase* (Stratagene) aus dem Plasmid pBRBamHI-L (Skare und Strominger, (1980), Proc. Natl. Acad. Sci. USA 77, 3860-64) unter Verwendung nachfolgender Bedingungen: Einleitender Denaturierungsschritt: 95° C, 2 Min., danach folgte eine 3-Stufen PCR (35 Zyklen) mit folgenden Bedingungen: Denaturierung: 95° C, 1 Min., Annealing 60° C, 1 Min., Elongation: 72° C, 1 Min., abschließender Polymerisationsschritt bei 72° C für 10 Min. mit anschleißender Dauerkühlung bei 4° C. Zur Amplifikation wurden die Primer P33 (5' Primer: 5'-GCAGG*AGATCT*AGCGGGGCAGGATCCATGCTAGTACTTCAATGGGCCTCTCTG-3'; SEQ ID NO:38) und P34 (3' Primer: 5'- GCAGGC*AGATCT*TTATACATACCTCTCGGCCTC-3'; SEQ ID NO:39) verwendet.

Diese drei Komponenten der für das chimäre SignalpeptidlPolypeptidpeptid/EBV-TM Polypeptidfragment kodierenden Nukleinsäure wurden jeweils nach der PCR Reaktion mit den entsprechenden Restrinktionsendonukleasen geschnitten, gereinigt und nacheinander in drei Schritten jeweils mit *Bam*H1 geschnittenen, nachfolgend genannten Expressionsvektoren eingebracht. Bei diesem Verfahren wurden nacheinander die kodierenden Regionen des LAMP- 1 Leaders, eines beliebiges Polynukleotids, hier beispielsweise des EBV-BZLF-1 Proteins oder des humanen MBP Proteins, sowie der EBV gp220/350 Transmembrandomäne kodierenden Nukleinsäuren in die Plasmide pcDNA3.1(+)mPGKPA, pcDNA3.1(+)hPGKPA, pcDNA3.1(+)hDHFRPA, pcDNA3.1(+)hEF-1αPA, pcDNA3.1(+)hUbCPA, pcDNA3.1(+)hICIPA, beziehungsweise pcDNA3.1 (+)dNeomPGKPA, pcDNA3.1(+)dNeohPGKPA, pcDNA3.1(+)dNeohDHFRPA, pcDNA3.1(+)dNeohEF-1αPA, pcDNA3.1(+)dNeohUbCPA, pcDNA3.1(+)dNeohICIPA, pcDNA3.1(+) und pcDNA3.1(+)dNeo ligiert. Die so erzeugten Expressionsvektoren erhielten die Bezeichnungen (pcDNA3.1(+)mPGKPA-Z/TM, pcDNA3.1(+)hPGKPA-Z/TM, pcDNA3.1 (+)hDHFRPA-Z/TM, pcDNA3.1(+)hEF-1αPA-Z/TM, pDNA3.1(+)hUbCPA-Z/TM, pcDNA3.1(+)hICIPA-Z/TM, sowie pcDNA3.1(+)dNeomPGKPA-Z/TM, pcDNA3.1(+)dNeohPGKPA-Z/TM, pcDNA3.1(+)dNeohDHFRPA-Z/TM, pcDNA3.1(+)dNeohEF-1αPA-Z/TM, pcDNA3.1(+)dNeohUbCPA-Z/TM, pcDNA3.1(+)dNeohICIPA-Z/TM, pcDNA3.1(+)-Z/TM (Figur 4) und pcDNA3.1(+)dNeo-Z/TM beziehungsweise (pcDNA3.1(+)mPGKPA-MBP/TM, pcDNA3.1(+)hPGhPA-MBP/TM, pcDNA3.1(+)hDHFRPA-MBP/TM, pcDNA3.1(+)hEF-1αPA-MBP/TM, pcDNA3.1(+)hUbCPA-MBP/TM, pcDNA3.1(+)hICIPA-MBP/TM, sowie pcDNA3.1(+)dNeomPGKPA-MBP/TM, pcDNA3.1(+)dNeohPGKPA-MBP/TM, pcDNA3.1(+)dNeohDHFRPA-MBP/TM, pcDNA3.1(+)dNeohEF-1αPA-MBP/TM, pcDNA3.1(+)dNeohUbCPA-MBP/TM, pcDNA3.1(+)dNeohICIPA-MBP/TM, pcDNA3.1(+)-MBP/TM und pcDNA3.1(+)dNeo-MBP/TM.

Die Korrektheit der in den Beispielen 1 bis 3 beschriebenen Konstrukte wurde durch Sequenzierung bestätigt.

Die in dem Beispielen 1 bis 3 beschriebenen Expressionsvektoren finden Verwendung bei den erfindungsgemäßen Verfahren zur Suche von T-Zellepitopen, bzw. zum Nachweis von T-Zellen mit bekannter Epitoprestriktion, bei welchen die meßbare Veränderung der Expression eines natürlicherweise in der APC exprimierten Polypeptids infolge einer Epitop-spezifischen Erkennung mittels einer T-Zelle, beispielsweise die natürlicherweise gesteigerte Expression des OX40Liganden oder des 4-1BB Liganden als Marker dient.

### Beispiel 4:

Klonierung von Gentransfervektoren, umfassend einen spezifisch in antigenpräsentierenden Zellen durch den Epitop-spezifischen Kontakt mit einer T-Zelle induzierbaren ersten Promotor, eine mit diesem ersten Promotor funktionell verbundene Nukleinsäure kodierend ein Markergen, einen in antigenpräsentierenden Zellen konstitutiven zweiten Promotor, und eine mit diesem zweiten Promotor funktionell verbundene Nukleinsäure.

Klonierung des erfindungsgemäßen Vektorbackbones pcDNA3.1(+)OX40LAeGFP, sowie der erfindungsgemäßen Vektoren pcDNA3.1(+)OX40LAeGFP-Z und pcDNA3.1(+)OX40LAeGFP-MBP.

Als Ausgangsvektor zur Klonierung des erfindungsgemäßen Vektorbackbones pcDNA3.1(+)OX40LAeGFP (Figur 5A), sowie der erfindungsgemäßen Vektoren pcDNA3.1(+)OX40LAeGFP-Z (Figur 5B) und pcDNA3.1(+)OX40LAeGFP-MBP (Figur 5C) diente das Plasmid pcDNA3.1(+) (Invitrogen). Der durch einen Epitop-spezifischen Kontakt einer T-Zelle mit einer Epitop-präsentierenden APC induzierbare Promotor des OX40-Liganden wurde aus Gesamt-DNS aus humanen PBMC mittels des PCR-Verfahrens amplifiziert.

Die 979 bp umfassende Sequenz des Promotors des OX40 Liganden wurde mittels PCR unter Verwendung nachfolgender Amplifikationsbedingungen erzeugt: Einleitender Denaturierungsschritt: 95° C, 2 Min., danach folgten zwei aufeinanderfolgende 3-Stufen PCR mit folgenden Bedingungen: Erste PCR Amplifikationsrunde: (10 Zyklen) Denaturierung: 95° C, 45 Sek., Annealing 50° C, 1 Min., Elongation: 72° C, 2 Min.1; direkt anschließend folgte eine zweite PCR Amplifikationsrunde (30 Zyklen) mit folgenden Bedingungen: Denaturierung: 95° C, 45 Sek., Annealing 60° C, 1 Min., Elongation: 72° C, 2 Min. mit einem abschließenden Polymerisationsschritt bei 72° C für 10 Min. mit anschleißender Dauerkühlung bei 4° C. Zur Amplifikation wurden die Primer P35 (5' Primer: 5'-GGCGGGG*GATCC*GGTACCTGGTGTCTATTG-3'; SEQ ID NO:40) und P36 (3` Primer: 5'-GGCGGG*AGATCT*CTTCACAATCTGGGTAG-3'; SEQ ID NO:41) verwendet. Die in den Primern enthaltenen Schittstellen (*Bam*H1 im 5' Primer; *Bgl*II im 3' Primer sind kursiv dargestellt). Die Sequenz des amplifizierten PCR Fragments ist in Figur 5 und SEQ ID NO:1 dargestellt. Das erhaltene PCR-Produkt wurde aufgereinigt und mit den Restriktionsenzymen *Bam*H1 und *Bgl*II verdaut, aufgereinigt und in das mit dem Restriktionsenzym Bg*l*II linearisierte Plasmids pcDNA3.1(+) kloniert. Die erhaltenen Spaltprodukte wurden Das in der Art erzeugte, 6414 bp (bp) umfassende Plasmid wurde pcDNA3.1(+)OX40L genannt.

Anschließend wurde die 224 bp umfassende *bovine growth hormone* (BGH) Polyadenylierungssequenz aus dem pcDNA3.1(+) Vektor mittels PCR unter Verwendung geeigneter PCR Bedingungen (Einleitender Denaturierungsschritt: 95° C, 2 Min., anschließende 3-Stufen PCR (35 Zyklen) mit folgenden Bedingungen: Denaturierung: 95° C, 30 Sek.; Annealing 55° C, 1 Min.; Elongation:. 72° C, 1 Min., und einem abschließenden Polymerisationsschritt bei 72° C für 10 Min. mit anschließender Dauerkühlung bei 4° C) amplifiziert. Zur Amplifikation wurden die Primer P37 (5' Primer: 5'-GGCGGG*AGATC*CTGTGCCTTCTAGTTGCC-3'; SEQ ID NO:42) und P38 (3' Primer: 5'-GGCGGG*GGATCC*CCATAGAGCCCACCGC-3'; SEQ ID NO:43) verwendet. Die in den Primern enthaltenen Schittstellen (*Bgl*II im 5' Primer; B*am*HI im 3' Primer sind kursiv dargestellt). Die mittels der PCR amplifizierte Bande wurde dann wie bereits beschrieben aufgereinigt, mit den Restriktionsenzymen *Bgl*II und *Bam*HI gespalten und die in der Art erhaltene Bande in das mit *Bgl*II linearisierte Plasmid pcDNA3.1(+)OX40L ligiert. Der so erzeugte Vektor wurde pcDNA3.1(+)OX40LA genannt.

Anschließend wurde das Gen für das "*enhanced green fluorescent protein*" (eGFP) mittels PCR unter Verwendung geeigneter PCR Bedingungen (Einleitender Denaturierungsschritt: 95° C, 2 Min., anschließende 3-Stufen PCR (35 Zyklen) mit folgenden Bedingungen: Denaturierung: 95° C, 30 Sek.; Annealing 55° C 1 Min.; Elongation: 72° C, 2 Min., und einem abschließenden Polymerisationsschritt bei 72° C für 10 Min. mit anschließender Dauerkühlung bei 4° C) aus dem Plasmid pRC/CMV-EGFP (Vogel et al., 1998, BioTechniques 143, 1967-1983) amplifiziert. Zur Amplifikation wurden die Primer P39 (5' Primer: 5'-GGCGGG*GGATCC*CGCCACCATGGTGAGCAAGG-3'; SEQ ID NO:44) und P40 (3' Primer: 5'-GGCGGG*AGATCT*TTACTTGTACAGCTCGTCC-3'; SEQ ID NO:45) verwendet. Die mittels der PCR amplifizierte spezifische Bande mit einer Größe von 752 bp wurde dann wie vorstehend beschrieben aufgereinigt, mit den Restriktionsenzymen *Bam*HI und *Bgl*II gespalten und die erhaltene Bande in das mit *Bgl*II linearisierte Plasmid pcDNA3.1(+)OX40LA ligiert. Der so erzeugte 7378 bp umfassende Vektor wurde pcDNA3.1(+)OX40LAeGFP (Figur 5A) genannt. Dieser Vektor kann als Vektorbackbone für die Klonierung der erfindungsgemäßen Vektoren zur Epitopsuche und dem Nachweis reaktiver T-Zellen verwendet werden.

Anschließend wurde die cDNS für das EBV BZLF1 Protein mittels PCR unter Verwendung geeigneter PCR-Bedingungen (Einleitender Denaturierungsschritt: 95° C, 2 Min., darauffolgend eine 3-Stufen PCR (15 Zyklen) mit folgenden Bedingungen: Denaturierung: 95° C, 30 Sek.; Annealing 52° C, 1 Min.; Elongation: 72° C, 2 Min., danach nochmals (25 Zyklen) mit folgenden Bedingungen: Denaturierung: 95° C, 30 Sek.; Annealing 63° C, 1 Min.; Elongation: 72° C, 2 Min., und einem abschließenden Polymerisationsschritt bei 72° C für 10 Min. mit anschließender Dauerkühlung bei 4°C) aus viraler DNS des EBV Stammes B95-8 in der Gesamt-DNS infizierter Marmoset-Zellen amplifiziert. Bei der PCR Reaktion zur Amplifikation des BZLF1 cDNS wurde dem PCR Ansatz 2,5% DMSO beigefügt. Zur Amplifikation wurden die Primer P41 (5' Primer: 5'-GGCGG*TCTAGA*TTAGAAATTTAAGAGATCC-3'; SEQ ID NO:46) und Primer P42 (3' Primer: 5'-GGCGGG*GAATTC*ATGATGGACCCAAACTCG-3'; SEQ ID NO:47) verwendet. Die mittels der PCR amplifizierte 970 Nukleotide umfassende Bande wurde dann wie vorstehend beschrieben aufgereinigt, mit den Restriktionsenzymen *Xba*I und *Eco*RI gespalten und die erhaltene Bande in das ebenfalls mit *Eco*RI und *Xba*I linearisierte Plasmid pcDNA3.1(+)OX40LAeGFP ligiert. Der so erzeugte Vektor wurde pcDNA3.1(+)OX40LAeGFP-Z (Figur 5B) genannt.

Alternativ wurde die cDNS für das MBP-Protein mittels PCR unter Verwendung geeigneter Bedingungen (Einleitender Denaturierungsschritt: 95° C, 2 Min.; darauf folgend eine 3-Stufen PCR (35 Zyklen) mit folgenden Bedingungen: Denaturierung: 95° C, 30 Sek.; Annealing 55° C, 1 Min.; Elongation: 72° C, 2 Min., und einem abschließenden Polymerisationsschritt bei 72° C für 10 Min. mit anschließender Dauerkühlung bei 4° C) aus Gesamt DNS von Hirnzellen amplifiziert. Zur Amplifikation wurden die Primer P43 (5' Primer: 5'-GGCGG*TCTAGA*ATGGCGTCACAGAAGAGACC-3 ; SEQ ID NO:48) und P44 (3' Primer: 5'-GGCGGG*GAATTC*TCAGCGTCTAGCCATGGG-3'; SEQ ID NO:49) verwendet. Die mittels der PCR amplifizierte 585 Nukleotide umfassende Bande wurde dann wie vorstehend beschrieben aufgereinigt, mit den Restriktionsenzymen *Xba*I und *Eco*RI gespalten und die erhaltene Bande in das ebenfalls mit *Eco*RI und *Xba*I linearisierte Plasmid pcDNA3.1(+)OX40LAeGFP ligiert. Der so erzeugte Vektor wurde pcDNA3.1(+)OX40LAeGFP-MBP (Figur 5C) genannt.

Zudem wurde in alle in den Beispielen 1-3 beschriebenen Expressionsplasmide ein spezifisch in antigenpräsentierenden Zellen durch den Epitop-spezifischen Kontakt mit einer T-Zelle induzierbarer erster Promotor (P1), hier exemplarisch der OX40 Ligand Promotor und 4 Varianten des 4-1BB Ligand Promotors, sowie eine mit diesem ersten Promotor funktionell verbundene Nukleinsäure kodierend ein Markergen, hier exemplarisch das eGFP eingefügt.

Hierbei wurden drei Nukleinsäurebausteine; (I) die Sequenz eines in APC induzierbaren ersten Promotors (P1), eine BGH Polyadenylierungssequenz und eine für einen Marker, exemplarisch die für eGFP kodierende Nukleinsäure jeweils nach erfolgter PCR Amplifikation mit den entsprechenden Restrinktionsendonukleasen geschnitten, gereinigt und nacheinander in drei Schritten jeweils mit *Bgl*II geschnittenen Expressionsvektoren eingebracht. Hierbei wurde für alle Klonierungen die *Bgl*II Schnittstelle, verwendet, welche derer an Position 13 des allen erzeugten Plasmiden zugrundeliegenden Vektors pcDNA3.1(+) (Invitrogen) lokalisiert ist. Anderweitig in den Vektoren aufgrund der vorgenommenen Klonierungen erzeute *Bgl*II Schnittstellen wurden mittels eines QuickChange Site-Directed Mutagenesis Kits nach dem Protokoll des Herstellers (Stratagene) in der Art zerstört, daß die Aminosäuresequenz des Polypeptids, beziehungsweise die Funktionalität der Nukleinsäure unverändert bleibt. Die Auswahl geeigneter Oligonukleotide zur Mutagenese und die Durchführung des Verfahrens sind etabliert und Stand der Technik.

### Amplifikation des humanen OX40 Ligand Promotors

Die 979 bp umfassende Sequenz des murinen PGK Promotors wurde mittels PCR aus isolierter chromosomaler DNA aus humanen PBMCs unter Verwendung nachfolgender Bedingungen gewonnen: Einleitender Denaturierungsschritt: 95° C, 2 Min., danach folgten zwei aufeinanderfolgende 3-Stufen PCR mit folgenden Bedingungen: Erste PCR Amplifikationsrunde: (10 Zyklen) Denaturierung: 95° C, 45 Sec., Annealing 50° C, 1 Min., Elongation: 72° C, 2 Min., direkt anschließend folgte eine zweite PCR Amplifikationsrunde (30 Zyklen) mit folgenden Bedingungen: Denaturierung: 95° C, 45 Sec., Annealing 60° C, 1 Min., Elongation: 72° C, 2 Min. mit einem abschließenden Polymerisationsschritt bei 72° C für 10 Min. mit anschleißender Dauerkühlung bei 4° C. Zur Amplifikation wurden die Primer P45 (5' Primer: 5'-GGCGGG*GGATCC*GGTACCTGGTGTCTATTG-3'; SEQ ID NO:50) und P46 (3' Primer: 5'-GGCGGG*AGATCT*CTTCACAATCTGGGTAG-3'; SEQ ID NO:51) verwendet. Die in den Primern enthaltenen Schittstellen (*Bam*H1 im 5' Primer; *Bgl*II im 3' Primer sind kursiv dargestellt).

### Amplifikation des humanen 4-1BB Ligand Promotors (Lange Variante des 4-1BB Ligand Promotors (V1))

Die 2047 bp umfassende Sequenz des humanen 4-1BB Ligand Promotors wurde mittels PCR aus isolierter chromosomaler DNA aus humanen PBMCs unter Verwendung nachfolgender Amplifikationsbedingungen isoliert: Einleitender Denaturierungsschritt: 98° C, 2 Min., danach folgten drei aufeinanderfolgende 3-Stufen PCR mit folgenden Bedingungen: Erste PCR Amplifikationsrunde: (30 Zyklen) Denaturierung: 95° C, 1 Min., Annealing 66,1° C, 1 Min., Elongation: 72° C, 4,5 Min., direkt anschließend folgte eine zweite PCR Amplifikationsrunde (15 Zyklen) mit folgenden Bedingungen: Denaturierung: 98° C, 1 Min., Annealing 58° C, 1 Min., Elongation: 72° C, 4,5 Min. und eine dritte PCR Amplifikationsrunde (25 Zyklen) mit folgenden Bedingungen: Denaturierung: 98° C, 1 Min., Annealing 71° C, 1 Min., Elongation: 72° C, 4,5 Min. mit einem abschließenden Polymerisationsschritt bei 72° C für 10 Min. mit anschleißender Dauerkühlung bei 4° C. Zur Amplifikation wurden die Primer P47 (5' Primer: 5'- GGCGGG*GGATCC*CCGGATTGGCCGCCTCCAGCAG-3'; SEQ ID NO:52) und P48 (3' Primer: 5'-GGCGGG*GGATCC*GACGAGAGACTGCGGGAAGACAC-3 ; SEQ ID NO:53) verwendet.

Das erhaltene PCR Amplifikat wurde dann gereinigt, mit *Bam*HI geschnitten und in den ebenfalls mit *Bam*H1 gespaltenen pcDNA3.1(+) Vektor inseriert. Das erhaltene Konstrukt wurde als pcDNA3.1(+)4-1BBL bezeichnet. Dann wurde die *Bgl*II Schnittstelle innerhalb des 4-1BB Ligand Promotors (an Position 1625 bis 1630 des 2023 Nukleotide umfassenden Volllänge-Promotors) mittels eines QuickChange Site-Directed Mutagenesis Kits nach dem Protokoll des Herstellers (Stratagene) durch den Austausch eines Nukleotides in Position 1626 (1626 G zu A)-(1624-AGATCT-1631) zu 1626 (1624-AAATCT-1631) zerstört. Das resultierende Plasmid erhielt die Bezeichung pcDNA3.1(+)4-1BBL1626.

### Amplifikation des mutierten (1626 G zu A) (humanen 4-1BB Ligand Promotors (Lange Variante des 4-1 BB Ligand Promotors (V1))

Die 2047 bp umfassende Sequenz des humanen 4-1BB Ligand Promotors wurde mittels PCR aus dem Plasmid pcDNA3.1(+)4-1BBL1626 unter Verwendung nachfolgender Amplifikationsbedingungen isoliert: Einleitender Denaturierungsschritt: 98° C, 2 Min., danach folgten drei aufeinanderfolgende 3-Stufen PCR mit folgenden Bedingungen: Erste PCR Amplifikationsrunde: (30 Zyklen) Denaturierung: 95° C, 1 Min., Annealing 66,1° C, 1 Min., Elongation: 72° C, 4,5 Min., direkt anschließend folgte eine zweite PCR Amplifikationsrunde (15 Zyklen) mit folgenden Bedingungen: Denaturierung: 98° C, 1 Min., Annealing 58° C, 1 Min., Elongation: 72° C, 4,5 Min. und eine dritte PCR Amplifikationsrunde (25 Zyklen) mit folgenden Bedingungen: Denaturierung: 98° C, 1 Min., Annealing 71° C, 1 Min., Elongation: 72° C, 4,5 Min. mit einem abschließenden Polymerisationsschritt bei 72° C für 10 Min. mit anschleißender Dauerkühlung bei 4° C. Zur Amplifikation wurden die Primer P49 (5' Primer: 5'-GGCGGG*GGATCC*CCGGATTGGCCGCCTCCAGCAG-3 ; SEQ ID NO:54) und P50 (3' Primer: 5'-GGCGGG*AGATCT*GACGAGAGACTGCGGGAAGACAC-3'; SEQ ID NO:55) verwendet.

### Amplifikation des humanen 4-1BB Ligand Promotors (im 5' Bereich verkürzte Variante des 4-1BB Ligand Promotors (V2))

Die 1433 bp umfassende Sequenz des humanen 4-1BB Ligand Promotors (V2) wurde mittels PCR aus dem Plasmid pcDNA3.1(+)4-1BBL1626 unter Verwendung nachfolgender Amplifikationsbedingungen isoliert: Einleitender Denaturierungsschritt: 98° C, 2 Min., danach folgten drei aufeinanderfolgende 3-Stufen PCR mit folgenden Bedingungen: Erste PCR Amplifikationsrunde: (30 Zyklen) Denaturierung: 95° C, 1 Min., Annealing 64,1° C, 1 Min., Elongation: 72° C, 4,5 Min., direkt anschließend folgte eine zweite PCR Amplifikationsrunde (15 Zyklen) mit folgenden Bedingungen: Denaturierung: 98° C, 1 Min., Annealing 58° C, 1 Min., Elongation: 72° C, 3,5 Min. und eine dritte PCR Amplifikationsrunde (25 Zyklen) mit folgenden Bedingungen: Denaturierung: 98° C, 1 Min., Annealing 69° C, 1 Min., Elongation: 72° C, 3,5 Min. mit einem abschließenden Polymerisationsschritt bei 72° C für 10 Min. mit anschleißender Dauerkühlung bei 4° C. Zur Amplifikation wurden die Primer P51 (5' Primer: 5'-GGCGGG*GGATCC*GGAGCCAGAGATAGGGAGAGTC-3'; SEQ ID NO:56) und P50 (3' Primer: 5'-GGCGGG*AGATCT*GACGAGAGACTGCGGGAAGACAC-3'; SEQ ID NO:55) verwendet.

### Amplifikation des humanen 4-1BB Ligand Promotors (im 5' Bereich weiter verkürzte Variante des 4-1BB Ligand Promotors (V3))

Die 874 bp umfassende Sequenz des humanen 4-1BB Ligand Promotors (V3) wurde mittels PCR aus dem Plasmid pcDNA3.1(+)4-1BBL1626 unter Verwendung nachfolgender Amplifikationsbedingungen isoliert: Einleitender Denaturierungsschritt: 98° C, 2 Min., danach folgten drei aufeinanderfolgende 3-Stufen PCR mit folgenden Bedingungen: Erste PCR Amplifikationsrunde: (30 Zyklen) Denaturierung: 95° C, 1 Min., Annealing 66° C, 1 Min., Elongation: 72° C, 2 Min., direkt anschließend folgte eine zweite PCR Amplifikationsrunde (15 Zyklen) mit folgenden Bedingungen: Denaturierung: 98° C, 1 Min., Annealing 55° C, 1 Min., Elongation: 72° C, 2 Min. und eine dritte PCR Amplifikationsrunde (25 Zyklen) mit folgenden Bedingungen: Denaturierung: 98° C, 1 Min., Annealing 69° C, 1 Min., Elongation: 72° C, 2 Min. mit einem abschließenden Polymerisationsschritt bei 72° C für 10 Min. mit anschleißender Dauerkühlung bei 4° C. Zur Amplifikation wurden die Primer P52 (5' Primer: 5'- GGCGGG*GGATCC*CAGCCAGAGACAGCGACAGAG-3'; SEQ ID NO:57) und P50 (3' Primer: 5'-GGCGGG*AGATCT*GACGAGAGACTGCGGGAAGACAC-3'; SEQ ID NO:55) verwendet.

### Amplifikation des humanen 4-1BB Ligand Promotors (im 5' Bereich weiter verkürzte Variante des 4-1BB Ligand Promotors (V4))

Die 386 bp umfassende Sequenz des humanen 4-1BB Ligand Promotors (V4) wurde mittels PCR aus dem Plasmid pcDNA3.1(+)4-1BBL1626 unter Verwendung nachfolgender Amplifikationsbedingungen isoliert: Einleitender Denaturierungsschritt: 98° C, 2 Min., danach folgten drei aufeinanderfolgende 3-Stufen PCR mit folgenden Bedingungen: Erste PCR Amplifikationsrunde: (30 Zyklen) Denaturierung: 95° C, 1 Min., Annealing 66° C, 1 Min., Elongation: 72° C, 2 Min., direkt anschließend folgte eine zweite PCR Amplifikationsrunde (15 Zyklen) mit folgenden Bedingungen: Denaturierung: 98° C, 1 Min., Annealing 55° C, 1 Min., Elongation: 72° C, 2 Min. und eine dritte PCR Amplifikationsrunde (25 Zyklen) mit folgenden Bedingungen: Denaturierung: 98° C, 1 Min., Annealing 69° C, 1 Min., Elongation: 72° C, 2 Min. mit einem abschließenden Polymerisationsschritt bei 72° C für 10 Min. mit anschleißender Dauerkühlung bei 4° C. Zur Amplifikation wurden die Primer P53 (5' Primer: 5'-GGCGGG*GGATCC*CCCACTGCAGAGGCAATCAACAAG-3'; SEQ ID NO:58) und P50 (3' Primer: 5'-GGCGGG*AGATCT*GACGAGAGACTGCGGGAAGACAC-3'; SEQ ID NO:55) verwendet.

Die 224 bp umfassende *bovine growth hormone* (BGH) Polyadenylierungssequenz und das Gen für das "*enhanced green fluorescent protein*" (eGFP) wurden wie bereits in Beispiel 4 beschrieben mittels PCR gewonnen.

Die drei Nukleinsäurekomponenten zur Expression eines Markers mittels eines induzierbaren Promotors (P1); nämlich die Nukleinsäuresequenz des Promotors, einer BGH Polyadenylierungs-Signalsequenz und einer für einen Marker kodierenden Nukleinsäure wurden wie nachfolgend beschrieben sequenziell in die nachfolgend aufgelisteten Plasmide eingefügt. Schritt 1: Verdau der mittels PCR gewonnenen Nukleinsäure des Promotors mit den Enzymen *Bam*H1 und *Bgl*II und Ligation der so gewonnenen Nukleinsäure in mit *Bgl*II geschnittenen Zielvektoren. Schritt 2: Verdau der mittels PCR gewonnenen Nukleinsäure des BGH Polyadenylierungs-Signalsequenz mit den Enzymen *Bgl*II und *Bam*H1 und Ligation der so gewonnenen Nukleinsäure in die in Schritt 1 erzeugten und erneut mit *Bgl*II geschnittenen Zielvektoren. Schritt 3: Verdau der mittels PCR gewonnenen Nukleinsäure des Markers (eGFP) mit den Enzymen *Bam*H1 und *Bgl*II und Ligation der so gewonnenen Nukleinsäure in die in Schritt 2 erzeugten und erneut mit *Bgl*II geschnittenen Zielvektoren.

Mit diesem Verfahren wurden 4 verschiedene spezifisch in antigenpräsentierenden Zellen durch den Epitop-spezifischen Kontakt mit einer T-Zelle induzierbare erste Promotoren (OX40L-, 4.1BBL(V1)- , 4.1BBL(V2)- 4.1BBL(V3)- und 4.1BBL(V1)-Promotor, sowie eine mit diesem ersten Promotor funktionell verbundene Nukleinsäure kodierend ein Markergen (eGFP) in die Plasmide pcDNA3.1(+), pcDNA3.1(+)dNeo, sowie in die in den Beispielen 1 bis 3 aufgelisteten Expressionsvektoren, enthaltend einen in antigenpräsentierenden Zellen konstitutiven zweiten Promotor, und eine mit diesem zweiten Promotor funktionell verbundene Nukleinsäure. Die in der Art erzeugten Plasmide wurden durch das Anfügen der Promotorbezeichnung (OX40L, 4.1BBL(V1), 4.1BBL(V2), 4.1BBL(V1) oder 4.1BBL(V1) an den ursprünglichen Namen gekennzeichnet. So wird beispielsweise das Plasmid pcDNA3.1(+)-Z/LAMP nach Einfügen der Nukleinsäuren für den OX40 Ligand Promotor, die BGH Polyadenylierungs-Signalsequenz und den Marker eGFP pcDNA3.1(+)-Z/LAMP/OX40L (Figur 5D) genannt.

Mögliche, den Ablauf der in den Beispielen 1 bis 4 beschriebenen Klonierungen störende Restrinktionsendonukleasespaltstellen wurden mittels eines QuickChange Site-Directed Mutagenesis Kits nach dem Protokoll des Herstellers (Stratagene) in der Art zerstört, daß die Aminosäuresequenz des Polypeptids, beziehungsweise die Funktionalität der Nukleinsäure unverändert bleibt. Die Auswahl geeigneter Oligonukleotide zur Mutagenese und die Durchführung des Verfahrens sind etabliert und Stand der Technik. Die Korrektheit der Sequenz und Orientierung der in die jeweiligen Vektoren integrierten Polynukleotide wurde mittels Sequenzierung bestätigt.

### Beispiel 5:

### Transfection der erfindungsgemäßen Vektoren in Populationen aufgereinigter APC oder gereinigter PBMC mittels der Nucleovector^{™} Technologie (Amaxa)

Die Gewinnung von peripheren mononukleären Zellen des Blutes (PBMC) aus dem Vollblut von freiwilligen Spendern oder Patienten erfolgte wie nachfolgend beschrieben: Frisch abgenommenes Vollblut (50 bis 100 ml; nicht älter als 8 h) eines gesunden Probanden wurde mit 20 IU/ml Heparin versetzt. 50 ml-Leukosep-Gefäße mit Trennmembran wurden mit 15 ml Ficoll-Histopaque (Sigma, Deisenhofen) befüllt und 2 Min. bei 500 x g zentrifugiert. Die Röhrchen wurden dann mit dem heparinisierten Vollblut befüllt und 1:1 (Vol/Vol) mit sterilem PBS/0,5 % BSA versetzt. Die Ansätze wurden 30 bis 40 Min. bei 400 x g zentrifugiert, ohne den Rotor am Ende des Laufes abzubremsen. Die lymphozytenhaltige, trübe Interphase wurde vorsichtig abgezogen und in 3 Volumina T-Zell-Medium (RPMI 1640 Medium mit 10% hitzeinaktiviertem (30 Min. bei 56° C) humanem AB Serum, 2 mM Glutamin und 100 mg/ml Kanamycin bzw. Gentamycin (alle Komponenten von PanSystems, Aidenbach)) dreimal gewaschen (200 x g; 10 bis 15 Min. bei Raumtemperatur) und mit einer Zellzahl von 2 x 10⁶ Zellen/ml in RPMI 1640 Medium (Gibco) mit 10% humanem AB Serum, 100µg/ml Streptomycin, 100 U/ml Penicillin, und 2 mM GlutaMAX (Invitrogen/Live Technologies) aufgenommen und bis zur weiteren Aufreinigung von definierten APC- und T-Zellpopulationen bei 37°C in einem befeuchteten Brutschrank mit 5% CO₂ Begasung aufbewahrt. Mittels dieses Verfahrens werden aus 100 ml Vollblut (60% Erythrozyten; 40% Leukozyten) etwa 1 x 10⁷ bis 1 x 10⁸ PBMCs mit einer durchschnittlichen Zusammensetzung von etwa 60%-70% T-Zellen, 5%-10% B-Zellen, 10% - 20% Monozyten/Makrophagen und etwa 10% sonstiger Zellen gewonnen.

Die Aufreinigung von B-Zellen aus frisch präparierten PBMC erfolgte durch positive Selektion mittels immunomagnetischer CD19 *MicroBeats* nach dem Protokoll des Herstellers (Miltenyi Biotec).

Dazu wurden isolierte PBMC gezählt und in einer Konzentration von 10⁷ Zellen/80 µl MACS Puffer (PBS, 0,5% BSA und 2 mM EDTA, entgast) aufgenommen. Pro 10⁷ PBMC wurden 20 µl MACS CD19 *MicroBeads* zugesetzt und 15 Min. bei 6°C inkubiert. Anschließend wurde die markierte Zellsuspension mit 2 ml Puffer pro 10⁷ PBMC versetzt und 10 Min. bei 300 x g in einer Hettich-Rotanta-Tischzentrifuge zentrifugiert. Der Überstand wurde sorgfältig abgezogen und die Zellen in 500 µl MACS-Puffer aufgenommen. Vor dem Auftragen auf die Säule wurden die Zellen mittels Filtration durch einen Zellsieb, 70 µm Maschengröße (BectonDickinson, Heidelberg) vereinzelt. Zur positiven Selektion der magnetisch markierten Zellen wurden Säulen des Typs XS+ gewählt (max. 10⁹ positive Zellen) und in einem MACS Separator (Miltenyi Biotec, Deutschland) plaziert. Die Säulen wurden ein- bis zweimal mit 10 ml MACS Puffer gewaschen bis der Durchfluß klar war. Anschließend wurden die vereinzelten Zellen auf die vorbereitete Säule aufgetragen und die Negativfraktion eluiert. Die Säule wurde wiederum 3 x mit MACS Puffer gewaschen, aus dem MACS-Separator genommen und in ein steriles Plastikgefäß überführt. Die B-Zellen wurden anschließend durch Auftrag von MACS-Puffer und Ausübung eines leichten Drucks mittels eines zur Säule gehörenden Stempels eluiert. Danach wurden die Zellen bis zur Transfektion mit einer Zellzahl von 1 x 10⁶ Zellen/ml in RPMI 1640 Medium (Gibco) mit 10% humanem AB Serum, 100µg/ml Streptomycin, 100 U/ml Penicillin, und 2 mM GlutaMAX (Invitrogen/Live Technologies) bei 37°C in einem befeuchteten Brutschrank mit 5% CO₂ Begasung aufbewahrt.

In gleicher Weise wurden CD3⁺ T-Zellen bei Verwendung von MACS CD3 *MicroBeads* durch positive Selektion aus PBMCs depletiert und die erhaltene Zellsuspension vor dem Nukleinsäuretransfer mittels FACS bezüglich seiner Zusammensetzung charakterisiert.

Die erhaltenen Zellpopulationen wurden anschließend mittels eines FACS-Gerätes unter Verwendung geeigneter, Farbstoff-markierter Antikörper gegen zelltypspezifische Oberflächenmoleküle (CDs) bezüglich ihrer Zusammensetzung charakterisiert. Mit diesem Verfahren konnten B-Zellen mit einer Reinheit von etwa 83 % gewonnen werden (Siehe Tabelle 1A). In Analogie wurden CD3-positive T-Zellen mittels des Miltenyi Verfahrens nach der Vorschrift des Herstellers aus PBMCs depletiert und die resultierende Zellpopulationen mittels FACS-Analysen charakterisiert. Die erhaltene Zellsuspension setzte sich aus 3,37% B-Zellen (CD19+), 58 CD4+ Zellen (Monozyten und verbleibenden T-Zellen), ca. 1,55 % kontaminierender T-Zellen (CD3⁺); davon ca. 0,35 % T-Helferzellen und 1,2 % CTL (CD8+) und 58 % Monozyten (CD3⁻, CD4⁺) zusammen.

Transfektion von CD3⁺-depletierten PBMC bzw. gereinigten B-Zellen mit den erfindungsgemä-ßen Vektoren.

Exemplarisch erfolgte die Transfektion von CD3⁺-depletierten PBMC (Figur 6A) bzw. der gereinigten B-Zellen (Figur 6B) mit dem erfindungsgemäßen Vektor pcDNA3.1(+)dNeo-eGFP mittels der Nucleovector^{™} Technologie unter Verwendung des *Human B Cell Nucleofector^{™}* Kits nach dem detaillierten Protokoll des Herstellers (Amaxa, Deutschland). Zur Kontrolle wurden die Zellen mit dem Plasmid pcdNA3.1(+) behandelt. Hierbei wurden je 1 x 10⁷ bis 3 x 10⁷ CD3-depletierte PBMCs bzw. gereinigte CD19⁺ B-Zellen pro abzentrifugiert (200 x g, 10 Min. bei Raumtemperatur) und die sedimentierten Zellen mit einer Konzentration von 1 x 10⁶ bis 3 x 10⁶ Zellen/ml in einer "Human B cell Nucleofactor" Lösung resuspendiert. Danach wurden 100 µl der Zellsuspension mit 5 µl DNS versetzt und die Probe unter Vermeidung von Luftbläschen in eine Küvette pippetiert. Anschließend wurden die Zellen mittels eines Nucleofector Geräts unter Verwendung eines fest installierten Programms des Herstellers (Amaxa, Programm U-15) transfiziert. Nach erfolgter Transfection wurden 500 µl vorgewärmtes Medium (RPMI 1640 Medium (Gibco) mit 10% humanem AB Serum, 100µg/ml Streptomycin, 100 U/ml Penicillin, und 2 mM GlutaMAX (Invitrogen/Live Technologies)) zugegeben und die Zellen danach bei 37°C in einem befeuchteten Brutschrank mit 5% CO₂ Begasung inkubiert. Die Transfektionseffizienz wurde dann zu mehreren Zeitpunkten nach erfolgter Transfektion (beispielsweise 12 bis 72 Stunden, exemplarisch für 17 Stunden (Figuren 6A,B; Tabellen 2A-D)) mittels eines FACS Gerätes durch die Messung der Anzahl fluoreszierender Zellen (Nachweis eines GFP-Reporters) bestimmt. Dazu wurden die transfizierten Zellen 2 x mit FACS Puffer (PBS ohne bivalente Ionen, 1% FKS, 0,9 mg/ml Acid) gewaschen und danach mit einer Dichte von 5 x 10⁵ Zellen in FACS Puffer resuspendiert. Anschließend wurde dann mit Hilfe eines FACS Gerätes (FACS Calibur, BD) die Anzahl fluoreszierender Zellen durch Messung der Fluoreszenz 1 (FL1) gegen den Forwardscatter bestimmt. Diese Versuche ergaben, daß mittels der Nucleovector^{™} Technologie (Amaxa) ca. 13,5 % der B-Zellen und 30% der Monozyten in der gemischten Population von CD3-depletierten PBMCs eine signifikante eGFP Produktion aufweisen (Tabelle 2A). In Populationen hochreiner B-Zellen belief sich die Transfektionsrate von B-Zellen dagegen nur auf etwa 7,4% (Tabelle 2B). Mit einem Kontrollvektor (pcDNA3.1(+) transfizierte Zellen wiesen in diesen Experimenten keine signifikante eGFP Produktion auf (Tabellen 2C,D). Diese Untersuchungen belegen die Eignung der Nucleovector^{™} Technologie von Amaxa zur Transfer der erfiridungsgemäßen Vektoren in APC.

**Tabelle 1A: FACS-Analyse der Zusammensetzung der mittels des Miltenyi Verfahrens zur CD3 Depletion gewonnenen Population CD3⁺-depletierter PBMCs.**

| Zelltyp | % |
|---|---|
| CD19⁺ | 3,37 |
| CD4⁺ | 58,39 |
| CD3⁺ | 1,52 |
| CD3⁻CD4⁺ | 58,30 |
| CD3⁺CD4⁺ | 0,37 |
| CD3⁺CD8⁺ | 1,19 |

**Tabelle 2C: Nachweis der Fluoreszenz aus verschiedenen Zellpopulationen in CD3-depletierten PBMC mittels FACS-Analyse nach Nucleofektion mit pcDNA3.1+ (Fig. 6A, mitte)**

| % fluores. Zellen | |
|---|---|
| total | 0,28 |
| CD19⁺ | 0,70 |
| CD4⁺/Th | 1,03 |
| CD3⁺ | 0,88 |
| CD3⁺CD4⁻ | 0,27 |

**Tabelle 2A: Nachweis der Fluoreszenz aus verschiedenen Zellpopulationen in CD3-depletierten PBMC mittels FACS-Analyse nach Nucleofektion mit pcDNA3.1dNeo-eGFP (Fig. 6A, rechts)**

| % fluores. Zellen | |
|---|---|
| total | 34,94 |
| CD19⁺ | 13,48 |
| CD4⁺/Th | 3,79 |
| CD3⁺ | 5,01 |
| CD3⁺CD4⁻ | 30,06 |

**Tabelle 1B: FACS-Analyse der Zusammensetzung der mittels des Miltenyi Verfahrens zur B-Zellseparation gewonnenen B-Zellen.**

| Zelltyp | % |
|---|---|
| CD19⁺ | 83,38 |
| CD4⁺ | 0,96 |
| CD3⁺ | 5,77 |
| CD3⁺CD4⁺ | 1,79 |
| CD3⁺CD8⁺ | 1,13 |

**Tabelle 2D: Nachweis der Fluoreszenz in angereicherten ungereinigten B-Zellen mittels FACS nach Nucleofektion mit pcDNA3.1+ (Fig. 6B, mitte)**

| % fluores. Zellen | |
|---|---|
| total | 0,31 |
| CD19⁺ | 0,43 |

**Tabelle 2B: Nachweis der Fluoreszenz in angereicherten ungereinigten B-Zellen mittels FACS nach Nucleofektion mit pcDNA3.1dNeo-eGFP (Fig. 6B, rechts)**

| % fluores. Zellen | |
|---|---|
| total | 8,92 |
| CD19⁺ | 7,39 |

Im Gegensatz dazu konnten mittels der Femtosekunden Lasertechnologie bei verschiedenen gereinigten APC Populationen (Monozyten, B-Zellen, dendritische Zellen) Transfektionsraten von mehr als 50% erzielt werden. Hierbei wurden je 1-5 x 10⁵ APC in einer mineaturisierten sterilen Zellkammer in 0,5 ml Kulturmedium und 0,2 µg des pcDNA3.1(+)dNeo-eGFP Plasmids suspendiert. Dann wurden die Zellen nach einem verfügbaren Protokoll (Tirlapur and König, (2001) Nature, 418, 290) behandelt und die Transfektionseffizienz wie bereits beschrieben mittels eines FACS Gerätes bestimmt. Dieses Verfahren wird derzeit von der Firma Jenlab GmbH zur Produktreife entwickelt, ist jedoch derzeit noch nicht kommerziell verfügbar.

### Beispiel 6:

### Steigerung der Marker-Expression in erfindungsgemäßen APC nach Kontakt mit Epitop-spezifischen T-Zellen

Die Austestung der Eignung der erfindungsgemäßen APC zum Nachweis Epitop-spezifischer T-Zellen und zur Epitopsuche wurde anhand eines sehr gut charakterisierten Modelsystems durchgeführt. Dieses beruht auf den Versuchungsergebnissen, dass alle HLA B8-positiven, EBVpositiven Probanden mit hoher Vorläuferfrequenz CD8⁺ zytotoxische T-Zellen besitzten, die ein spezifisches Epitop (RAKFKQLL; Aminosäure 190-197) innerhalb des EBV Proteins BZLF1 erkennen (Bogedain et al., 1995, J. Virol. 69, 4872-4879; Benninger-Döring et al., 1999, Virol. 264, 289-297).

### A: Gesteigerte Expression eines natürlichen Markers (OX40L) auf transfizierten APC nach Inkubation mit dem erfindungsgemäßen Plasmid pcDNA3.1(+)dNeo-Z/LAMP

Bei diesen Untersuchungen wurden Monozyten eines HLA-B8 positiven EBV seropositiven Spender (SD) und zur Kontrolle einem HLA-B8 positiven EBV seronegativen Spender (SN) unter Verwendung eines Gemisches Antikörper-markierter Magnetischer Kügelchen (Miltenyi) nach dem Protokoll des Herstellers negativ aufgereinigt und die gereinigten Monozyten in RPMI Medium (10% FKS) durch Zugabe von 25 ng/ml IL-4 und 800 U/ml GM-CSF zu Dendritischen Zellen (DC) differenziert. Hierbei wurde am Tag 4 der Inkubation das Medium gewechselt und durch Medium mit GM-CSF, IL4 und 10 ng/ml TNF-α ersetzt. Nach einer 7 tägigen Kultur wurden die Zellen mittels der Nukleofector oder Femtolaser Technologie mit dem Plasmid pcDNA3.1(+)dNeo-Z/LAMP und zur Kontrolle mit dem Plasmid pcDNA3.1(+)dNeo transfiziert. Nach weiteren 12 bis 36 Stunden Kultur in RPMI 1640 Medium (Gibco) mit 10% humanem AB Serum, 100µg/ml Streptomycin, 100 U/ml Penicillin, und 2 mM GlutaMAX (Invitrogen/Live Technologies) wurden die Zellen dann in 20 parallelen Ansätzen in verschiedenen Verhältnissen (10:1 bis 1:10) von erfindungsgemäß veränderten DC und T-Zellen derselben Patienten mit einer Gesamtzellzahl von 5 x 10⁶ Zellen in RPMI 1640 Medium (Gibco) mit 10% humanem AB Serum, 100µg/ml Streptomycin, 100 U/ml Penicillin, und 2 mM GlutaMAX (Invitrogen/Live Technologies) für 2 bis 72 Stunden bei 37°C in einem befeuchteten Brutschrank mit 5% CO₂ Begasung inkubiert. Danach wurde die Expression des OX40 Liganden auf der Oberfläche der dendritischen Zellen mittels FACS Analyse unter Verwendung des OX40L-spezifischen Antikörpers (5A8, (Imura et al. (1996), J. Exp. Med. 183:2185) bestimmt. Hierbei konnte bei etwa 2 bis 20% (in Abhängigkeit von dem Versuchsprotokoll) der mit dem Plasmid pcDNA3.1(+)dNeo-Z/LAMP behandelten DC des HLA-B8 positiven EBV seropositiven Spender (SD) im Vergleich zu den mit dem selben Plasmid behandelten DC des HLA-B8 positiven EBV seronegativen Spender (SN) und den mit dem pcDNA3.1(+)dNeo Kontrollvektor behandelten DCs beider Spender eine meßbar gesteigerte Oberflächenexpression des OX40L beobachtet werden. Diese gesteigerte Oberflächenexpression des OX40L war über einen Zeitraum von 4 bis 72 Std. meßbar. Ähnliche Ergebnisse wurden in Kokultivierungsexperimenten bei Verwendung von B-Zellen und Monozyten als APC erzielt.

### B: Gesteigerte Expression eines natürlichen Markers (OX40L) auf transfizierten APC nach Transfektion mit den erfindungsgemäßen Plasmiden pcDNA3(+)OX40LAeGFP-Z und pcDNA3.1(+)dNeo-Z/LAMP/OX40L

Bei diesen Untersuchungen wurden B-Zellen sowie Monozyten eines HLA-B8 positiven EBV seropositiven Spender (SD) und zur Kontrolle einem HLA-B8 positiven EBV seronegativen Spender (SN) unter Verwendung spezifischer Antikörper-markierter Magnetischer Kügelchen (Miltenyi) nach dem Protokoll des Herstellers positiv aufgereinigt und nach einer 1 bis 24 stündigen Kultivierung mittels der Nukleofector oder Femtolaser Technologie mit den Plasmiden pcDNA3(+)OX40LAeGFP-Z und pcDNA3.1(+)dNeo-Z/LAMP/OX40L und, zur Kontrolle mit dem Vektor pcDNA3.1(+)dNeo transfiziert. Nach weiteren 12 bis 36 Stunden Kultur in RPMI 1640 Medium (10% humanem AB Serum, 100µg/ml Streptomycin, 100 U/ml Penicillin, und 2 mM GlutaMAX) wurden die Zellen dann in 20 parallelen Ansätzen in verschiedenen Verhältnissen (10:1 1 bis 1:10) von erfindungsgemäß veränderten APC (B-Zellen oder Monozyten) und T-Zellen derselben Patienten mit einer Gesamtzellzahl von 5 x 10⁶ Zellen in RPMI 1640 Medium (10% humanem AB Serum, 100µg/ml Streptomycin, 100 U/ml Penicillin, und 2 mM GlutaMAX) für 12 bis 72 Stunden bei 37°C in einem befeuchteten Brutschrank mit 5% CO₂ Begasung inkubiert. Danach wurde die Anzahl eGFP Marker-positiver APC mittels eines FACS Geräts, entsprechend dem in Beispiel 5 beschrieben Verfahren bestimmt. Hierbei konnte bei etwa 1 bis 20% (in Abhängigkeit von der Versuchsdurchführung) der mit den Plasmiden pcDNA3(+)OX40LAeGFP-Z und pcDNA3.1(+)dNeo-Z/LAMP/OX40L transfizierten APC des HLA-B8 positiven EBV seropositiven Spender (SD) im Vergleich zu den mit dem selben Plasmid behandelten DC des HLA-B8 positiven EBV seronegativen Spender (SD) und den mit dem pcDNA3.1(+)dNeo Kontrollvektor behandelten DCs beider Spender eine meßbar gesteigerte Markerproduktion beobachtet werden. Diese gesteigerte Fluoreszenz war über einen Zeitraum von 4 bis 72 Std. meßbar. Ähnliche Ergebnisse wurden bei Verwendung von DC als APC erzielt.

### Beispiel 7:

### Isolierung Marker-positiver APC mittels des FACSSorting Verfahrens und Isolierung von Polynukleotiden aus selektionierten Zellen

Zur Identifizierung der von den reaktiven T-Zellen des HLA B8-positiven Spenders (SD) erkannten Polypeptide wurden alle APC, welche eine erhöhte Markerexpression aufwiesen mittels der FACSSort Technologie selektioniert, gesammelt und aus diesen APC Gesamt DNS präpariert. Zur Transformation der Gesamt-DNS in Bakterien wurden 20 µl der DH10B Bakteriensuspension mit der gewonnenen Gesamt-DNS versetzt und etwa 5 mSec. bei einer Spannung von 2.400 V elektroporiert. Danach wurden die Bakterien kurz auf Eis abgekühlt, anschließend mit 1 ml LB-Medium versetzt und 1,5 Std. bei 37°C unter Schütteln inkubiert. Schließlich wurden unterschiedliche Mengen der Bakteriensuspension auf LB_{Amp} Selektionsplatten ausplattiert. Diese wurden über Nacht bei 37 °C inkubiert. In diesen Experimenten konnte durch Transfektion von 1 µg Gesamt-DNS eine signifikante Zahl Ampicillin-resistenter Klonien/Platte (10-100) erzeugt werden. Durch PCR-Analyse der Bakterienklone unter Verwendung geeigneter EBV BZLF1 spezifischer Primer, sowie durch Sequenzierung der aus den Bakterienklonen gewonnenen Plasmide mittels geeigneter Sequenzierprimer konnte gezeigt werden, dass mehr als 90%der resistenten Bakterienklone die für das BZLF1 Protein kodierenden ExpressionsvektorenVektoren enthielten.

### Beispiel 8.

### Nachweis spezifischer vektorkodierter Polynukleotide aus dem Genom stabil transfizierter Insektenzellen

In den frühen 90-ern wurde von Wolff und Mitarbeitern eine sehr sensitive Methode beschrieben, die es erlaubt, selbst episomaler, aber auch stabil in das Genom der transfizierten Zellen integrierter Plasmide nachzuweisen (Wolff *et al.* 1991): Dieses Nachweisverfahren beruht auf der Beobachtung, dass episomal in der Zelle vorliegende Plasmide, nicht jedoch stabil in das Genom einer Wirtszelle integrierte Plasmide nach Gesamt-DNS Extraktion und anschließender Elektroporation der DNS in Bakterien zur Ausbildung Antibiotika-resistenter Kolonien führen. Das Plasmid muß hierzu einen bakteriellen Replikationsursprung und ein selektierbares bakterielles Resistenzgen enthalten.

In Kontrollexperimenten konnte nach Elektroporation von 1 pg, 5 pg und 10 pg des für das kleine Hüllprotein (HBsAg) des Hepatitis B Virus kodierende Plasmid *pAM-HBsAg* (Deml et al., J. Virol. Methods, (1999), 79:191-203) eine Transformationseffizienz von etwa 10⁸ Kolonien pro µg transfizierter Plasmid-DNS ermittelt werden. Eine vergleichbare Elektroporationseffizienz wurde beobachtet, wenn die Plasmid-DNS zusammen mit 1 µg chromosomaler DNS aus Drosophila Schneider-2 (DS-2) Zellen verabreicht wurde. Somit wird die Elektroporationseffizienz von Plasmiden nicht durch die Anwesenheit von chromosomaler DNS beeinträchtigt. Durch Elektroporation der entsprechenden linearisierter Plasmid-DNS konnten dagegen nur sehr wenige Ampicillin-resistente Bakterienkolonien erzeugt werden.

Um zu überprüfen, ob das durch Lipofektion in DS-2 Insektenzellen transfizierte Plasmid pAM-HBsAg extrachromosomal oder integriert im Genom der Zellen vorliegt, wurde in drei unabhängigen Ansätzen Gesamt-DNS aus dieser Zellinie präpariert und in Bakterien transfiziert. Zur Kontrolle wurden Bakterien mit Gesamt-DNS aus nicht transfizierten DS-2 Zellen transformiert. In beiden Fällen konnte durch Transfektion von 1 µg Gesamt-DNS nur sehr wenige Ampicillin-resistente Klonien erzeugt werden. Durch PCR-Analyse unter Verwendung HBsAg-spezifischer Primer konnte gezeigt werden, dass keiner dieser resistenten Bakterienklone das HBsAg-Gen enthielt. Aus diesen Experimenten kann somit abgeleitet werden, dass die aus stabil transfizierten DS-2 Zellen präparierte Gesamt-DNS keine nachweisbaren Mengen episomal vorliegender *pAM-HBsAg* Plasmid-DNS enthielt.

In weiteren Elektroporationsstudien sollte gezeigt werden, dass Plasmid-DNS integriert im Genom der stabil transfizierten klonalen DS-2 Zellinie vorliegt. Dazu wurde aus der stabil transfizierten DS-2 Zellinie und nichttransfizierten DS-2 Zellen Gesamt-DNS gewonnen, mit ausgewählten Restriktionsenzymen geschnitten, religiert und in Bakterien transfiziert. Durch diese Vorgehensweise sollten integrierte Kopien des Expressionsplasmids *pAM-HBsAg* in extrachromosomale Plasmide überführt werden, die nach Elektroporation in Bakterien durch Vermittlung einer Ampicillinresistenz nachgewiesen werden können. Das zur Spaltung der Gesamt-DNS eingesetzte Restriktionsenzym EcoRV schneidet das Plasmid *pAM-HBsAg* singulär im Bereich zwischen dem Mtn-Promotor und dem Leserahmen für das HBsAg-Reporter-Gen. Durch Verdau der chromosomalen DNS der stabil mit *pAM-HBsAg* transfizierten DS-2 Zellen mit *Eco*RV*,* Religation und Elektroporation in *E. coli* konnten mehr als 200 Ampicillin-resistente Bakterienkolonicn gewonnen werden. Mehr als 80 % der etablierten Bakterienklone erwiesen sich in der PCR als HBsAg-positiv. Zur weiteren Charakterisierung dieser Klone wurde die Plasmid-DNS aus *E. coli* präpariert und mit *Eco*RV verdaut. Die meisten dieser Bakterienklone enthielten das "Input"-Plasmid *pAM-HBsAg* in der ursprünglichen Größe von etwa 8,2 kBp.

Diese Plasmide mit zu *pAM-HBsAg* unveränderter Größe resultieren wahrscheinlich aus Plasmiden, die in mehrfacher Kopienzahl an einem Integrationsort in das Genom der Wirtszelle integrierten. Etwa 15 % der analysierten HBsAg-positiven Bakterienklone beinhalteten Plasmide, die eine von dem Koexpressionsplasmid *pAM-HBsAg* abweichende Größe aufwiesen. Diese Plasmide wurden wahrscheinlich an Übergangsbereichen von chromosomaler DNS und der integrierten Plasmid-DNS aus dem Genom ausgeschnitten, wobei neben der Plasmid-kodierten eine weitere in Nachbarschaft zum integrierten Plasmid befindliche, genomische EcoRV Schnittstelle genutzt wurde. Nach Transfektion von Bakterien mit in gleicher Weise behandelter chromosomaler DNS aus nicht transfizierten DS-2 Zellen konnten 22 Ampicillin resistente Bakterienkolonien erzeugt werden. Diese erwiesen sich alle in der PCR-Analyse als HBsAg-negativ.

Vergleichbare Ergebnisse wurden nach Elektroporation von mit der Restriktionsendonuklease *Eco* RI verdauter Gesamt-DNS aus der stabil transfizierten DS-2 Zellinie und nicht transfizierten DS-2 Zellen erzielt. *Eco*RI spaltet das Plasmid *pAM-HBsAg* an zwei Stellen, wodurch zwei Fragmente von etwa 4,7 kBp und 3,5 kBp entstehen. Das größere Fragment beinhaltet neben der kodierenden Region für den Metallothionein (Mtn)-Promotor und das HBsAg-Reporter-Konstrukt auch den bakteriellen Replikationsursprung und das Ampicillin-Resistenzgen, und somit alle wichtigen Komponenten, um nach Religation und Transformation Amphicillinresistente Bakterien erzeugen zu können. Das kleinere 3,5 kBp Fragment beinhaltet dagegen keine der zur Ausbildung Ampicillin-resistenten Bakterienklone erforderlichen Sequenzen. Nach der Religation und Elektroporation von mit *Eco*RI verdauter DNS konnten 295 Bakterienkolonien auf Amphicillin-haltigen LB-Platten ausgezählt werden. Durch Analyse der aufgereinigten Plasmid-DNS mittels *Eco*RI Restriktionsverdau, konnte gezeigt werden, dass 43 von 46 getesteten HBsAg-positiven Klonen ein 4,7 kBp DNS-Fragment beinhalteten. 4 der 46 extrahierten Plasmide entsprachen nach einem *Eco*RI Verdau dem Bandenmuster des *EcoRI* geschnittenen *pAM-HBsAg* Plasmids. Nach der analytischen Restriktionsenzymspaltung mit *Eco*RI, konnte bei einigen Plasmid-Präparationen zusätzlich zum 4,7 kBp Fragment noch kleinere DNS-Fragmente detektiert werden. Diese Fragmente könnten von chromosomalen Sequenzen stammen, die während des *Eco*RI Verdaus aus chromosomaler DNS gespalten und mit dem 4,7 kBp Fragment zu einem größeren Plasmid religiert wurden. Etwa 5 % der analysierten HBsAg-positiven Bakterienklone enthalten ausschließlich 1 Plasmid, das die kodierende Sequenz des HBsAg beinhaltet, jedoch in der Größe von dem 4,7 kBp Fragment abweicht. Diese Plasmide entstammen wahrscheinlich wiederum dem Übergangsbereich zwischen chromosomaler DNS und der in mehrfacher Kopienzahl an einem Integrationsort in das Genom der Wirtszelle einrekombinierten Plasmid-DNS. Die nach Transfektion von chromosomaler DNS aus nicht transfizierten DS-2 Zellen erzeugten Ampicillin-resistenten Bakterienkolonien erwiesen sich in der PCR-Analyse mit spezifischen *Primern* als HBsAg-negativ. Diese Untersuchungen zeigen, daß sich das beschriebene Verfahren vorzüglich zur Isolierung und Charakterisierung von beliebigen, episomal oder in dem Genom von Zellen integriert vorliegenden Nukleinsäuren eignet und somit auch zur Isolierung und Charakterisierung von Nukleinsäuren aus markerpositiven Zellen verwendet werden kann.

## Patentansprüche

1. Gentransfervektor, umfassend einen spezifisch in antigenpräsentierenden Zellen durch den Epitop-spezifischen Kontakt mit einer T-Zelle induzierbaren ersten Promotor, eine mit diesem ersten Promotor funktionell verbundene Nukleinsäure kodierend ein Markergen, einen in antigenpräsentierenden Zellen konstitutiven zweiten Promotor, und eine mit diesem zweiten Promotor funktionell verbundene Nukleinsäure.

2. Gentransfervektor nach Anspruch 1, wobei der induzierbare erste Promotor ein Promotor für den OX40 Liganden (OX40L), den 4-1BB Liganden (4-1BBL), das kostimulatorische Protein B7.1 (CD80), das kostimulatorische Protein B7.2 (CD86) oder für den Fas Liganden (FasL) ist.

3. Gentransfervektor nach Anspruch 1 oder 2, wobei das Markergen ein fluoreszierendes Polypetid, Luziferase (LUC), alkalische Phosphatase (AP), sekretorische alkalische Phosphatase (SEAP), Chloramphenicol Acetyltransferase (CAT), Photinus-Luciferase, β-Glucuronidase (GUS), Renilla Luciferase, β-Galaktosidase (β-Gal), mikrobielle Struktur- oder Hüllproteine oder natürlicherweise in APC nicht vorkommende intrazelluläre und membranständige Polypeptide kodiert.

4. Gentransfervektor nach einem der Ansprüche 1 bis 3, wobei der konstitutive zweite Promotor der frühe SV40 Promotor, der Cytomegalovirus (CMV) Promotor, der *Respiratory Syncytial Virus* (RSV) Promotor, der Maus Mamma Tumorvirus (MMTV) LTR Promotor, der humane Immundefizienz Virus Typ 1 (HIV-1) LTR Promotor, der Adenovirus *major late-*Promotor (Ad MLP), der Herpes Simplex Virus (HSV) Promotor, der murine 3-Phosphoglycerat-Kinase (PGK) Promotor, der humane PGK-1 Promotor, der humane Ubiquitin C-Promotor, der humane EF-1α Promotor, der humane β-Caseinpromotor, der murine Metallothionein Promotor, der humane Actin 5c Promotor oder der humane ICI Promotor ist.

5. Gentransfervektor nach einem der Ansprüche 1 bis 4, wobei die mit dem zweiten Promotor funktionell verbundene Nukleinsäure aus einer cDNA-Bank oder einer genomischen Bank stammt.

6. Gentransfervektor nach einem der Ansprüche 1 bis 5, ferner umfassend einen bakteriellen Replikationsursprung (ori) und ein Resistenzgen.

7. Gentransfervektor nach einem der Ansprüche 1 bis 6, ferner umfassend Erkennungssequenzen für Restriktionsendonukleasen, die den konstitutiven zweiten Promotor und die mit diesem zweiten Promotor funktionell verbundene Nukleinsäure sowie den bakteriellen Replikationsursprung und die kodierende Sequenz für die bakterielle Resistenz flankieren.

8. Gentransfervektor nach einem der Ansprüche 1 bis 7, ferner umfassend einen dritten Promotor funktionell verbunden mit einem Markergen.

9. Verfahren zum Nachweis Epitop-spezifischer T-Zellen und zum Nachweis von Zielepitopen reaktiver T-Zellen, umfassend die folgenden Schritte:
a) in Kontakt bringen und Transduktion zuvor isolierter APC-haltiger und/oder T-zellhaltiger Körperflüssigkeit mit den Gentransfervektoren nach Anspruch 1 bis 8 oder mit einem Gentransfervektor, umfassend einen spezifisch in antigenpräsentierenden Zellen durch den Epitop-spezifischen Kontakt mit einer T-Zelle induzierbaren Promotor (P₁) und eine mit diesem Promotor funktionell verbundene Nukleinsäure kodierend ein Markergen, und einem Gentransfervektor, umfassend einen in antigenpräsentierenden Zellen konstitutiven Promotor (P₂) und eine mit diesem Promotor funktionell verbundene Nukleinsäure;
b) Inkubieren der Körperflüssigkeit enthaltend die transduzierten APC oder von isolierten transduzierten APC mit der Körperflüssigkeit enthaltend die T-Zellen oder die isolierten T-Zellen,
c) Nachweis von Marker exprimierenden APC, und
d) gegebenenfalls Isolierung und Charakterisierung der mit dem zweiten Promotor funktionell verbundenen für die Zielepitope reaktiver T-Zellen kodierenden Nukleinsäure.

10. Verfahren zum Nachweis Epitop-spezifischer T-Zellen und zum Nachweis von Zielepitopen reaktiver T-Zellen, umfassend die folgenden Schritte:
a) in Kontakt bringen und Transduktion zuvor isolierter APC-haltiger und/oder T-zellhaltiger Körperflüssigkeit mit einem in APC konstitutiven Promotor (P₂), und einer mit diesem Promotor funktionell verbundenen Nukleinsäure,
b) Inkubieren der Körperflüssigkeit enthaltend die transduzierten APC oder von isolierten transduzierten APC mit der Körperflüssigkeit enthaltend die T-Zellen oder die isolierten T-Zellen,
c) Nachweis von Marker exprimierenden APC, wobei die Marker in APC natürlich genomisch vorliegende Nukleinsäuren sind, die funktionell mit ihrem natürlichen Promotor, der in seiner Aktivität infolge einer Epitop-spezifischen Erkennung der APC durch eine T-Zelle messbar verändert wird, verbunden sind, und
d) gegebenenfalls Isolierung und Charakterisierung der mit dem konstitutiven Promotor (P₂) funktionell verbundenen für die Zielepitope reaktiver T-Zellen kodierenden Nukleinsäure.

11. Verfahren nach Anspruch 9 oder 10, wobei die APC-haltige Körperflüssigkeit in Schritt a) Blut, Liquor, eine gereinigte PBMC-Population oder eine separierte APC-Population ist.

12. Verfahren nach einem der Ansprüche 9 bis 11, wobei der Schritt des in Kontakt bringens 0,5 bis 36 Stunden oder länger, insbesondere 0,5 bis 2, 2 bis 6, 6 bis 12, 12 bis 36 oder 36 bis 168 Stunden dauert.

13. Verfahren nach einem der Ansprüche 9 bis 12, wobei die isolierten T-Zellen CD4+ T-Zellen, CD8⁺ T-Zellen, CD4⁺CD25⁺ regulatorische T-Zellen, CD161⁺ NKT Zellen oder ein beliebiges Gemisch aus CD4⁺ T-Zellen, CD8⁺ T-Zellen, CD4⁺CD25⁺ T-Zellen und CD161⁺ NKT-Zellen sind.

14. Verfahren nach einem der Ansprüche 9 bis 13, wobei der Marker in Schritt c) ein Polypeptid ist, das natürlicherweise nach Epitop-spezifischer Erkennung einer APC durch eine T-Zelle in seiner Expression meßbar gesteigert oder reduziert wird.

15. Antigenpräsentierende Zelle, transduziert mit Gentransfervektoren nach einem der Ansprüche 1 bis 8.

16. Antigenpräsentierende Zelle nach Anspruch 15, wobei die antigenpräsentierende Zelle eine dendritische Zelle, Monozyt, Makrophage, B-Zelle, vaskuläre endotheliale Zelle, epitheliale oder mesenchymale Zelle ist.

17. Verwendung der antigenpräsentierende Zelle nach Anspruch 15 oder 16 zum Nachweis Epitop-spezifischer T-Zellen und zum Nachweis von Zielepitopen reaktiver T-Zellen.

18. Kit zum diagnostischen Nachweis autoreaktiver T-Zellen, umfassend Gentransfervektoren nach einem der Ansprüche 1-8.

## Claims

1. A gene transfer vector, comprising a first promoter which is specifically inducible in antigen presenting cells by the epitope-specific contact with a T-cell, a nucleic acid encoding a marker gene functionally linked to said first promoter, a second promoter which is constitutive in antigen presenting cells and a nucleic acid which is functionally linked to said second promoter.

2. The gene transfer vector according to claim 1, wherein said inducible first promoter is a promoter for the OX40 ligand (OX40L), the 4-1 BB ligand (4-1 BBL), the co-stimulatory protein B7.1 (CD80), the co-stimulatory protein B7.2 (CD86) or for the Fas ligand (FasL).

3. The gene transfer vector according to claim 1 or 2, wherein said marker gene encodes a fluorescent polypeptide, luciferase (LUC), the alkaline phosphatase (AP), the secretory alkaline phosphatase (SEAP), the chloramphenicol acetyl transferase (CAT), the photinus-luciferase, the β-glucoronidase (GUS), the renilla luciferase, the β-galactosidase (β-Gal), microbial structure or coat proteins or intracellular and membrane associated polypeptides that do not occur naturally in APC.

4. The gene transfer vector according to any of the claims 1 to 3, wherein said constitutive second promoter is the early SV40 promoter, the cytomegalo virus (CMV) promoter, the respiratory syncytial virus (RSV) promoter, the mouse mammary tumour virus (MMTV) LTR promoter, the human immune, deficiency virus type 1 (HIV-1) LTR promoter, the adeno virus major late-promoter (Ad MLP), the herpes simplex virus (HSV) promoter, the murine 3-phosphoglycerate-kinase (PGK) promoter, the human PGK-1 promoter, the human ubiquitin C-promoter, the human EF-1α promoter, the human β-casein promoter, the murine metallothionein promoter, the human actin 5c promoter or the human ICI promoter.

5. The gene transfer vector according to any of the claims 1 to 4, wherein said nucleic acid being functionally linked to said second promoter is derived from a cDNA library or a genomic library.

6. The gene transfer vector according to any of the claims 1 to 5, further comprising a bacterial origin of replication (ori) and a resistance gene.

7. The gene transfer vector according to any of the claims 1 to 6, further comprising recognition sequences for restriction endonucleases flanking said constitutive second promoter and said nucleic acid being functionally linked to said second promoter and said bacterial origin of replication and said coding sequence for the bacterial resistance.

8. The gene transfer vector according to any of the claims 1 to 7, further comprising a third promoter functionally linked to a marker gene.

9. A method for the detection of epitope-specific T-cells and for the detection of target epitopes of reactive T-cells, comprising the following steps:
a) contacting and transducting of previously isolated APC-containing and/or T-cell-containing body fluid with the gene transfer vectors according to the claims 1 to 8 or with a gene transfer vector comprising a first promoter (P₁) which is specifically inducible in antigen presenting cells by epitope-specific contact with a T-cell and a nucleic acid encoding a marker gene functionally linked to said first promoter, and gene transfer vector comprising a second promoter which is constitutive in antigen presenting cells and a nucleic acid which is functionally linked to said second promoter,
b) incubating the body fluid containing the transduced APC or of isolated transduced APC with the body fluid containing the T-cells or the isolated T-cells,
c) detecting marker-expressing APC, and
d) optionally, isolating and characterising the nucleic acid which is functionally linked to said second promoter and which is encoding the target epitopes of reactive T-cells.

10. A method for the detection of epitope-specific T-cells and for the detection of target epitopes of reactive T-cells, comprising the following steps:
b) contacting and transducting of previously isolated APC-containing and/or T-cell-containing body fluid with a promoter (P₂) which is constitutive in APC, and a nucleic acid which is functionally linked to said promoter,
c) incubating the body fluid containing the transduced APC or of isolated transduced APC with the body fluid containing the T-cells or the isolated T-cells,
d) detecting marker-expressing APC, wherein the marker are genomic nucleic acids occurring in APCs and are functionally linked to their naturally promoter, the promoter being measurably altered in its activity due to an epitope-specific recognition of the APC by a T-cell, and
e) optionally, the isolating and characterising the nucleic acid which is functionally linked to said second promoter (P₂) and which is encoding the target epitopes of reactive T-cells.

11. The method according to claim 9 or 10, wherein said APC-containing body fluid in step a) is blood, liquor, a purified PBMC-population or a separated APC-population.

12. The method according to any of the claims 9 to 11, wherein said step of contacting takes 0.5 to 36 hours or longer, especially 0.5 to 2, 2 to 6, 6 to 12, 12 to 36 or 36 to 168 hours.

13. The method according to any of the claims 9 to 12, wherein said isolated T-cells are CD4⁺ T-cells, CD8⁺ T-cells, CD4⁺CD25⁺ regulatory T-cells, CD161⁺ NKT cells, or any mixture of CD4⁺ T-cells, CD8⁺ T-cells, CD4⁺CD25⁺ T-cells and CD161⁺ NKT-cells.

14. The method according to any of the claims 9 to 13, wherein said marker in step c) is a polypeptide which is naturally measurably induced or reduced in its expression after an epitope-specific recognition of an APC by a T-cell.

15. An antigen presenting cell transduced with gene transfer vectors according to any of the claims 1 to 8.

16. The antigen presenting cell according to claim 15, wherein said antigen presenting cell is a dendritic cell, a monocyte, a macrophage, a B-cell, a vascular endothelial cell, an epithelial or a mesenchymal cell.

17. The use of the antigen presenting cell according to claim 15 or 16 for the detection of epitope-specific T-cells and for the detection of target epitopes of reactive T-cells.

18. Kit for the diagnostic detection of autoreactive T-cells comprising gene transfer vectors according to any of the claims 1 to 8.

## Revendications

1. Vecteur de transfert de gènes comprenant un premier promoteur, pouvant sous l'effet du contact spécifique d'épitope avec une cellule T, subir une induction spécifique dans des cellules présentatrices d'antigènes, un acide nucléique lié de manière fonctionnelle à ce premier promoteur et qui code pour un gène marqueur, un deuxième promoteur constitutif dans les cellules présentatrices d'antigènes et un acide nucléique lié de manière fonctionnelle à ce deuxième promoteur.

2. Vecteur de transfert de gènes selon la revendication 1, dans lequel le premier promoteur inductible est un promoteur du ligand du OX40 (OX40L), du ligand du 4-1BB (4-1BBL), de la protéine de costimulation B7.1 (CD80), de la protéine de costimulation B7.2 (CD86) ou du ligand de Fas (FasL).

3. Vecteur de transfert de gènes selon la revendication 1 ou 2, dans lequel le gène marqueur code pour un polypeptide fluorescent, la luciférase (LUC), la phosphatase alcaline (PA), la phosphatase alcaline sécrétée (SEAP), la chloramphénicol acétyltransférase (CAT), la luciférase de Photinus, la β-glucuronidase (GUS), la luciférase de Renilla, la β-galactosidase (β-Gal), les protéines structurales ou les protéines d'enveloppe microbiennes ou des polypeptides intracellulaires et membranaires qui ne sont pas naturellement présents dans les CPA.

4. Vecteur de transfert de gènes selon l'une des revendications 1 à 3, dans lequel le deuxième promoteur constitutif est le promoteur précoce de SV40, le promoteur du cytomégalovirus (CMV), le promoteur du virus respiratoire syncytial (RSV), le promoteur LTR du virus de la tumeur mammaire de souris (MMTV), le promoteur LTR du virus de l'immunodéficience humaine de type 1 (VIH-1), le promoteur tardif majeur de l'adénovirus (Ad MLP), le promoteur de l'herpèsvirus simplex (HSV), le promoteur de la 3-phosphoglycérate kinase (PGK) murine, le promoteur de la PGK-1 humaine, le promoteur de l'ubiquitine C humaine, le promoteur de l'EF-1α humain, le promoteur de la β-caséine humaine, le promoteur de la métallothionéine murine, le promoteur de l'actine 5C humaine ou le promoteur de l'ICI humain.

5. Vecteur de transfert de gènes selon l'une des revendications 1 à 4, dans lequel l'acide nucléique lié de manière fonctionnelle au deuxième promoteur provient d'une banque d'ADNc ou d'une banque génomique.

6. Vecteur de transfert de gènes selon l'une des revendications 1 à 5, comprenant en outre une origine de réplication (ori) bactérienne et un gène de résistance.

7. Vecteur de transfert de gènes selon l'une des revendications 1 à 6, comprenant en outre des séquences de reconnaissance pour les endonucléases de restriction qui flanquent le deuxième promoteur constitutif et l'acide nucléique lié de manière fonctionnelle à ce deuxième promoteur ainsi que l'origine de réplication bactérienne et la séquence codant pour la résistance bactérienne.

8. Vecteur de transfert de gènes selon l'une des revendications 1 à 7, comprenant en outre un troisième promoteur fixé de manière fonctionnelle à un gène marqueur.

9. Procédé pour détecter les cellules T spécifiques d'épitope et pour détecter les cellules T réactives aux épitopes cibles, comprenant les étapes suivantes :
a) mise en contact et transduction d'un fluide corporel contenant des CPA et/ou de cellules T préalablement isolées avec les vecteurs de transfert de gènes selon la revendication 1 à 8 ou avec un vecteur de transfert de gènes comprenant un promoteur (P₁), pouvant, sous l'effet du contact spécifique d'épitope avec une cellule T, subir une induction spécifique dans des cellules présentatrices d'antigènes et un acide nucléique lié de manière fonctionnelle à ce promoteur qui code pour un gène marqueur et avec un vecteur de transfert de gènes comprenant un promoteur constitutif (P₂) dans des cellules présentatrices d'antigènes et un acide nucléique lié de manière fonctionnelle à ce promoteur ;
b) incubation du fluide corporel contenant les CPA transduites ou des CPA transduites isolées avec le fluide corporel contenant les cellules T ou les cellules T isolées ;
c) détermination des CPA exprimant des marqueurs, et
d) le cas échéant, isolement et caractérisation de l'acide nucléique codant pour les cellules T réactives aux épitopes cibles et lié de manière fonctionnelle au deuxième promoteur.

10. Procédé pour détecter les cellules T spécifiques d'épitope et pour détecter les cellules T réactives aux épitopes cibles comprenant les étapes suivantes :
a) mise en contact et transduction d'un fluide corporel contenant des CPA et/ou de cellules T préalablement isolées avec un promoteur constitutif (P₂) dans les CPA et un acide nucléique lié de manière fonctionnelle à ce promoteur,
b) incubation du fluide corporel contenant les CPA transduites ou des CPA transduites isolées avec le fluide corporel contenant les cellules T ou les cellules T isolées ;
c) détermination de CPA exprimant des marqueurs, les marqueurs dans les CPA étant des acides nucléiques naturellement présents dans le génome, qui sont liés de manière fonctionnelle à leur promoteur naturel dont on peut modifier de manière mesurable l'activité après une reconnaissance, spécifique d'épitope, des CPA par une cellule T, et
d) le cas échéant, isolement et caractérisation de l'acide nucléique codant pour les cellules T réactives aux épitopes cibles et lié de manière fonctionnelle au promoteur constitutif (P₂).

11. Procédé selon la revendication 9 ou 10, dans lequel le fluide corporel contenant les CPA à l'étape a) est du sang, une liqueur, une population de PBMC purifiée ou une population de CPA séparée.

12. Procédé selon l'une des revendications 9 à 11, dans lequel l'étape de la mise en contact dure d'une demi-heure à 36 heures ou plus, notamment d'une demi-heure à 2 heures, de 2 à 6 heures, de 6 à 12 heures, de 12 à 36 heures ou de 36 à 168 heures.

13. Procédé selon l'une des revendications 9 à 12, dans lequel les cellules T isolées sont des cellules T CD4⁺, des cellules T CD8⁺, des cellules T régulatrices CD4⁺CD25⁺, des cellules NKT CD161⁺ ou un mélange quelconque de cellules T CD4⁺, de cellules T CD8⁺, de cellules T CD4⁺CD25⁺ et de cellules NKT CD161⁺.

14. Procédé selon l'une des revendications 9 à 13, dans lequel le marqueur de l'étape c) est un polypeptide dont on augmente ou réduit l'expression de manière mesurable naturellement après la reconnaissance, spécifique d'épitope, d'une CPA par une cellule T.

15. Cellule présentatrices d'antigènes, transduite avec les vecteurs de transfert de gènes selon l'une des revendications 1 à 8.

16. Cellule présentatrices d'antigènes selon la revendication 15, dans laquelle la cellule présentatrices d'antigènes est une cellule dendritique, un monocyte, un macrophage, une cellule B, une cellule endothéliale vasculaire, une cellule épithéliale ou mésenchymale.

17. Utilisation de la cellule présentatrices d'antigènes selon la revendication 15 ou 16 pour détecter les cellules spécifiques d'épitope et pour détecter les cellules T réactives aux épitopes cibles.

18. Trousse pour la détection et le diagnostic des cellules T autoréactives comprenant les vecteurs de transfert de gènes selon l'une des revendications 1 à 8.


### SEQUENCE LISTING

<110> Deml, Ludwig
<120> Verfahren zur Identifizierung von Zielepitopen der T-Zell-vermittelten Immunantwort und zum Nachweis Epitop-spezifischer T-Zellen
<130> DEM-002 PCT
<140> unknown
   <141> 2002-11-20
<150> 101 56 863.0
   <151> 2001-11-20
<160> 58
   <170> PatentIn version 3.1
<210> 1
   <211> 1000
   <212> DNA
   <213> Homo sapiens
<400> 1
   ggcgggggat ccggtacctg gtgtctattg atagaatacc cttactttcc aagttctgag      60
   gccagccaca acctcaaaga aagaaataat cttcaaagct tatttacatc tattcatttg     120
   ttcattcatg gaaaaattat gtacgggaaa aatgtgaagt ggaaggaaag gagacaagga     180
   tacccttctg cttcttttct ggtatgcaga aaattaatca tataattgtt tattgaattt     240
   gcctcaaagg gttaaatgag agaaagattg caagtccatc taacacagtg tctgacacat     300
   agtaaatgct tccaaaaggt agtttatttt tatgcaatag actacaacca aggatctctc     360
   ctttaccttc tgcatgaccc agtgtcctat tttcttgttt tgtctttttc attcaagaat     420
   gatctgtgtg tgctttcttt ctgaatctgt gagaggggtt caatgtttag ttacatagta     480
   aagtgcagat aagaaccttg ggccagcatt tcccctaaat cttttctatg atttaagaca     540
   ccttggggaa gggatatatg agctgctaca ttaacggatg atgattttta tctttaccaa     600
   gtgacccaca atttgttact ggtgcaataa gttaagaaaa tagcatactt ttatgactgt 660
   catttcacag tcactcaaat cagaactgga atgtcacaaa aacaactccc tgttagccca 720
   gaggaaaaaa aaaaagaaag aaagtaaagg ggaaattcag attagtcaca aagaagttcc 780
   cccgcctgcc tgcaaaagtt gcagcgttaa aactgagaga gtccgctttg ctctttcaat 840
   cgccttttat ctctggccct gggacctttg cctattttct gattgatagg ctttgttttg 900
   tcttacctcc ttctttctgg gaaaactcag ttttatcgca cgttcccctt ttccatatct 960
   tcatcttccc tctacccaga ttgtgaagag atctcccgcc 1000
<210> 2
   <211> 2023
   <212> DNA
   <213> Homo sapiens
<400> 2
   ccggattggc cgcctccagc aggccagagc agatgggaaa tgtgtttcct ccaactcgct 60
   caacactgtc cctttcttgc aaggggaatt tctccacctg gactctccga gcttctgctt 120
   gtagggtccc gaggggctcc tgccgggaac catccagtgg gtgggtgggg gcaggccagg 180
   gaaggcccag gggtcccggg gaaggtggcc aggagggctg agcatgacac tcagggttcc 240
   actggggggt tggggatcag ggggcagatg ctccaggagt gagggtggtt gaggcttggc 300
   tgaagcctga cccttcttag gaagtgctgg atgtggggga acccggggga gtcctagtga 360
   tggggggcta gggggtgctg agaggagggg aaggagacaa agagagacta aagaaggaaa 420
   aggagagaga cagggagaga cagagagtca gagagagaca gagggaatca gagacagaga 480
   gaaacaggga gagagacaga aagagtgaga gccagagaca tacagagaca gggagagaca 540
   cagagatgca taaagagagg gagtcagaga tggagagaga cagagataac ggagccagag 600
   atagggagag tcagagacag agagaaagaa acacacagac tgacacagat aaaagagcca 660
   gagacaggga gaggcagaga caaagagaga tacagagaca gagataacag agccagagac 720
   agagagacag agacagagat aacagagcca gagacaggga gagagtgtca gagacagaga 780
   cagacacaga gataacagag ccagagatag ggagagtcag agacagagag aaagagacac 840
   aagacagaca tagataaaag agccagagac agggagaggc agagacaaag agagatacag 900
   agacagagat agcagagcca gagacaggga gagagtgtca gagacagaga gacagacaca 960
   gagataacag agccagagat agggagagtc agagacagag agaaagagac agacagacat 1020
   agataaaaga gccagagaca gggagagaga cacagagaca gagacataga gacagagata 1080
   acagagccag agacagagtc agagacagag acagagagag ccagacacag agacagagag 1140
   agagagtcac agccagagac agcgacagag ataacagaag cagagagagg gagaaagata 1200
   acagagccag agacagtcag agacagagat agagagacag agataacaga gccagagaca 1260
   gatacagaga cagagataac ggagccagag atagggagag agagtcagag acagagagag 1320
   atatatagac agagataaca gagccagaca tggggagaga gagtcagagg cagagagaga 1380
   gaagacagat agagagacag agccagtgat aacagagcca gagacaggga gagagagtca 1440
   gagagagata caatgagaga cagagataac agccagagac agagttagac agagagatac 1500
   agagacagag ataacagagc cagagacagg gagagagaat tagagacaga gatagagaga 1560
   gacagagaca gagataatac aggcacagag gcacgcatag acataaattg gcagagagag 1620
   agaaagatct ctttccaccc actgcagagg caatcaacag agacagagaa agacgttaac 1680
   ggggagacac agagagcaaa ggagactgag tcagcaagag acccacagag ataccgggaa 1740
   agagcggcag agagggagaa ccagggcgat ggagagacag cagggagaaa ggaacctgga 1800
   gccgagcttg gaaggccgga aacggaaagg agagcgaaaa gcggagagag atccgagtgg 1860
   agaaaattcc gcagagtcac ggggacgagg ggaaaggctc tgggctggga aggggcgtgg 1920
   ccgcgggcgg aggggcgtgg ccgcgggcgg aggggcgtgg cctccttttg tagccaagca 1980
   gctataaaaa gcggcgcgct gtgtcttccc gcagtctctc gtc 2023
<210> 3
   <211> 1433
   <212> DNA
   <213> Artificial
<400> 3
   ggagccagag atagggagag tcagagacag agagaaagaa acacacagac tgacacagat 60
   aaaagagcca gagacaggga gaggcagaga caaagagaga tacagagaca gagataacag 120
   agccagagac agagagacag agacagagat aacagagcca gagacaggga gagagtgtca 180
   gagacagaga cagacacaga gataacagag ccagagatag ggagagtcag agacagagag 240
   aaagagacac aagacagaca tagataaaag agccagagac agggagaggc agagacaaag 300
   agagatacag agacagagat agcagagcca gagacaggga gagagtgtca gagacagaga 360
   gacagacaca gagataacag agccagagat agggagagtc agagacagag agaaagagac 420
   agacagacat agataaaaga gccagagaca gggagagaga cacagagaca gagacataga     480
   gacagagata acagagccag agacagagtc agagacagag acagagagag ccagacacag     540
   agacagagag agagagtcac agccagagac agcgacagag ataacagaag cagagagagg     600
   gagaaagata acagagccag agacagtcag agacagagat agagagacag agataacaga     660
   gccagagaca gatacagaga cagagataac ggagccagag atagggagag agagtcagag     720
   acagagagag atatatagac agagataaca gagccagaca tggggagaga gagtcagagg     780
   cagagagaga gaagacagat agagagacag agccagtgat aacagagcca gagacaggga     840
   gagagagtca gagagagata caatgagaga cagagataac agccagagac agagttagac     900
   agagagatac agagacagag ataacagagc cagagacagg gagagagaat tagagacaga     960
   gatagagaga gacagagaca gagataatac aggcacagag gcacgcatag acataaattg    1020
   gcagagagag agaaagatct ctttccaccc actgcagagg caatcaacag agacagagaa    1080
   agacgttaac ggggagacac agagagcaaa ggagactgag tcagcaagag acccacagag    1140
   ataccgggaa agagcggcag agagggagaa ccagggcgat ggagagacag cagggagaaa    1200
   ggaacctgga gccgagcttg gaaggccgga aacggaaagg agagcgaaaa gcggagagag    1260
   atccgagtgg agaaaattcc gcagagtcac ggggacgagg ggaaaggctc tgggctggga    1320
   aggggcgtgg ccgcgggcgg aggggcgtgg ccgcgggcgg aggggcgtgg cctccttttg    1380
   tagccaagca gctataaaaa gcggcgcgct gtgtcttccc gcagtctctc gtc           1433
<210> 4
   <211> 874
   <212> DNA
   <213> Artificial
<400> 4
   cagccagaga cagcgacaga gataacagaa gcagagagag ggagaaagat aacagagcca      60
   gagacagtca gagacagaga tagagagaca gagataacag agccagagac agatacagag     120
   acagagataa cggagccaga gatagggaga gagagtcaga gacagagaga gatatataga     180
   cagagataac agagccagac atggggagag agagtcagag gcagagagag agaagacaga     240
   tagagagaca gagccagtga taacagagcc agagacaggg agagagagtc agagagagat     300
   acaatgagag acagagataa cagccagaga cagagttaga cagagagata cagagacaga     360
   gataacagag ccagagacag ggagagagaa ttagagacag agatagagag agacagagac     420
   agagataata caggcacaga ggcacgcata gacataaatt ggcagagaga gagaaagatc 480
   tctttccacc cactgcagag gcaatcaaca gagacagaga aagacgttaa cggggagaca 540
   cagagagcaa aggagactga gtcagcaaga gacccacaga gataccggga aagagcggca 600
   gagagggaga accagggcga tggagagaca gcagggagaa aggaacctgg agccgagctt 660
   ggaaggccgg aaacggaaag gagagcgaaa agcggagaga gatccgagtg gagaaaattc 720
   cgcagagtca cggggacgag gggaaaggct ctgggctggg aaggggcgtg gccgcgggcg 780
   gaggggcgtg gccgcgggcg gaggggcgtg gcctcctttt gtagccaagc agctataaaa 840
   agcggcgcgc tgtgtcttcc cgcagtctct cgtc 874
<210> 5
   <211> 386
   <212> DNA
   <213> Artificial
<400> 5
<21.0> 6
   <211> 24
   <212> DNA
   <213> Artificial
<400> 6
   gctggttctt tccgcctcag aagc 24
<210> 7
   <211> 23
   <212> DNA
   <213> Artificial
<400> 7
   cactgcattc tagttgtggt ttg 23
<210> 8
   <211> 32
   <212> DNA
   <213> Artificial
<400> 8
   gcaggctcgc gactaccggg taggggaggc gc 32
<210> 9
   <211> 35
   <212> DNA
   <213> Artificial
<400> 9
   gcaggcggat ccacgcgctt ctacaaggcg cttgc 35
<210> 10
   <211> 30
   <212> DNA
   <213> Artificial
<400> 10
   gcaggctcgc gacggggttg gggttgcgcc 30
<210> 11
   <211> 33
   <212> DNA
   <213> Artificial
<400> 11
   gcaggcggat cctttggaaa tacagctggg gag 33
<210> 12
   <211> 32
   <212> DNA
   <213> Artificial
<400> 12
   gcaggctcgc gacgatggcc ctgcccagtc cc 32
<210> 13
   <211> 32
   <212> DNA
   <213> Artificial
<400> 13
   gcaggcggat ccgacagcag cgggaggacc tc 32
<210> 14
   <211> 32
   <212> DNA
   <213> Artificial
<400> 14
   gcaggctcgc gaggctccgg tgcccgtcag tg 32
<210> 15
   <211> 33
   <212> DNA
   <213> Artificial
<400> 15
   gcaggcggat ccacctagcc agcttgggtc tcc 33
<210> 16
   <211> 33
   <212> DNA
   <213> Artificial
<400> 16
   gcaggctcgc gaggcctccg cgccgggttt tgg 33
<210> 17
   <211> 35
   <212> DNA
   <213> Artificial
<400> 17
   gcaggcggat ccgtctaaca aaaaagccaa aaacg 35
<210> 18
   <211> 34
   <212> DNA
   <213> Artificial
<400> 18
   gcaggctcgc gagctgtaat ttctaatcta aacc 34
<210> 19
   <211> 33
   <212> DNA
   <213> Artificial
<400> 19
   gcaggcggat ccagcagcag agtgcggcaa cac 33
<210> 20
   <211> 30
   <212> DNA
   <213> Artificial
<400> 20
   ggcgggggat ccctgtgcct tctagttgcc 30
<210> 21
   <211> 28
   <212> DNA
   <213> Artificial
<400> 21
   ggcgggagat ctccatagag cccaccgc 28
<210> 22
   <211> 30
   <212> DNA
   <213> Artificial
<400> 22
   ggcggagatc tttagaaatt taagagatcc 30
<210> 23
   <211> 30
   <212> DNA
   <213> Artificial
<400> 23
   ggcgggagat ctatgatgga cccaaactcg 30
<210> 24
   <211> 31
   <212> DNA
   <213> Artificial
<400> 24
   ggcggagatc tatggcgtca cagaagagac c 31
<210> 25
   <211> 30
   <212> DNA
   <213> Artificial
<400> 25
   ggcgggagat cttcagcgtc tagtcatggg 30
<210> 26
   <211> 32
   <212> DNA
   <213> Artificial
<400> 26
   ggcggtctag agccgccacc atgcccatcg tg 32
<210> 27
   <211> 33
   <212> DNA
   <213> Artificial
<400> 27
   ggcggggaat tctcacagca gcctggcctt gtg 33
<210> 28
   <211> 32
   <212> DNA
   <213> Artificial
<400> 28
   ggcgggagat ctcgccacca tggtgagcaa gg 32
<210> 29
   <211> 31
   <212> DNA
   <213> Artificial
<400> 29
   ggcgggagat ctttacttgt acagctcgtc c 31
<210> 30
   <211> 24
   <212> DNA
   <213> Artificial
<400> 30
   gcaggagatc ttatggcgcc ccgc 24
<210> 31
   <211> 26
   <212> DNA
   <213> Artificial
<400> 31
   gcaggcggat cctcaaagag tgctga 26
<210> 32
   <211> 30
   <212> DNA
   <213> Artificial
<400> 32
   ggcggggatc cttagaaatt taagagatcc 30
<210> 33
   <211> 30
   <212> DNA
   <213> Artificial
<400> 33
   ggcgggagat ctatgatgga cccaaactcg 30
<210> 34
   <211> 31
   <212> DNA
   <213> Artificial
<400> 34
   ggcggagatc tatggcgtca cagaagagac c 31
<210> 35
   <211> 30
   <212> DNA
   <213> Artificial
<400> 35
   ggcgggggat cctcagcgtc tagccatggg 30
<210> 36
   <211> 26
   <212> DNA
   <213> Artificial
<400> 36
   gcaggagatc taacagcacg ctgatc 26
<210> 37
   <211> 27
   <212> DNA
   <213> Artificial
<400> 37
   gcaggcagat ctctagatag tctggta 27
<210> 38
   <211> 53
   <212> DNA
   <213> Artificial
<400> 38
   gcaggagatc tagcggggca ggatccatgc tagtacttca atgggcctct ctg 53
<210> 39
   <211> 33
   <212> DNA
   <213> Artificial
<400> 39
   gcaggcagat ctttatacat acctctcggc ctc 33
<210> 40
   <211> 30
   <212> DNA
   <213> Artificial
<400> 40
   ggcgggggat ccggtacctg gtgtctattg 30
<210> 41
   <211> 29
   <212> DNA
   <213> Artificial
<400> 41
   ggcgggagat ctcttcacaa tctgggtag 29
<210> 42
   <211> 29
   <212> DNA
   <213> Artificial
<400> 42
   ggcgggagat cctgtgcctt ctagttgcc 29
<210> 43
   <211> 28
   <212> DNA
   <213> Artificial
<400> 43
   ggcgggggat ccccatagag cccaccgc 28
<210> 44
   <211> 32
   <212> DNA
   <213> Artificial
<400> 44
   ggcgggggat cccgccacca tggtgagcaa gg 32
<210> 45
   <211> 31
   <212> DNA
   <213> Artificial
<400> 45
   ggcgggagat ctttacttgt acagctcgtc c 31
<210> 46
   <211> 30
   <212> DNA
   <213> Artificial
<400> 46
   ggcggtctag attagaaatt taagagatcc 30
<210> 47
   <211> 30
   <212> DNA
   <213> Artificial
<400> 47
   ggcggggaat tcatgatgga cccaaactcg 30
<210> 48
   <211> 31
   <212> DNA
   <213> Artificial
<400> 48
   ggcggtctag aatggcgtca cagaagagac c 31
<210> 49
   <211> 30
   <212> DNA
   <213> Artificial
<400> 49
   ggcggggaat tctcagcgtc tagccatggg 30
<210> 50
   <211> 30
   <212> DNA
   <213> Artificial
<400> 50
   ggcgggggat ccggtacctg gtgtctattg 30
<210> 51
   <211> 29
   <212> DNA
   <213> Artificial
<400> 51
   ggcgggagat ctcttcacaa tctgggtag 29
<210> 52
   <211> 34
   <212> DNA
   <213> Artificial
<400> 52
   ggcgggggat ccccggattg gccgcctcca gcag 34
<210> 53
   <211> 35
   <212> DNA
   <213> Artificial
<400> 53
   ggcgggggat ccgacgagag actgcgggaa gacac 35
<210> 54
   <211> 34
   <212> DNA
   <213> Artificial
<400> 54
   ggcgggggat ccccggattg gccgcctcca gcag 34
<210> 55
   <211> 35
   <212> DNA
   <213> Artificial
<400> 55
   ggcgggagat ctgacgagag actgcgggaa gacac 35
<210> 56
   <211> 34
   <212> DNA
   <213> Artificial
<400> 56
   ggcgggggat ccggagccag agatagggag agtc 34
<210> 57
   <211> 33
   <212> DNA
   <213> Artificial
<400> 57
   ggcgggggat cccagccaga gacagcgaca gag 33
<210> 58
   <211> 36
   <212> DNA
   <213> Artificial
<400> 58
   ggcgggggat cccccactgc agaggcaatc aacaag 36

### SEQUENCE LISTING

<110> Deml, Ludwig
<120> Verfahren zur Identifizierung von Zielepitopen der T-Zell-vermittelten Immunantwort und zum Nachweis Epitop-spezifischer T-Zellen
<130> DEM-002 PCT
<140> PCT/DE 02/04270
   <141> 2002-11-20
<150> 101 56 863.0
   <151> 2001-11-20
<160> 58
   <170> PatentIn version 3.1
<210> 1
   <211> 1000
   <212> DNA
   <213> Homo sapiens
<400> 1
   ggcgggggat ccggtacctg gtgtctattg atagaatacc cttactttcc aagttctgag     60
   gccagccaca acctcaaaga aagaaataat cttcaaagct tatttacatc tattcatttg    120
   ttcattcatg gaaaaattat gtacgggaaa aatgtgaagt ggaaggaaag gagacaagga    180
   tacccttctg cttcttttct ggtatgcaga aaattaatca tataattgtt tattgaattt    240
   gcctcaaagg gttaaatgag agaaagattg caagtccatc taacacagtg tctgacacat    300
   agtaaatgct tccaaaaggt agtttatttt tatgcaatag actacaacca aggatctctc    360
   ctttaccttc tgcatgaccc agtgtcctat tttcttgttt tgtctttttc attcaagaat    420
   gatctgtgtg tgctttcttt ctgaatctgt gagaggggtt caatgtttag ttacatagta    480
   aagtgcagat aagaaccttg ggccagcatt tcccctaaat cttttctatg atttaagaca    540
   ccttggggaa gggatatatg agctgctaca ttaacggatg atgattttta tctttaccaa    600
   gtgacccaca atttgttact ggtgcaataa gttaagaaaa tagcatactt ttatgactgt    660
   catttcacag tcactcaaat cagaactgga atgtcacaaa aacaactccc tgttagccca    720
   gaggaaaaaa aaaaagaaag aaagtaaagg ggaaattcag attagtcaca aagaagttcc    780
   cccgcctgcc tgcaaaagtt gcagcgttaa aactgagaga gtccgctttg ctctttcaat    840
   cgccttttat ctctggccct gggacctttg cctattttct gattgatagg ctttgttttg    900
   tcttacctcc ttctttctgg gaaaactcag ttttatcgca cgttcccctt ttccatatct    960
   tcatcttccc tctacccaga ttgtgaagag atctcccgcc                         1000
<210> 2
   <211> 2023
   <212> DNA
   <213> Homo sapiens
<400> 2
   ccggattggc cgcctccagc aggccagagc agatgggaaa tgtgtttcct ccaactcgct      60
   caacactgtc cctttcttgc aaggggaatt tctccacctg gactctccga gcttctgctt     120
   gtagggtccc gaggggctcc tgccgggaac catccagtgg gtgggtgggg gcaggccagg     180
   gaaggcccag gggtcccggg gaaggtggcc aggagggctg agcatgacac tcagggttcc     240
   actggggggt tggggatcag ggggcagatg ctccaggagt gagggtggtt gaggcttggc      300
   tgaagcctga cccttcttag gaagtgctgg atgtggggga acccggggga gtcctagtga      360
   tggggggcta gggggtgctg agaggagggg aaggagacaa agagagacta aagaaggaaa      420
   aggagagaga cagggagaga cagagagtca gagagagaca gagggaatca gagacagaga     480
   gaaacaggga gagagacaga aagagtgaga gccagagaca tacagagaca gggagagaca     540
   cagagatgca taaagagagg gagtcagaga tggagagaga cagagataac ggagccagag     600
   atagggagag tcagagacag agagaaagaa acacacagac tgacacagat aaaagagcca     660
   gagacaggga gaggcagaga caaagagaga tacagagaca gagataacag agccagagac     720
   agagagacag agacagagat aacagagcca gagacaggga gagagtgtca gagacagaga     780
   cagacacaga gataacagag ccagagatag ggagagtcag agacagagag aaagagacac     840
   aagacagaca tagataaaag agccagagac agggagaggc agagacaaag agagatacag     900
   agacagagat agcagagcca gagacaggga gagagtgtca gagacagaga gacagacaca     960
   gagataacag agccagagat agggagagtc agagacagag agaaagagac agacagacat    1020
   agataaaaga gccagagaca gggagagaga cacagagaca gagacataga gacagagata    1080
   acagagccag agacagagtc agagacagag acagagagag ccagacacag agacagagag    1140
   agagagtcac agccagagac agcgacagag ataacagaag cagagagagg gagaaagata    1200
   acagagccag agacagtcag agacagagat agagagacag agataacaga gccagagaca    1260
   gatacagaga cagagataac ggagccagag atagggagag agagtcagag acagagagag    1320
   atatatagac agagataaca gagccagaca tggggagaga gagtcagagg cagagagaga    1380
   gaagacagat agagagacag agccagtgat aacagagcca gagacaggga gagagagtca    1440
   gagagagata caatgagaga cagagataac agccagagac agagttagac agagagatac    1500
   agagacagag ataacagagc cagagacagg gagagagaat tagagacaga gatagagaga    1560
   gacagagaca gagataatac aggcacagag gcacgcatag acataaattg gcagagagag    1620
   agaaagatct ctttccaccc actgcagagg caatcaacag agacagagaa agacgttaac    1680
   ggggagacac agagagcaaa ggagactgag tcagcaagag acccacagag ataccgggaa    1740
   agagcggcag agagggagaa ccagggcgat ggagagacag cagggagaaa ggaacctgga    1800
   gccgagcttg gaaggccgga aacggaaagg agagcgaaaa gcggagagag atccgagtgg    1860
   agaaaattcc gcagagtcac ggggacgagg ggaaaggctc tgggctggga aggggcgtgg    1920
   ccgcgggcgg aggggcgtgg ccgcgggcgg aggggcgtgg cctccttttg tagccaagca    1980
   gctataaaaa gcggcgcgct gtgtcttccc gcagtctctc gtc                      2023
<210> 3
   <211> 1433
   <212> DNA
   <213> Artificial
<220>
   <223> ... im 5' Bereich verkürzter humaner 4-1 BB Ligand Promotor
<400> 3
   ggagccagag atagggagag tcagagacag agagaaagaa acacacagac tgacacagat      60
   aaaagagcca gagacaggga gaggcagaga caaagagaga tacagagaca gagataacag     120
   agccagagac agagagacag agacagagat aacagagcca gagacaggga gagagtgtca     180
   gagacagaga cagacacaga gataacagag ccagagatag ggagagtcag agacagagag     240
   aaagagacac aagacagaca tagataaaag agccagagac agggagaggc agagacaaag     300
   agagatacag agacagagat agcagagcca gagacaggga gagagtgtca gagacagaga     360
   gacagacaca gagataacag agccagagat agggagagtc agagacagag agaaagagac     420
   agacagacat agataaaaga gccagagaca gggagagaga cacagagaca gagacataga     480
   gacagagata acagagccag agacagagtc agagacagag acagagagag ccagacacag     540
   agacagagag agagagtcac agccagagac agcgacagag ataacagaag cagagagagg     600
   gagaaagata acagagccag agacagtcag agacagagat agagagacag agataacaga     660
   gccagagaca gatacagaga cagagataac ggagccagag atagggagag agagtcagag     720
   acagagagag atatatagac agagataaca gagccagaca tggggagaga gagtcagagg     780
   cagagagaga gaagacagat agagagacag agccagtgat aacagagcca gagacaggga     840
   gagagagtca gagagagata caatgagaga cagagataac agccagagac agagttagac     900
   agagagatac agagacagag ataacagagc cagagacagg gagagagaat tagagacaga     960
   gatagagaga gacagagaca gagataatac aggcacagag gcacgcatag acataaattg    1020
   gcagagagag agaaagatct ctttccaccc actgcagagg caatcaacag agacagagaa    1080
   agacgttaac ggggagacac agagagcaaa ggagactgag tcagcaagag acccacagag    1140
   ataccgggaa agagcggcag agagggagaa ccagggcgat ggagagacag cagggagaaa    1200
   ggaacctgga gccgagcttg gaaggccgga aacggaaagg agagcgaaaa gcggagagag    1260
   atccgagtgg agaaaattcc gcagagtcac ggggacgagg ggaaaggctc tgggctggga    1320
   aggggcgtgg ccgcgggcgg aggggcgtgg ccgcgggcgg aggggcgtgg cctccttttg    1380
   tagccaagca gctataaaaa gcggcgcgct gtgtcttccc gcagtctctc gtc           1433
<210> 4
   <211> 874
   <212> DNA
   <213> Artificial
<220>
   <223> ... im 5' Bereich verkürzter humaner 4-1 BB Ligand Promotor
<400> 4
   cagccagaga cagcgacaga gataacagaa gcagagagag ggagaaagat aacagagcca 60
   gagacagtca gagacagaga tagagagaca gagataacag agccagagac agatacagag 120
   acagagataa cggagccaga gatagggaga gagagtcaga gacagagaga gatatataga 180
   cagagataac agagccagac atggggagag agagtcagag gcagagagag agaagacaga 240
   tagagagaca gagccagtga taacagagcc agagacaggg agagagagtc agagagagat 300
   acaatgagag acagagataa cagccagaga cagagttaga cagagagata cagagacaga 360
   gataacagag ccagagacag ggagagagaa ttagagacag agatagagag agacagagac 420
   agagataata caggcacaga ggcacgcata gacataaatt ggcagagaga gagaaagatc 480
   tctttccacc cactgcagag gcaatcaaca gagacagaga aagacgttaa cggggagaca 540
   cagagagcaa aggagactga gtcagcaaga gacccacaga gataccggga aagagcggca 600
   gagagggaga accagggcga tggagagaca gcagggagaa aggaacctgg agccgagctt 660
   ggaaggccgg aaacggaaag gagagcgaaa agcggagaga gatccgagtg gagaaaattc 720
   cgcagagtca cggggacgag gggaaaggct ctgggctggg aaggggcgtg gccgcgggcg 780
   gaggggcgtg gccgcgggcg gaggggcgtg gcctcctttt gtagccaagc agctataaaa 840
   agcggcgcgc tgtgtcttcc cgcagtctct cgtc 874
<210> 5
   <211> 386
   <212> DNA
   <213> Artificial
<220>
   <223> ... im 5' Bereich verkürzter humaner 4-1 BB Ligand Promotor
<400> 5
   cccactgcag aggcaatcaa cagagacaga gaaagacgtt aacggggaga cacagagagc 60
   aaaggagact gagtcagcaa gagacccaca gagataccgg gaaagagcgg cagagaggga 120
   gaaccagggc gatggagaga cagcagggag aaaggaacct ggagccgagc ttggaaggcc 180
   ggaaacggaa aggagagcga aaagcggaga gagatccgag tggagaaaat tccgcagagt 240
   cacggggacg aggggaaagg ctctgggctg ggaaggggcg tggccgcggg cggaggggcg 300
   tggccgcggg cggaggggcg tggcctcctt ttgtagccaa gcagctataa aaagcggcgc 360
   gctgtgtctt cccgcagtct ctcgtc 386
<210> 6
   <211> 24
   <212> DNA
   <213> Artificial
<220>
   <223> Primer
<400> 6
   gctggttctt tccgcctcag aagc 24
<210> 7
   <211> 23
   <212> DNA
   <213> Artificial
<220>
   <223> Primer
<400> 7
   cactgcattc tagttgtggt ttg 23
<210> 8
   <211> 32
   <212> DNA
   <213> Artificial
<220>
   <223> Primer
<400> 8
   gcaggctcgc gactaccggg taggggaggc gc 32
<210> 9
   <211> 35
   <212> DNA
   <213> Artificial
<220>
   <223> Primer
<400> 9
   gcaggcggat ccacgcgctt ctacaaggcg cttgc 35
<210> 10
   <211> 30
   <212> DNA
   <213> Artificial
<220>
   <223> Primer
<400> 10
   gcaggctcgc gacggggttg gggttgcgcc 30
<210> 11
   <211> 33
   <212> DNA
   <213> Artificial
<220>
   <223> Primer
<400> 11
   gcaggcggat cctttggaaa tacagctggg gag 33
<210> 12
   <211> 32
   <212> DNA
   <213> Artificial
<220>
   <223> Primer
<400> 12
   gcaggctcgc gacgatggcc ctgcccagtc cc 32
<210> 13
   <211> 32
   <212> DNA
   <213> Artificial
<220>
   <223> Primer
<400> 13
   gcaggcggat ccgacagcag cgggaggacc tc 32
<210> 14
   <211> 32
   <212> DNA
   <213> Artificial
<220>
   <223> Primer
<400> 14
   gcaggctcgc gaggctccgg tgcccgtcag tg 32
<210> 15
   <211> 33
   <212> DNA
   <213> Artificial
<220>
   <223> Primer
<400> 15
   gcaggcggat ccacctagcc agcttgggtc tcc 33
<210> 16
   <211> 33
   <212> DNA
   <213> Artificial
<220>
   <223> Primer
<400> 16
   gcaggctcgc gaggcctccg cgccgggttt tgg 33
<210> 17
   <211> 35
   <212> DNA
   <213> Artificial
<220>
   <223> Primer
<400> 17
   gcaggcggat ccgtctaaca aaaaagccaa aaacg 35
<210> 18
   <211> 34
   <212> DNA
   <213> Artificial
<220>
   <223> Primer
<400> 18
   gcaggctcgc gagctgtaat ttctaatcta aacc 34
<210> 19
   <211> 33
   <212> DNA
   <213> Artificial
<220>
   <223> Primer
<400> 19
   gcaggcggat ccagcagcag agtgcggcaa cac 33
<210> 20
   <211> 30
   <212> DNA
   <213> Artificial
<220>
   <223> Primer
<400> 20
   ggcgggggat ccctgtgcct tctagttgcc 30
<210> 21
   <211> 28
   <212> DNA
   <213> Artificial
<220>
   <223> Primer
<400> 21
   ggcgggagat ctccatagag cccaccgc 28
<210> 22
   <211> 30
   <212> DNA
   <213> Artificial
<220>
   <223> Primer
<400> 22
   ggcggagatc tttagaaatt taagagatcc 30
<210> 23
   <211> 30
   <212> DNA
   <213> Artificial
<220>
   <223> Primer
<400> 23
   ggcgggagat ctatgatgga cccaaactcg 30
<210> 24
   <211> 31
   <212> DNA
   <213> Artificial
<220>
   <223> Primer
<400> 24
   ggcggagatc tatggcgtca cagaagagac c 31
<210> 25
   <211> 30
   <212> DNA
   <213> Artificial
<220>
   <223> Primer
<400> 25
   ggcgggagat cttcagcgtc tagccatggg 30
<210> 26
   <211> 32
   <212> DNA
   <213> Artificial
<220>
   <223> Primer
<400> 26
   ggcggtctag agccgccacc atgcccatcg tg 32
<210> 27
   <211> 33
   <212> DNA
   <213> Artificial
<220>
   <223> Primer
<400> 27
   ggcggggaat tctcacagca gcctggcctt gtg 33
<210> 28
   <211> 32
   <212> DNA
   <213> Artificial
<220>
   <223> Primer
<400> 28
   ggcgggagat ctcgccacca tggtgagcaa gg 32
<210> 29
   <211> 31
   <212> DNA
   <213> Artificial
<220>
   <223> Primer
<400> 29
   ggcgggagat ctttacttgt acagctcgtc c 31
<210> 30
   <211> 24
   <212> DNA
   <213> Artificial
<220>
   <223> Primer
<400> 30
   gcaggagatc ttatggcgcc ccgc 24
<210> 31
   <211> 26
   <212> DNA
   <213> Artificial
<220>
   <223> Primer
<400> 31
   gcaggcggat cctcaaagag tgctga 26
<210> 32
   <211> 30
   <212> DNA
   <213> Artificial
<220>
   <223> Primer
<400> 32
   ggcggggatc cttagaaatt taagagatcc 30
<210> 33
   <211> 30
   <212> DNA
   <213> Artificial
<220>
   <223> Primer
<400> 33
   ggcgggagat ctatgatgga cccaaactcg 30
<210> 34
   <211> 31
   <212> DNA
   <213> Artificial
<220>
   <223> Primer
<400> 34
   ggcggagatc tatggcgtca cagaagagac c 31
<210> 35
   <211> 30
   <212> DNA
   <213> Artificial
<220>
   <223> Primer
<400> 35
   ggcgggggat cctcagcgtc tagccatggg 30
<210> 36
   <211> 26
   <212> DNA
   <213> Artificial
<220>
   <223> Primer
<400> 36
   gcaggagatc taacagcacg ctgatc 26
<210> 37
   <211> 27
   <212> DNA
   <213> Artificial
<220>
   <223> Primer
<400> 37
   gcaggcagat ctctagatag tctggta 27
<210> 38
   <211> 53
   <212> DNA
   <213> Artificial
<220>
   <223> Primer
<400> 38
   gcaggagatc tagcggggca ggatccatgc tagtacttca atgggcctct ctg 53
<210> 39
   <211> 33
   <212> DNA
   <213> Artificial
<220>
   <223> Primer
<400> 39
   gcaggcagat ctttatacat acctctcggc ctc 33
<210> 40
   <211> 30
   <212> DNA
   <213> Artificial
<220>
   <223> Primer
<400> 40
   ggcgggggat ccggtacctg gtgtctattg 30
<210> 41
   <211> 29
   <212> DNA
   <213> Artificial
<220>
   <223> Primer
<400> 41
   ggcgggagat ctcttcacaa tctgggtag 29
<210> 42
   <211> 29
   <212> DNA
   <213> Artificial
<220>
   <223> Primer
<400> 42
   ggcgggagat cctgtgcctt ctagttgcc 29
<210> 43
   <211> 28
   <212> DNA
   <213> Artificial
<220>
   <223> Primer
<400> 43
   ggcgggggat ccccatagag cccaccgc 28
<210> 44
   <211> 32
   <212> DNA
   <213> Artificial
<220>
   <223> Primer
<400> 44
   ggcgggggat cccgccacca tggtgagcaa gg 32
<210> 45
   <211> 31
   <212> DNA
   <213> Artificial
<220>
   <223> Primer
<400> 45
   ggcgggagat ctttacttgt acagctcgtc c 31
<210> 46
   <211> 30
   <212> DNA
   <213> Artificial
<220>
   <223> Primer
<400> 46
   ggcggtctag attagaaatt taagagatcc 30
<210> 47
   <211> 30
   <212> DNA
   <213> Artificial
<220>
   <223> Primer
<400> 47
   ggcggggaat tcatgatgga cccaaactcg 30
<210> 48
   <211> 31
   <212> DNA
   <213> Artificial
<220>
   <223> Primer
<400> 48
   ggcggtctag aatggcgtca cagaagagac c 31
<210> 49
   <211> 30
   <212> DNA
   <213> Artificial
<220>
   <223> Primer
<400> 49
   ggcggggaat tctcagcgtc tagccatggg 30
<210> 50
   <211> 30
   <212> DNA
   <213> Artificial
<220>
   <223> Primer
<400> 50
   ggcgggggat ccggtacctg gtgtctattg 30
<210> 51
   <211> 29
   <212> DNA
   <213> Artificial
<220>
   <223> Primer
<400> 51
   ggcgggagat ctcttcacaa tctgggtag 29
<210> 52
   <211> 34
   <212> DNA
   <213> Artificial
<220>
   <223> Primer
<400> 52
   ggcgggggat ccccggattg gccgcctcca gcag 34
<210> 53
   <211> 35
   <212> DNA
   <213> Artificial
<220>
   <223> Primer
<400> 53
   ggcgggggat ccgacgagag actgcgggaa gacac 35
<210> 54
   <211> 34
   <212> DNA
   <213> Artificial
<220>
   <223> Primer
<400> 54
   ggcgggggat ccccggattg gccgcctcca gcag 34
<210> 55
   <211> 35
   <212> DNA
   <213> Artificial
<220>
   <223> Primer
<400> 55
   ggcgggagat ctgacgagag actgcgggaa gacac 35
<210> 56
   <211> 34
   <212> DNA
   <213> Artificial
<220>
   <223> Primer
<400> 56
   ggcgggggat ccggagccag agatagggag agtc 34
<210> 57
   <211> 33
   <212> DNA
   <213> Artificial
<220>
   <223> Primer
<400> 57
   ggcgggggat cccagccaga gacagcgaca gag 33
<210> 58
   <211> 36
   <212> DNA
   <213> Artificial
<220>
   <223> Primer
<400> 58
   ggcgggggat cccccactgc agaggcaatc aacaag 36